# FASCICULE DE BREVET EUROPEEN

(11) **EP 2 734 518 B1**
(45) Date de publication et mention de la délivrance du brevet: **27.04.2016**
(21) Numéro de dépôt: 12743518.8
(22) Date de dépôt: 17.07.2012
(51) Int. Cl.: C07D 401/14, C07F 9/6558, G01N 33/58

(54) **AGENTS COMPLEXANTS ET COMPLEXES DE LANTHANIDE CORRESPONDANTS**
KOMPLEXBILDNER UND ENTSPRECHENDE LANTHANIDKOMPLEXE
COMPLEXING AGENTS AND CORRESPONDING LANTHANIDE COMPLEXES

(30) Priorité: 18.07.2011 FR 1156519
(43) Date de publication de la demande: 28.05.2014
(73) Titulaire: Cisbio Bioassays, 30200 Codolet (FR); Ecole Normale Supérieure de Lyon, 69007 Lyon (FR); Centre National de la Recherche Scientifique, 75016 Paris (FR)
(72) Inventeur: LAMARQUE, Laurent, F-30290 St Victor La Coste (FR); MAURY, Olivier, F-69126 Brindas (FR); PARKER, David, Durham City Durham DH15WA (GB); ZWIER, Jurriaan, F-30650 Rochefort du Gard (FR); WALTON, James W., Hallington Newcastle Upon Tyne Tyne and Wear NE19 2LW (GB); BOURDOLLE, Adrien, 44300 Nantes (FR)
(74) Mandataire: Nevant, Marc
(86) Numéro de dépôt international: PCT/FR2012/051691
(87) Numéro de publication internationale: WO 2013/011236

(56) Documents cités:
- EP-A1- 0 203 047
- EP-A1- 0 321 353
- WO-A1-2005/021538
- WO-A1-2005/058877
- WO-A1-2007/128874
- Harri Takalo ET AL: "Synthesis and Luminescence of Novel Eu"' Complexing Agents and Labels with 4-(Phenylethyny1)pyridine Subunits", HELVETICA CHIMICA ACTA, 1 janvier 1996 (1996-01-01), pages 789-802, XP055018996, Extrait de l'Internet: URL:http://onlinelibrary.wiley.com/store/1 0.1002/hlca.19960790321/asset/19960790321_ ftp.pdf?v=1&t=gyg00f71&s=fcb313ca9776bd67f 7628bd4ab68fdf79847fb01 [extrait le 2012-02-09]
- Martti Latva ET AL: "Evaluation of solution structures of highly luminescent europium(IlI) chelates by using laser induced excitation of the 7F o "-* 5D o transition", Inorganica Chimica acta, 1 janvier 1998 (1998-01-01), pages 63-72, XP055019096, Extrait de l'Internet: URL:http://pdn.sciencedirect.com/science?_ ob=MiamiImageURL&_cid=271401&_user=987766& _pii=S0020169397055539&_check=y&_origin=br owse&_zone=rslt_list_item&_coverDate=1998- 01-03&wchp=dGLzVBA-zSkzV&md5=c429925e29cf1 ac1852b56e9bcc6c192/1-s2.0-S00201693970555 39-main.pdf [extrait le 2012-02-10]
- KOVACS ET AL: "A general synthesis of mono- and disubstituted 1,4,7-triazacyclononanes", TETRAHEDRON LETTERS, ELSEVIER, AMSTERDAM, NL, vol. 36, no. 51, 18 décembre 1995 (1995-12-18), pages 9269-9272, XP005917508, ISSN: 0040-4039, DOI: 10.1016/0040-4039(95)02009-E

## Description

La présente invention a pour objet des agents complexants ou ligands, des complexes de lanthanides, leur utilisation pour marquer des molécules et les détecter par des techniques de fluorescence en temps résolu.

### Etat de la technique :

Les complexes de lanthanide ont vu leur utilisation augmenter de manière très importante depuis une vingtaine d'années dans le domaine des sciences de la vie. Ces composés fluorescents présentent en effet des caractéristiques spectroscopiques intéressantes, qui en font des marqueurs de choix pour détecter des molécules biologiques. Ces composés fluorescents sont particulièrement appropriés pour être utilisés en conjonction avec des fluorophores compatibles pour effectuer des mesures de FRET (acronyme de l'expression anglaise « Förster resonnance energy transfer »), dont l'application pour étudier les interactions entre biomolécules est exploitée de manière commerciale par plusieurs sociétés, dont Cisbio Bioassays et sa gamme de produits HTRF®. La durée de vie relativement longue des complexes de lanthanides permet également d'effectuer des mesures de fluorescence en temps résolu, c'est-à-dire avec un délai après excitation des fluorophores, ce qui permet de limiter les interférences de fluorescence dues au milieu de mesure. Cette dernière caractéristique est d'autant plus utile que le milieu de mesure se rapproche d'un milieu biologique, qui comprend de nombreuses protéines dont la fluorescence pourrait interférer avec celle des composés étudiés.

De nombreux complexes de lanthanides ont été décrits. Latva et al par exemple, ont divulgué 41 complexes d'Eu(III) et de Tb(III), dont ils ont étudié la luminescence (Journal of Luminescence Volume 75, n° 2, Septembre 1997, Pages 149-169). Le composé **39** en particulier, est constitué d'un cycle 1,4,7-triazacyclononane (ci-après « TACN »), dont les atomes d'azote sont substitués par des chromophores dérivés de phényléthynylpyridine. Bien que le rendement quantique du complexe constitué de ce chromophore et d'Eu(III) soit considéré comme bon par les auteurs, ce complexe n'est pas adapté au couplage avec une biomolécule. Par ailleurs, l'utilisation de ce composé en milieu aqueux peut être problématique puisque il est très hydrophobe. Enfin, l'absorption de ce complexe est optimale à 315 nm, alors que les lampes laser souvent utilisées dans les dosages biologiques émettent à la longueur d'onde de 337 nm.

D'Aléo et al ont décrit la synthèse de complexes de lanthanides constitués de trois ligands dérivés de l'acide dipicolonique (Inorg Chem. 2008 Nov 17;47(22):10258-68). L'un de ces ligands (L1) est constitué d'une molécule d'acide dipicolinique substituée par un groupe phényléthynyl, lui-même portant un éther-oxyde de polyéthylèneglycol (ci-après « PEG ») sur le groupe phényle. D'après les auteurs, le groupe PEG confère à ce produit une bonne solubilité en milieux aqueux et dans les solvants organiques. Toutefois, ces complexes ne sont pas suffisamment stables en milieu aqueux et ne sont pas utilisables dans une réaction de bioconjuguaison.

La demande internationale de brevet WO2005/058877 est relative à des complexes de lanthanides dont certains sont basés sur un cycle TACN dont trois atomes d'azote sont substitués par des chromophores constitués d'un dérivé de pyridine, notamment de phénylpyridine. Les inventeurs proposent par ailleurs d'inclure dans ces composés un groupe réactif pour pouvoir les conjuguer facilement avec des biomolécules. Il est ainsi proposé d'inclure ce groupe réactif *via* un bras d'espacement soit au niveau d'un carbone du cycle TACN, soit au niveau de la pyridine du chromophore.

Plusieurs autres complexes de lanthanides ont été divulgués et certains sont exploités de manière commerciale : on peut citer en particulier les cryptates macropolycycliques de lanthanides (EP 0 180 492, EP 0 321 353, EP 0 601 113 , WO 2001/96877, WO2008/063721), les complexes de lanthanide comportant un motif dérivé de la coumarine lié à un motif diéthylène triamine penta acide (US 5,622,821), et ceux comprenant des dérivés de pyridine (US 4,920,195, US 4,761,481), de bipyridine (US 5,216,134), ou de terpyridine (US 4,859,777, US 5,202,423, US 5,324,825).

La demande de brevet WO89/01475 décrit la préparation de macrocycles triazotés dont l'un des atomes de carbone porte un groupe L-Z, et notamment du 2-(4-N-benzamidyl)butyl-1,4,7-triazacyclonane (intermédiaire 12). La synthèse d'hétéromacrocycles azotés dont l'un des atomes de carbone est substitué est également décrite par Cox et al (J. Chem. Soc., Perkin Trans. 1, 1990, 2567-2576) et Craig et al (J. Chem. Soc., Chem. Commun., 1989, 794-796).

La présente invention vise à pallier aux inconvénients des composés de l'art antérieur, et à fournir des complexes de lanthanides fluorescents présentant une meilleure brillance que les composés de l'art antérieur lorsqu'ils sont excités aux alentours de 337 nm, si possible une bonne solubilité en milieu aqueux, un spectre d'émission adapté à leur utilisation dans des expériences de FRET, ainsi qu'une bonne praticité pour le marquage de biomolécules.

### Description de l'invention :

Les problèmes mentionnés précédemment ont été résolus grâce à des agents complexants constitués d'un macrocycle triazoté (1,4,7-triazacyclononane, ci-après 147TACN, 1,5,9-triazacyclododécane, ci-après 159TACD, 1,4,8-triazacyclodécane, ci-après 148TACD ou 1,4,8-triazacycloundécane, ci-après 148TACU) dont les atomes d'azote sont substitués par des chromophores de type phényléthynylpyridine, ces chromophores comprenant de un à trois groupes affectant la densité électronique de la molécule (ci-après groupe « O-donneur », « S-donneur », « NHCO-donneur », « SCO-donneur », « NHCS-donneur », « SCS-donneur » ou alkyle inférieur) directement lié au groupe phényle, et de manière optionnelle au moins un groupement polyéthylène-glycol (PEG) qui va conférer à la molécule une bonne solubilité en milieu aqueux. Ces composés peuvent également comporter un groupe réactif permettant leur conjugaison avec une molécule à marquer. Les agents complexants selon l'invention forment des complexes stables avec les lanthanides, et peuvent être utilisés pour produire des conjugués fluorescents de molécules d'intérêt. Les complexes de lanthanides selon l'invention présentent d'excellentes propriétés photophysiques, en particulier en ce qui concerne leur rendement quantique, la durée de vie de leur luminescence et leur spectre d'excitation qui est très bien adapté à une excitation laser à environ 337 nm. La présence de trois chromophores augmente de façon significative le coefficient d'absorption molaire (epsilon) et par conséquent la brillance du complexe. La brillance (rendement quantique x coefficient d'absorption molaire) de ces complexes dans des milieux biologiques est également meilleure que celle des composés de l'art antérieur.

### AGENTS COMPLEXANTS

Les agents complexants selon l'invention sont les composés de formule (I) : 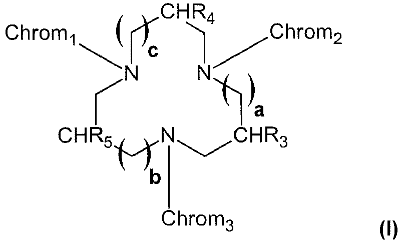 dans laquelle :
- soit a=b=c=1 (1,5,9-triazacyclododécane), soit a=b=c=0 (1,4,7-triazacyclononane), soit a=1 et b=c=0 (1,4,8-triazacyclodécane), soit a=b=1 et c=0 (1,4,8-triazacycloundécane) ;
- R₃, R₄ et R₅ sont chacun choisis parmi les atomes ou groupes suivants : H, -L-G ;
- Chrom₁, Chrom₂ et Chrom₃ sont des chromophores identiques ou différents et répondent tous trois à une seule des formules suivantes : 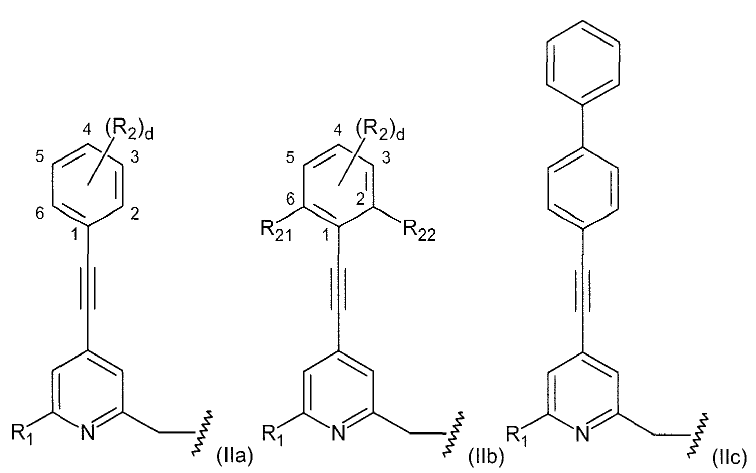 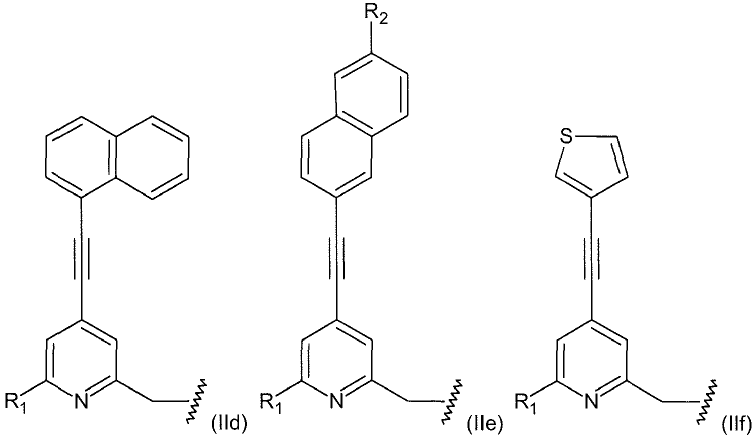 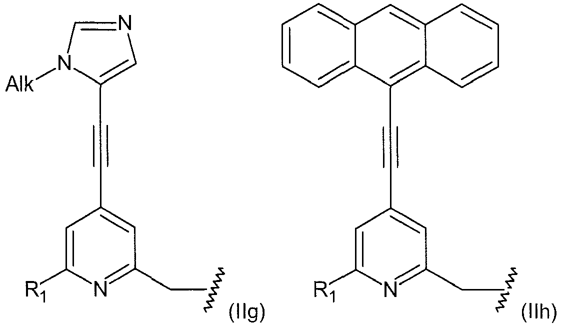 dans lesquelles
   - d est un nombre entier allant de 1 à 3
   - chaque R₁ est choisi parmi les groupes suivants : -COOH, -PO(OH)R₆, R₆ représentant un groupe choisi parmi : phényle, benzyle, méthyle, éthyle, propyle, *n-*butyle, sec-butyle, isobutyle, *tert*-butyle;
   - R₂₁ et R₂₂ représentent indépendamment l'un de l'autre un atome d'hydrogène ou un un alkyle linéaire ou ramifié comprenant de 1 à 6 atomes de carbone ;
   - chaque R₂ est choisi parmi les groupes suivants :
      - *groupes alkyles* linéaires ou ramifiés comprenant de 1 à 6 atomes carbones éventuellement substitués par un groupe PEG ;
      - *groupes O-donneurs choisis parmi:* OH ; -OPhényle ; -O-CH₂-CO-N-Alk ; -O-CH₂-CO-O-Alk ; -O-CH₂-CO-NH ; -O-CH₂-CO-OH; O-CH₂-CO-N-LG ; -O-CH₂-CO-O-L-G -O-L-G ; -O-Alk; -O-PEG; le groupe de formule : 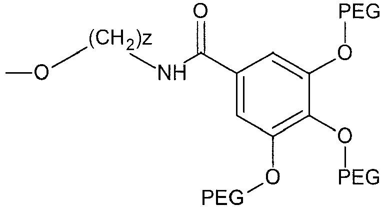 dans laquelle z est un nombre entier allant de 1 à 5 ;
      - *groupes S-donneurs choisis parmi :* -S-L-G ; -S-Alk; -S-PEG; le groupe de formule : 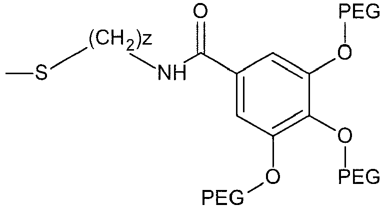 dans laquelle z est un nombre entier compris entre 1 et 5 ;
      - *groupes NHCO-donneurs choisis parmi :* -NHCO-L-G ; -NHCO(OAlk); -NHCO(NHAlk); -NHCO(NAIk1Alk2); -NHCO(SAlk); -NHCO(Alk); -NHCO-PEG; -NHCO-phényle ; un groupe de formule : 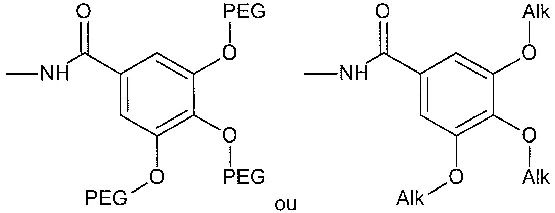
      - *groupes SCO-donneurs choisis parmi :* -SCO-L-G; -SCO(OAlk); -SCO(NHAlk); -SCO(NAlk₁Alk₂); -SCO(SAlk); -SCO(Alk) ; -SCO-PEG;
      - *groupes NHCS-donneurs choisis parmi :* -NHCS(OAlk); -NHCS(NHAlk); -NHCS(NAlk₁Aik₂); -NHCS(SAlk); -NHCS(Alk); -NHCS-PEG; -NHCS-L-G;
      - *groupes SCS-donneurs choisis parmi :* -SCS(OAlk); -SCS(NHAlk); -SCS(NAlk₁Alk₂); -SCS(SAlk); -SCS(Alk) ; -SCS-PEG; -SCS-L-G;
         - PEG est un groupe de formule-CH₂-(CH₂OCH₂)y-CH₂OCH₃, y étant un nombre entier allant de 1 à 5;
         - Alk, Alk1 et Alk2 sont chacun un alkyle linéaire ou ramifié en C₁-C₁₀ éventuellement substitué par un groupe -O-PEG ;
         - L est un bras d'espacement;
         - G est un groupe réactif permettant la liaison du complexe ou de l'agent complexant à une molécule à marquer;
      étant entendu que :
- lorsque R₂₂ et R₂₁ sont des groupes alkyles, d=1 et lorsque l'un des groupes R₂₂ ou R₂₁ est un groupe alkyle et que l'autre est un atome d'hydrogène, d=1 ou d= 2 ;
- lorsque R₂ comporte un groupe -L-G, R₃= R₄ = R₅ = H ;
- lorsque R₂ ne comporte pas de groupe -L-G, soit l'un des groupes R₃, R₄ et R₅ est un groupe
- L-G soit R₃ = R₄ = R₅ = H; et
- lorsque plusieurs groupes R₂ sont présents, au moins un est en position 4 du cycle benzénique.

De préférence, Chrom₁, Chrom₂ et Chrom₃ sont des chromophores identiques ou différents et répondent tous trois à une seule des formules suivantes :

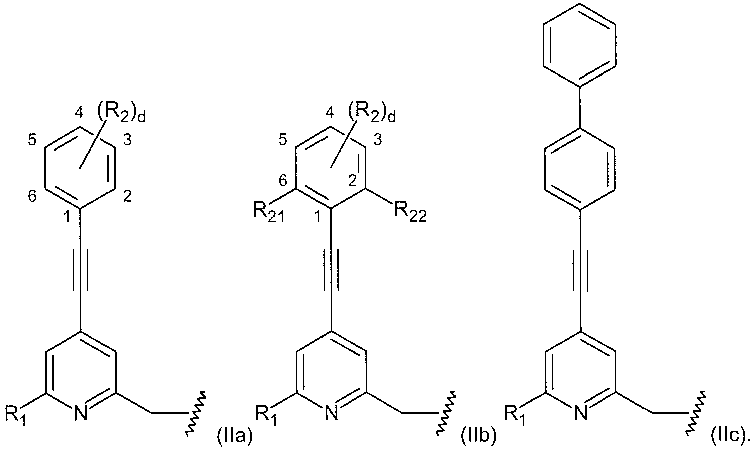

L'invention concerne en particulier les agents complexants de formule (I) : 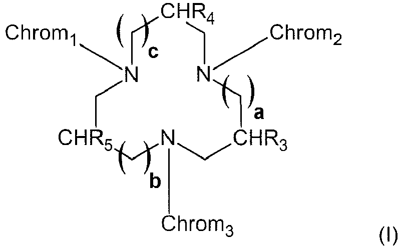 dans laquelle :
- soit a=b=c=1 (1,5,9-triazacyclododécane), soit a=b=c=0 (1,4,7-triazacyclononane), soit a=1et b=c=0 (1,4,8-triazacyclodécane), soit a=b=1 et c=0 (1,4,8-triazacycloundécane) ;
- R₃, R₄ et R₅ sont chacun choisis parmi les atomes ou groupes suivants : H, -L-G ;
- Chrom₁, Chrom₂ et Chrom₃ sont des chromophores identiques ou différents de formule (II) : 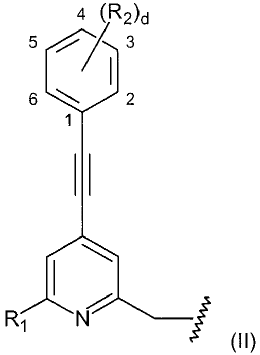 dans laquelle
   - d est un nombre entier allant de 1 à 3 ;
   - R₁ est choisi parmi les groupes suivants : -COOH, -PO(OH)R₆, R₆ représentant un groupe choisi parmi : phényle, benzyle, méthyle, éthyle, propyle, *n*-butyle, sec-butyle, isobutyle, *tert*-butyle, les groupes phényle et méthyle étant préférés;
   - chaque R₂ est choisi parmi les groupes suivants :
      - *groupes alkyles* linéaires ou ramifiés comprenant de 1 à 6 atomes de carbone éventuellement substitués par un groupe PEG ;
      - *groupes O-donneurs choisis parmi :* -O-L-G ; -O-Alk; -O-PEG; le groupe de formule : 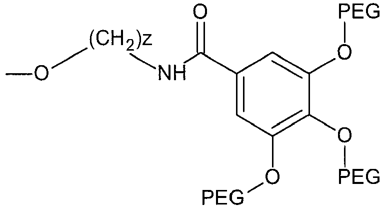 dans laquelle z est un nombre entier allant de 1 à 5 ;
      - *groupes S-donneurs choisis parmi :* -S-L-G ; -S-Alk; -S-PEG; le groupe de formule : 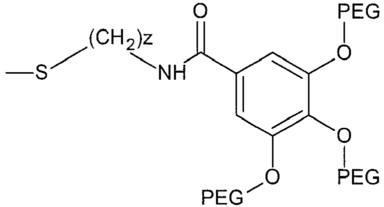 dans laquelle z est un nombre entier allant de 1 à 5 ;
      - *groupes NHCO-donneurs choisis parmi :* -NHCO-L-G ; -NHCO(OAlk); -NHCO(NHAlk); -NHCO(NAlk₁Alk₂); -NHCO(SAlk); -NHCO(Alk); -NHCO-PEG; le groupe de formule : 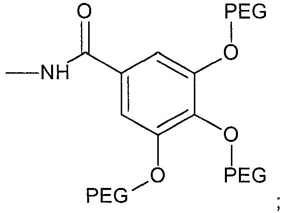
      - *groupes SCO-donneurs choisis parmi :* -SCO-L-G; -SCO(OAlk); -SCO(NHAlk); -SCO(NAlk₁Alk₂); -SCO(SAlk); -SCO(Alk) ; -SCO-PEG;
      - *groupes NHCS-donneurs choisis parmi :* -NHCS(OAlk); -NHCS(NHAlk); -NHCS(NAlk₁Alk₂); -NHCS(SAlk); -NHCS(Alk); -NHCS-PEG; -NHCS-L-G ;
      - *groupes SCS-donneurs choisis parmi :* -SCS(OAlk); -SCS(NHAlk); -SCS(NAlk₁Alk₂); -SCS(SAlk); -SCS(Alk) ; -SCS-PEG; -SCS-L-G;
         - PEG est un groupe de formule -CH₂-(CH₂OCH₂)y-CH₂OCH₃, y étant un nombre entier allant de 1 à 5;
         - Alk, Alk1 et Alk2 sont chacun un alkyle linéaire ou ramifié en C₁-C₁₀ éventuellement substitué par un groupe -O-PEG ;
   - L est un bras d'espacement;
   - G est un groupe réactif permettant la liaison du complexe ou de l'agent complexant à une molécule à marquer;
   étant entendu que :
   - lorsque R₂ comporte un groupe -L-G, R₃ = R₄ = R₅ = H ;
   - lorsque R₂ ne comporte pas de groupe -L-G, soit l'un des groupes R₃, R₄ et R₅ est un groupe -L-G, soit R₃ = R₄ = R₅ = H; et
   - lorsque plusieurs groupes R₂ sont présents, au moins un est en position 4 du cycle benzénique.

Une famille préférée de composés de formule (I) comprend les composés où :
*soit* chrom₁, chrom₂ et chrom₃ sont identiques et sont chacun substitués par un à trois groupes R₂ ne comportant pas de groupe -L-G, et les groupes R₃-R₅ sont des atomes d'hydrogène ;
*soit* chrom₁, chrom₂ et chrom₃ sont identiques et sont substitués par un à trois groupes R₂ ne comportant pas de groupe -L-G, et un des groupes R₃-R₅ est un groupe L-G, les autres étant des atomes d'hydrogène ;
*soit* deux des groupes chrom₁, chrom₂ et chrom₃ sont identiques et sont chacun substitués par un à trois groupes R₂ ne comportant pas de groupe -L-G et le troisième chromophore est substitué par un groupe R₂ comportant un groupe -L-G, les groupes R₃-R₅ sont des atomes d'hydrogène.

Les sous-familles d'agents complexants suivants sont préférées :
- composés de formule (I) caractérisés en ce que l'un des groupes R₂ comporte un groupe -L-G ;
- composés de formule (I) caractérisés en ce que l'un des groupes R₃, R₄ ou R₅ comporte un groupe -L-G ;
- composés de formule (I) caractérisés en ce qu'il ne comporte aucun groupe -L-G.

Parmi les composés de formule (I) et ceux appartenant aux sous-familles précitées, les composés suivants sont préférés :
- Ceux qui sont caractérisés en ce que a=b=c=1 (1,5,9-triazacyclododécane, 159TACD) ;
- Ceux qui sont caractérisés en ce que a=b=c=0 (1,4,7-triazacyclononane, 147TACN) ;
- Ceux qui sont caractérisés en ce que a=1 et b=c=0 (1,4,8-triazacyclodécane, 148TACD) ;
- Ceux qui sont caractérisés en ce que a=b=1 et c=0 (1,4,8-triazacycloundécane, 148TACU).

Parmi les composés de formule (I) et ceux appartenant aux sous-familles précitées, les composés ayant les caractéristiques suivantes sont également préférés :
- Les composés caractérisés en ce que les groupes R₁ des chromophores chrom₁, chrom₂ et chrom₃ sont des groupes -COOH ;
- Les composés caractérisés en ce que les groupes R₁ des chromophores chrom₁, chrom₂ et chrom₃ sont des groupes -PO(OH)R₆ ;
- Les composés caractérisés en ce que le groupe R₁ du chromophore chrom₁ est un groupe -COOH et les groupes R₁ des chromophores chrom₂ et chrom₃ sont des groupes -PO(OH)R₆ ;
- Les composés caractérisés en ce que le groupe R₁ du chromophore chrom₁ est un groupe -PO(OH)R₆, et les groupes R₁ des chromophores chrom₂ et chrom₃ sont des groupes -COOH.

L'une des caractéristiques techniques essentielles des composés selon l'invention est la présence, sur les chromophores, de groupes R₂ qui affectent la densité électronique de la molécule et contribuent aux bonnes propriétés spectroscopiques de ces composés. Ainsi, parmi les composés de formule (I) et ceux appartenant aux sous-familles précitées, les composés dont les groupes donneurs R₂ sont les suivants sont préférés :
- les composés caractérisés en ce que les groupes R₂ sont des *groupes alkyles* linéaires ou ramifiés comprenant de 1 à 6 atomes carbones éventuellement substitués par un groupe PEG ;
- les composés caractérisés en ce que les groupes R₂ sont des *groupes* O-*donneurs choisis parmi* : -O-L-G ; -O-Alk; -O-PEG; le groupe de formule : 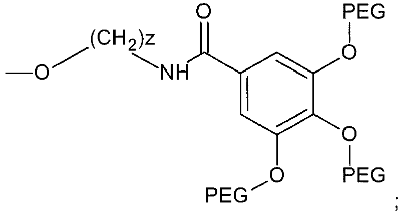
- les composés caractérisés en ce que les groupes R₂ sont des *groupes S-donneurs choisis parmi :* -S-L-G ; -S-Alk; -S-PEG; le groupe de formule : 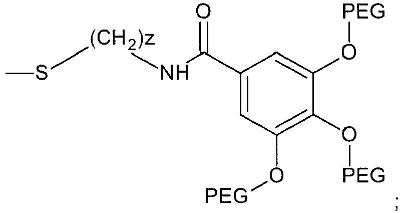
- les composés caractérisés en ce que les groupes R₂ sont des *groupes NHCO-donneurs choisis parmi* : -NHCO-L-G ; -NHCO(OAlk); -NHCO(NHAlk); -NHCO(NAlk₁Alk₂); -NHCO(SAlk); -NHCO(Alk); -NHCO-PEG; le groupe de formule : 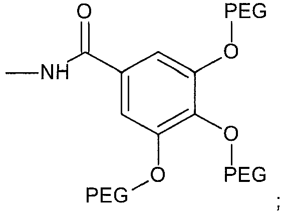
- les composés caractérisés en ce que les groupes R₂ sont des *groupes SCO-donneurs choisis parmi :* -SCO-L-G; -SCO(OAlk); -SCO(NHAlk); -SCO(NAlk₁Alk₂); -SCO(SAlk); -SCO(Alk) ; -SCO-PEG;
- les composés caractérisés en ce que les groupes R₂ sont des *groupes NHCS-donneurs choisis parmi* -NHCS(OAlk); -NHCS(NHAlk); -NHCS(NAlk₁Alk₂); -NHCS(SAlk); -NHCS(Alk); -NHCS-PEG; -NHCS-L-G ;
- les composés caractérisés en ce que les groupes R₂ sont des *groupes SCS-donneurs choisis parmi :* -SCS(OAlk); -SCS(NHAlk); -SCS(NAlk₁Alk₂); -SCS(SAlk); -SCS(Alk) ; -SCS-PEG; -SCS-L-G.

Les composés selon l'invention comportent de un à trois groupes R₂ par chromophore, et de préférence comportent :
- un seul groupe R₂ en position 4 du groupe phényle, ou bien
- un groupe R₂ en position 4 et un groupe R₂ en position 3 du groupe phényle, ou bien
- un groupe R₂ en position 4 et un groupe R₂ en position 5 du groupe phényle, ou bien
- un groupe R₂ en position 4 et un groupe R₂ en position 2 du groupe phényle, ou bien
- un groupe R₂ en position 4 et un groupe R₂ en position 6 du groupe phényle, ou bien
- un groupe R₂ en position 4, un groupe R₂ en position 3 et un groupe R₂ en position 5 du groupe phényle, ou bien
- un groupe R₂ en position 4, un groupe R₂ en position 2 et un groupe R₂ en position 6 du groupe phényle.

Lorsque plusieurs groupes R₂ sont présents sur un chromophore, ils sont de préférence identiques.

Enfin, les composés précédents caractérisés en ce qu'ils comportent au moins un groupe PEG sont également préférés.

Le tableau 1 représente différentes familles de composés préférés selon l'invention : ces composés répondent à la formule générale (I) et certaines de leurs caractéristiques sont indiquées dans la première colonne (type de macrocycle triazoté) et la première ligne (nature des substituants R₂ et R₁ des chromophores). Chaque signe « + » représente une famille de composés préférés. Ainsi, la case correspondant à l'intersection de la première colonne et de la première ligne désigne la famille de composés comportant un cycle 147TACN, dont les chromophores portent des groupes R₂ qui sont des groupes alkyles donneurs et dont les trois groupes R₁ sont des groupes COOH. L'abréviation « R₁ : (COOH), 2(POOHR₆) » signifie que un des groupes R₁ est un groupe COOH et les deux autres groupes R₁ sont des groupes POOHR₆).

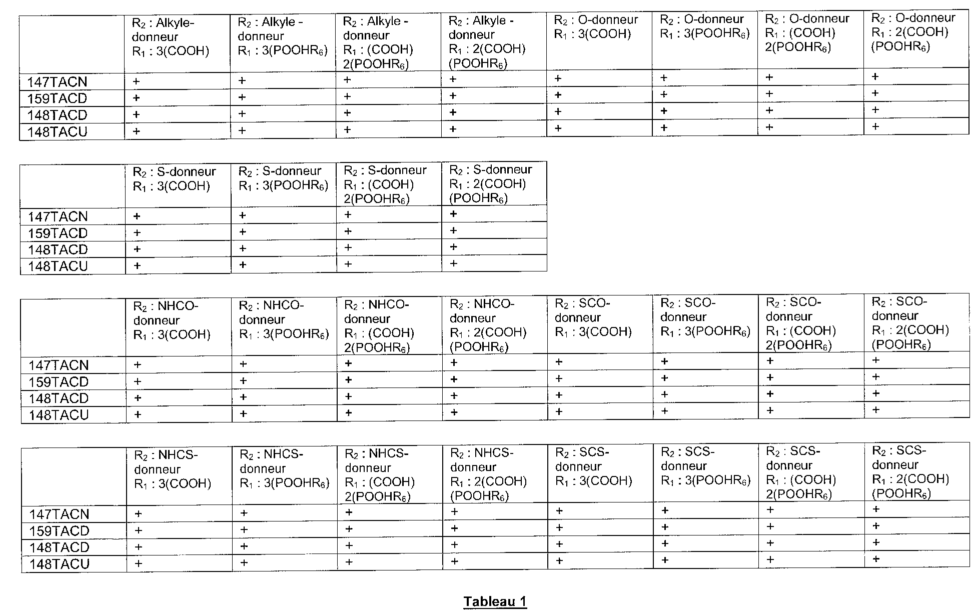

Pour chacune des familles de composés du tableau 1, les sous-familles suivantes sont préférées :
- composés caractérisés en ce que l'un des groupes R₂ comporte un groupe -L-G ;
- composés caractérisés en ce que l'un des groupes R₃, R₄ ou R₅ comporte un groupe - L-G;
- composés caractérisés en ce qu'ils ne comportent aucun groupe -L-G.

Par ailleurs, pour chacune des familles du tableau 1, les composés comportent de un à trois groupes R₂ par chromophore, et de préférence comportent :
- un seul groupe R₂ en position 4 du groupe phényle, ou bien
- un groupe R₂ en position 4 et un groupe R₂ en position 3 du groupe phényle, ou bien
- un groupe R₂ en position 4 et un groupe R₂ en position 5 du groupe phényle, ou bien
- un groupe R₂ en position 4 et un groupe R₂ en position 2 du groupe phényle, ou bien
- un groupe R₂ en position 4 et un groupe R₂ en position 6 du groupe phényle, ou bien
- un groupe R₂ en position 4, un groupe R₂ en position 3 et un groupe R₂ en position 5 du groupe phényle, ou bien
- un groupe R₂ en position 4, un groupe R₂ en position 2 et 1 groupe R₂ en position 6 du groupe phényle.

Les composés du tableau 1 caractérisés en ce qu'ils comportent au moins un groupe PEG sont également préférés.

Enfin, lorsque les composés du tableau 1 comportent un groupe R₆, celui-ci est préférentiellement un groupe méthyle ou phényle.

L'invention a également pour objet les composés, ligands et complexes décrits dans le tableau 2.

Le groupe réactif G porté par un bras d'espacement L, permet de coupler les composés selon l'invention avec une espèce que l'on souhaite rendre fluorescente, par exemple une molécule organique, un peptide ou une protéine. Les techniques de conjugaison de deux molécules organiques sont basées sur l'utilisation de groupes réactifs et relèvent des connaissances générales de l'homme du métier. Ces techniques classiques sont décrites par exemple dans Bioconjugate Techniques, G.T. Hermanson, Academic Press, Second Edition 2008, p. 169-211.

Typiquement, le groupe réactif est un groupe électrophile ou nucléophile qui peut former une liaison covalente lorsqu'il est mis en présence d'un groupe nucléophile ou électrophile approprié, respectivement. La réaction de conjugaison entre un composé selon l'invention comportant un groupe réactif et une molécule organique, un peptide ou une protéine portant un groupe fonctionnel entraîne la formation d'une liaison covalente comportant un ou plusieurs atomes du groupe réactif.
De préférence, le groupement réactif G est un groupe dérivé d'un des composés ci-après : un acrylamide, une amine activée (par exemple une cadavérine ou une éthylènediamine), un ester activé, un aldéhyde, un halogénure d'alkyle, un anhydride, une aniline, un azide, une aziridine, un acide carboxylique, un diazoalcane, un haloacétamide, une halotriazine, telle que la monochlorotriazine, la dichlorotriazine, une hydrazine (y compris les hydrazides), un imido ester, un isocyanate, un isothiocyanate, un maléimide, un halogénure de sulfonyle, ou un thiol, une cétone, une amine, un halogénure d'acide, un ester d'hydroxysuccinimidyle, un ester d'hydroxysulfosuccinimidyle, un azidonitrophényle, un azidophényle, un 3-(2-pyridyl dithio)-propionamide, un glyoxal, une triazine, un groupe acétylénique, et en particulier les groupes de formule : 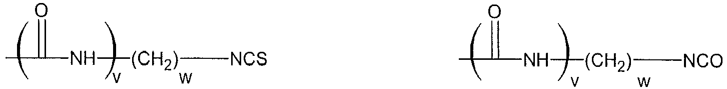 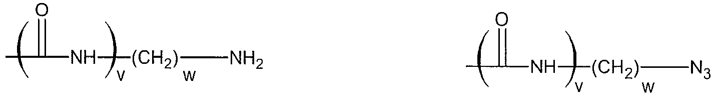 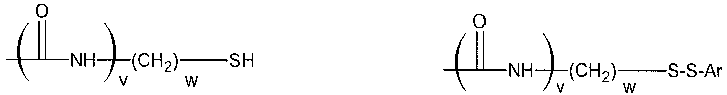 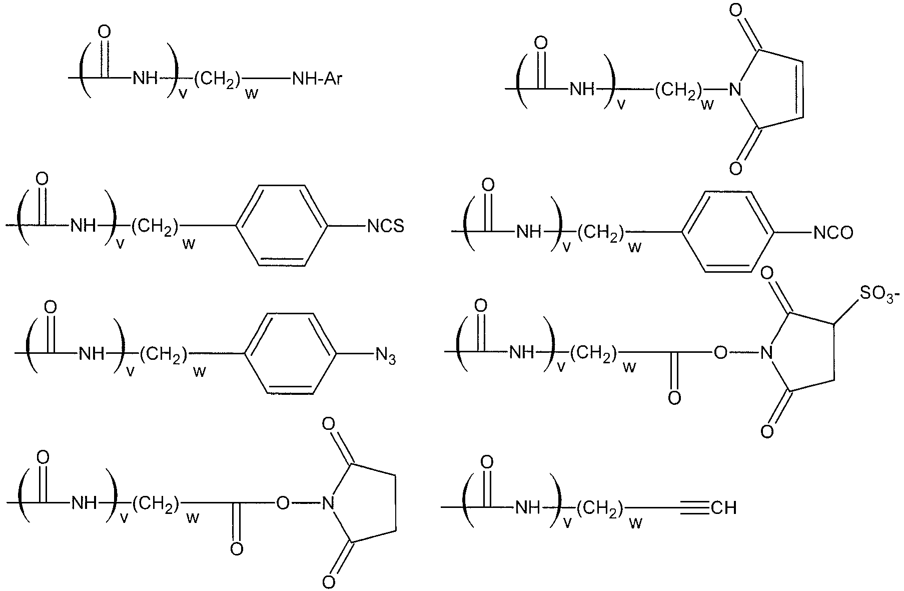 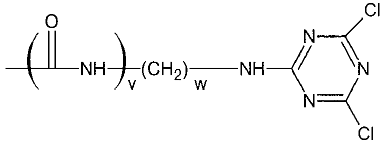 dans lesquels w varie de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène.

De manière préférée, le groupement réactif G est un acide carboxylique, une amine, un ester de succinimidyle, un haloacétamide, une hydrazine, un isothiocyanate, un groupe maléimide, une amine aliphatique.

Ces groupes réactifs peuvent être directement liés à l'agent complexant par une liaison covalente ou bien *via* un bras d'espacement constitué de manière avantageuse par un radical organique bivalent, choisi parmi les groupes alkylènes linéaires ou ramifiés en C₁-C₂₀, contenant éventuellement une ou plusieurs doubles ou triples liaisons; les groupes cycloalkylènes en C₅-C₈ et les groupes arylènes en C₆-C₁₄, lesdits groupes alkylènes, cycloalkylènes ou arylènes contenant éventuellement un ou plusieurs hétéroatomes, tels que l'oxygène, l'azote, le soufre, le phosphore ou un ou plusieurs groupe(s) carbamoyle ou carboxamido, et lesdits groupes alkylènes, cycloalkylènes ou arylènes étant éventuellement substitués par des groupes alkyle en C₁-C₈, aryle en C₆-C₁₄, sulfonate ou oxo.

En particulier, les bras d'espacement peuvent être choisis parmi les groupes divalents suivants :
1) 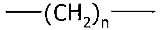
2) 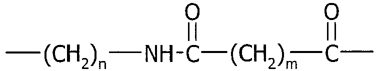
3) 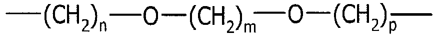
4) 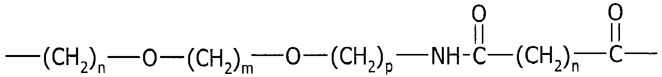
5) 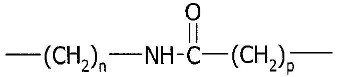
6) 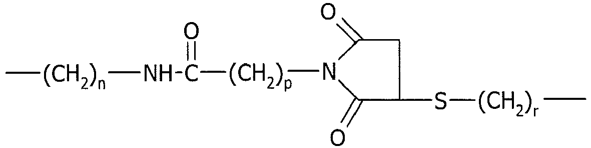
7) 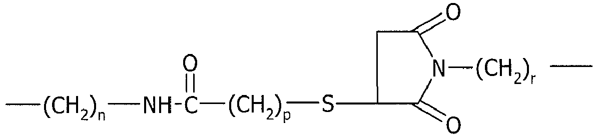
8) 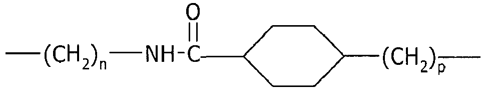
9) 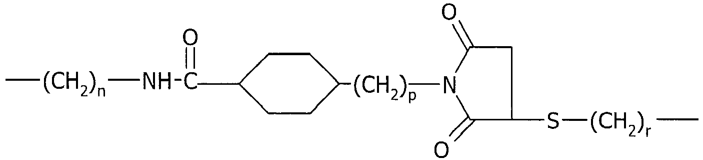
10) 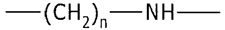
11) 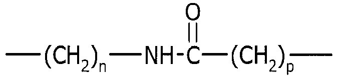
12) 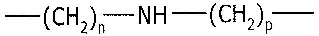
13) 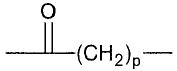
dans lesquels n, m, p, r sont des nombres entiers de 1 à 16, de préférence de 1 à 5.

De manière préférée, le groupe -LG est constitué d'un groupement réactif G choisi parmi : un acide carboxylique, une amine, un ester de succinimidyle, un haloacétamide, une hydrazine, un isothiocyanate, un groupe maléimide, une amine aliphatique, et d'un bras d'espacement L constitué d'une chaîne alkylène comprenant de 1 à 5 atomes de carbone. De manière encore plus préférée, le groupe -LG est une amine portée par une chaîne alkylène comprenant de 1 à 5 atomes de carbone.

Une molécule organique, un peptide ou une protéine susceptible d'être marquée par un composé selon l'invention comprendra donc un groupe fonctionnel avec lequel réagira le groupe réactif du complexe de lanthanide ou de l'agent complexant. Par exemple, la molécule organique, la protéine ou le peptide comporte un des groupes suivants : amine, amide, thiol, alcool, aldéhyde, cétone, hydrazine, hydroxylamine, amine secondaire, halogénure, époxyde, ester (carboxylate d'alkyle), acide carboxylique, des groupes comportant des doubles liaisons ou une combinaison de ces groupes fonctionnels. Les groupes amines ou thiols présents naturellement sur les protéines sont souvent utilisés pour procéder au marquage de ces molécules.

### COMPLEXES

L'invention concerne également les complexes de lanthanides constitués d'un atome de lanthanide complexé par un agent complexant tel que décrit ci-dessus, le lanthanide étant choisi parmi : Eu³⁺, Tb³⁺, Gd³⁺, Dy³⁺, Nd³⁺, Er³⁺. De préférence, le lanthanide est Tb³⁺ ou Eu³⁺ et de manière encore plus préférée Eu³⁺.

Ces complexes sont préparés en mettant en contact les agents complexants selon l'invention et un sel de lanthanide. Ainsi la réaction entre un équivalent d'agent complexant et 1 à 5 équivalents de sel de lanthanide (europium ou terbium sous forme de chlorures, d'acétates ou de triflates) dans un solvant (acétonitrile, méthanol ou autre solvant compatible avec ces sels) à reflux pendant plusieurs heures conduit au complexe correspondant.

Comme indiqué précédemment, les complexes fluorescents obtenus présentent d'excellentes propriétés photophysiques, en particulier en ce qui concerne leur rendement quantique, la durée de vie de leur luminescence et leur spectre d'excitation qui est très bien adapté à une excitation laser à environ 337 nm. De plus la répartition des bandes de leurs spectres d'émission est centrée autour de 620 nm conférant ainsi aux complexes des propriétés exceptionnelles et très favorables dans une utilisation de FRET avec des accepteurs de type cyanine ou allophycocyanine (telle que la XL665 commercialisée par Cisbio Bioassays). Du fait de la grande stabilité de ces complexes dans les milieux biologiques contenant des cations divalents (Mn²⁺, Ca²⁺, Mg²⁺...) ou de l'EDTA, leur luminescence reste excellente comparée aux complexes de l'art antérieur.

### CONJUGUES

Les agents complexants et complexes selon l'invention comportant un groupe réactif sont particulièrement adaptés au marquage de molécules organiques ou biologiques comportant un groupe fonctionnel susceptible de réagir avec le groupe réactif pour former une liaison covalente. Ainsi l'invention concerne aussi l'utilisation de complexes pour le marquage de molécules biologiques (protéines, anticorps, enzymes, hormones etc...).

L'invention concerne également les molécules marquées par un complexe selon l'invention. Toutes les molécules organiques ou biologiques peuvent être conjuguées avec un complexe selon l'invention si elles possèdent un groupe fonctionnel susceptible de réagir avec le groupe réactif. En particulier, les conjugués selon l'invention comportent un complexe selon l'invention et une molécule choisie parmi : un acide aminé, un peptide, une protéine, un anticorps, un sucre, une chaîne glucidique, un nucléoside, un nucléotide, un oligonucléotide, un substrat d'enzyme (en particulier un substrat d'enzyme suicide telle qu'une benzylguanine ou une benzylcytosine (substrats des enzymes commercialisées sous les dénominations Snaptag et Cliptag), un chloroalcane (substrat de l'enzyme commercialisée sous la dénomination Halotag), le coenzyme A (substrat de l'enzyme commecialisée sous le nom ACPtag ou MCPtag).

### SYNTHESE

La préparation des agents complexants (ligands) et des complexes selon l'invention est décrite de manière schématique ci-après, et de manière plus détaillée dans la partie expérimentale.

### Synthèse des macrocycles 1,4,7-triazacyclononane (147TACN), 1,5,9-triazacyclododécane (TACD), 1,4,8-triazacyclodécane (1,4,8TACD) et 1,4,8-triazacycloundécane (148TACU)

Plusieurs procédés de synthèse des cycles 147TACN et 159TACD ont été décrits et les sels de trihydrochlorure de 147TACN et 159TACD sont disponibles commercialement. Les azamacrocycles 148TACD et 148TACU peuvent être préparés en utilisant la procédure décrite par Anders et al (European Journal Inorganic Chemistry (2006) 1444-1455).

Lorsque les 3 chromophores greffés sur le macrocycle ne sont pas identiques, c'est-à-dire lorsque l'un d'entre eux porte un groupe réactif -L-G pour permettre la conjugaison du produit avec un composé à marquer, la synthèse des produits selon l'invention nécessite l'utilisation de macrocycles triazotés dont 1 ou 2 des amines secondaires sont protégées par des groupes protecteurs. Parker et al ont décrit la synthèse de composés TACN dont l'une des amines est protégée par un groupe benzoyle (Journal Chemical Society Perkin trans 1 (1990) 2567), cependant la déprotection de l'amine n'est pas compatible avec la synthèse des composés selon l'invention.

Les composés 147TACN, 159TACD, 148TACD et 148TACU monoprotégés selon l'invention possèdent une amine protégée par un groupement résistant à l'hydrogénation, par exemple par un groupe carbamate (Boc). Ces composés ne sont pas disponibles dans le commerce, les éléments publiés dans la littérature ne permettent pas leur synthèse (absence de procédures expérimentales) et il est également impossible de les préparer de façon directe et avec un rendement convenable tant la formation des macrocycles di-et trisubstitués est favorisée (Kovacs et al Journal Chemical Society, Chemical Commmunication 36 (1995) 9269-9272). Au vu de ces problèmes techniques, une autre stratégie a été élaborée (SCHEMA 4). Elle s'appuie sur la préférence des macrocycles triazotés (147TACN, 159TACD, 148TACD et 148TACU) pour la disubstitution en protégeant tout d'abord deux amines des *macrocycles* par un groupement protecteur P, orthogonal au groupement Boc ou à tout autre groupe résistant à l'hydrogénation, c'est-à-dire dont les conditions de déprotection sont différentes de celles du groupe Boc. Le choix du groupement P s'est porté sur le groupement protecteur Cbz comme le propose la littérature en utilisant comme précurseur le Cbz-ON (2-benzyloxycarbonyloxyimino)-2-phénylacétonitrile). Cependant, ce composé n'étant pas commercialisé, la synthèse a été élaborée en utilisant l'un de ses analogues, le Moz-ON ((2-(4-méthoxybenzyloxycarbonyloxy imino)-2-phénylacétonitrile) qui lui est disponible.

Comme décrit avec le Cbz-ON, deux équivalents de Moz-ON ont été condensés sur le macrocycle triazoté pour donner les composés disubstitués avec des rendements convenables. La purification de ces produits s'est avérée délicate car le groupement Moz est sensible à l'acidité. Même celle de la silice est suffisante pour entraîner une dégradation des produits lors de la purification sur colonne de chromatographie. Pour éviter cette dégradation, les milieux réactionnels ont été purifiés sur colonne d'alumine neutre ce qui a permis de conduire par exemple aux composés **28a** et **28b** avec des rendements respectifs de 74% et 34%. Dans l'étape suivante, le groupement Boc est introduit à l'aide du N-(*tert-*butoxycarbonyloxy)succinimide pour donner par exemple les macrocycles triazotés trisubstitués **29a** et **29b.** Enfin les amines portant les groupements Moz sont déprotégées par hydrogénolyse. L'utilisation du Pd/C à 10% comme catalyseur n'a pas permis de déprotéger les amines lorsque le milieu réactionnel est hydrogéné pendant 48 h sous 3,45 bars de pression. En revanche, l'utilisation du catalyseur de Pearlman (Pd(OH)₂/C) a permis l'obtention des macrocycles triazotés monoprotégés. Pour isoler facilement ces produits, les sels d'hydrochlorure ont été formés par addition d'une petite quantité d'acide chlorhydrique à froid, ces derniers étant recueillis par précipitation dans l'éther diéthylique. Cette méthodologie permet désormais de préparer à l'échelle de plusieurs grammes les composés (147TACN, 159TACD, 148TACD et 148TACU) monoprotégés avec un groupement Boc par exemple **30a** et **30b.**

La méthode de synthèse des macrocycles triazotés monoprotégés selon l'invention comprend donc les étapes suivantes :
(i) protection de deux des trois amines du macrocycle triazoté par un groupe protecteur Gp₁ sensible à l'hydrogénation ;
(ii) purification des composés disubstitués sur colonne choisie parmi : une colonne d'alumine basique ; une colonne de silice ayant subi un traitement préalable avec de la triéthylamine ou de l'ammoniac; une colonne d'alumine basique ou neutre, cette dernière étant préférée ;
(iii) protection de la troisième amine dudit macrocycle triazoté par un deuxième groupe protecteur Gp₂ insensible à l'hydrogénation ;
(iv) déprotection des amines protégées par les groupes protecteurs Gp₁ par hydrogénation, de préférence avec le catalyseur de Pearlman ;
(v) purification des produits monoprotégés, par exemple par formation de sels de dihydrochlorure par ajout d'acide chlorhydrique à froid et précipitation dans l'éther diéthylique.

Gp₁ et Gp₂ sont choisis parmi les groupes protecteurs des amines, qui sont connus de l'homme du métier comme décrit dans Protecting Groups P. J. Kocieński, Corrected Edition (2000) 185-244: Carbobenzyloxy (Cbz), *p*-Méthoxybenzylcarbonyle (Moz) *tert*-Butyloxycarbonyle (Boc), 9-Fluorénylméthyloxycarbonyle (Fmoc), Acétyle (Ac), Benzoyle (Bz), Benzyle (Bn), p-Méthoxybenzyle (PMB), 3,4-Diméthoxybenzyle (DMPM), p-méthoxyphenyle (PMP), Tosyl (Ts), Nosyl (Ns), trifluoroacetamides, alkoxycarbonyles, allyloxycarbonyle (Aloc), 2-(triméthylsilyl)éthoxycarbonyle, 2,2,2-trichloroéthoxycarbonyle (Troc), 2-(triméthylsilyl)éthoxycarbonyle (Teoc), B-(triméthylsilyl)éthanesulfonyl (SES), benzhydryl, trityl, 9-phénylfluorényl et N-silyl imines.

Gp₁ est préférentiellement un groupe Cbz ou Moz.

En ce qui concerne Gp₂, les groupes Fmoc, Tosyl, Nosyl, Boc(triméthylsilyloxy) sont préférés, le groupe Boc étant encore plus préféré.

Le composé obtenu est un composé de formule suivante : 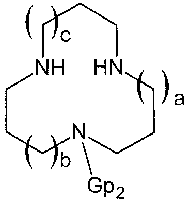 dans laquelle a=b=c=0 ou a=b=c=1. Le composé dans lequel Gp₂ est le groupe Boc est préféré. On comprend à la lecture de la méthode de synthèse décrite juste ci-dessus, et en particulier de l'étape (v), que les macrocycles triazotés monoprotégés selon l'invention se présentent sous la forme de sel de dihydrochlorure.

La préparation des agents complexants de formule (I) dans laquelle R₃, R₄ ou R₅ est un groupe -L-G nécessite l'utilisation de macrocycles particuliers dont le carbone 2 ou 3 est substitué par le groupe -L-G, ou par un précurseur de ce groupe, notamment un précurseur dans lequel le groupe réactif est sous forme protégée. La synthèse de certains de ces azamacrocycles est décrite notamment dans la demande internationale de brevet WO 2005/058877, ainsi que par Hovinen et al (Tetrahedron Letters 46 (2005) 4387-4389). La méthodologie développée dans ces deux références est basée sur la condensation entre un dérivé trinosylé et un diol en utilisant une réaction de Mitsunobu. Cette méthode présente cependant l'inconvénient de générer beaucoup de sous-produits pas toujours facile à séparer du milieu réactionnel mais surtout ne permet pas la synthèse d'azamacrocycles de type 147TACN fonctionnalisé comme décrit dans l'article de Honiven. Une autre stratégie dans des conditions moins douces a été publiée par le groupe de Wu et al (Synthetic Communications, 34 (2004) 845-851, Synthetic Communications 25 (1995) 1427-1437, Chinese Journal of Chemistry, 16 (1998) 538-541) permettant d'obtenir les azamacrocycles en condensant un dérivé tritosylé sur le diol correspondant avec des rendements voisins de 50%. Un inconvénient majeur de cette deuxième méthodologie réside en la déprotection des groupements tosyles qui est en général réalisée dans des conditions très dures (100°C, acide bromhydrique, dans l'acide acétique 33%, ou bien à 100°C dans l'acide sulfurique concentré 96%).

Les agents complexants dont le groupe -LG est lié au macrocycle ont donc ici été préparés selon une stratégie basée sur la condensation d'un dérivé trinosylé avec un dérivé dibromé en utilisant le carbonate de césium comme base dans de l'acétonitrile. Les conditions expérimentales utilisées sont proches de celles décrites par Fukuyama et al (Tetrahedron, 58 (2002) 6267-6276) et sont décrites dans le SCHEMA 4 et la partie expérimentale.

### Synthèse des chromophores

Les chromophores peuvent être fabriqués selon une approche telle que présentée dans le schéma A, et dans laquelle les groupes R₁ et R₂ seront éventuellement protégés, selon leur nature, en particulier s'ils sont des groupes carboxylates, phosphates ou des groupes réactifs.

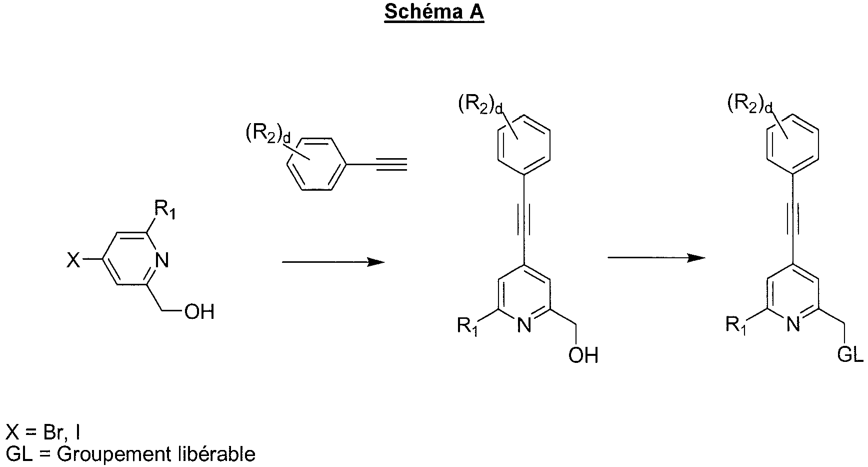

Cette approche a été employée pour la synthèse de chromophores similaires à ceux utilisables pour la préparation des agents complexants selon l'invention, comme par exemple par :
- Picot et al (Inorganic Chemistry 46 (2007), 2659-2665 : chromophore phényléthynylpyridine, groupe hexyloxy sur le groupement phényle ;
- Picot et al (Journal of American Chemical Society 130 (2008) 1532-1533): chromophore phényléthynylpyridine portant un groupe tris(triéthylèneglycol)phényl assurant la solubilité des composés ; et
- D'Aléo et al ( Sensitization of Eu(III) luminescence by donor-phenylethynyl-functionalized DTPA and DO3A macrocycles, C.R. Chimie 2010): phényléthynylpyridine portant un groupe OPeg).

La préparation des réactifs nécessaires à cette synthèse relève des connaissances générales de l'homme du métier, lorsque ces réactifs ne sont pas disponibles dans le commerce.
La synthèse des alcynes substitués est décrite dans les SCHEMA 1 et 13, ainsi que dans la partie expérimentale.
La synthèse des chromophores « carboxylates » est décrite dans le SCHEMA 2 et dans la partie expérimentale.

La synthèse des chromophores « phosphinates » est décrite dans le SCHEMA 3 et dans la partie expérimentale.

### Greffage des chromophores sur le macrocycle

La synthèse des agents complexants de formule (I) ne comportant pas de groupe réactif -L-G peut être réalisée par alkylation d'un macrocycle triazoté avec un dérivé du chromophore souhaité (Schéma B) comportant un groupement libérable, tel qu'un groupe mésylate (-OMs), triflate (-OTf), tosylate (-OTs), ou encore un atome de chlore, brome ou iode. Il peut être nécessaire de protéger les groupes R₁ susceptibles de réagir lors de l'alkylation, de poser des problèmes de solubilité ou bien encore de polarité. Par exemple lorsque R₁ est un groupe carboxylate ou phosphinate, des dérivés estérifiés correspondants peuvent être utilisés.

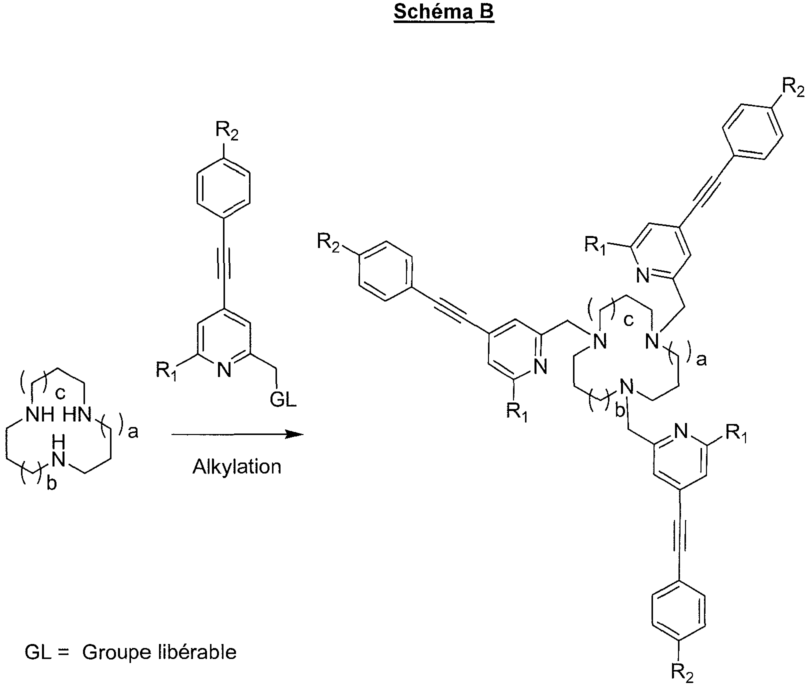

Cette synthèse est décrite de manière plus détaillée dans le SCHEMA 5 et la partie expérimentale.

La même approche est utilisée lorsque le groupe -L-G est directement lié au macrocycle, par exemple pour les agents complexants de formule (I) dans lesquels l'un des groupes R₃, R₄ ou R₅ est un groupe -L-G.

La préparation de complexes dont l'un des chromophores comprend un groupe réactif -L-G peut être réalisée de la même façon, par alkylation d'un macrocycle triazoté comportant une fonction amine protégée, préparé par exemple selon un procédé tel que celui décrit plus haut. Une fois les deux chromophores greffés sur le macrocycle, le troisième chromophore portant le groupe -L-G (compris dans le groupe R_{2'}) pourra être à son tour greffé après déprotection de l'amine protégée (schéma C). 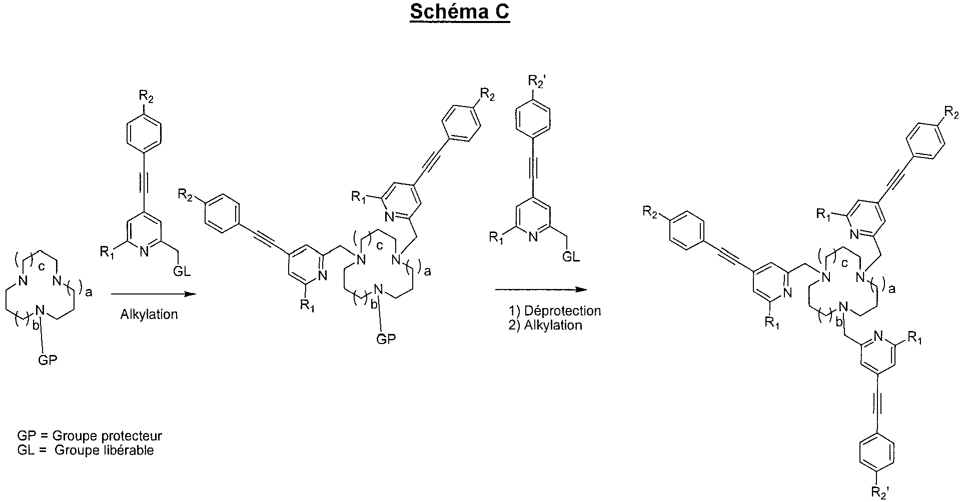 (R'₂ est tel que défini pour R₂ ci-dessus).

Cette synthèse est décrite de manière plus détaillée dans les SCHEMAS 6, 7, 8, 9 et la partie expérimentale.

Une stratégie inverse peut être envisagée dans laquelle on introduit d'abord sur un azamacrocycle diprotégé un chromophore comprenant un groupe réactif L-G masqué en utilisant une réaction d'alkylation. Les deux groupements protecteurs du macrocycle orthogonaux au groupe protégeant la fonction réactive L-G sont éliminés et les deux autres chromophores sont ensuite introduits par simple alkylation conduisant au squelette de l'agent complexant (Schéma D). 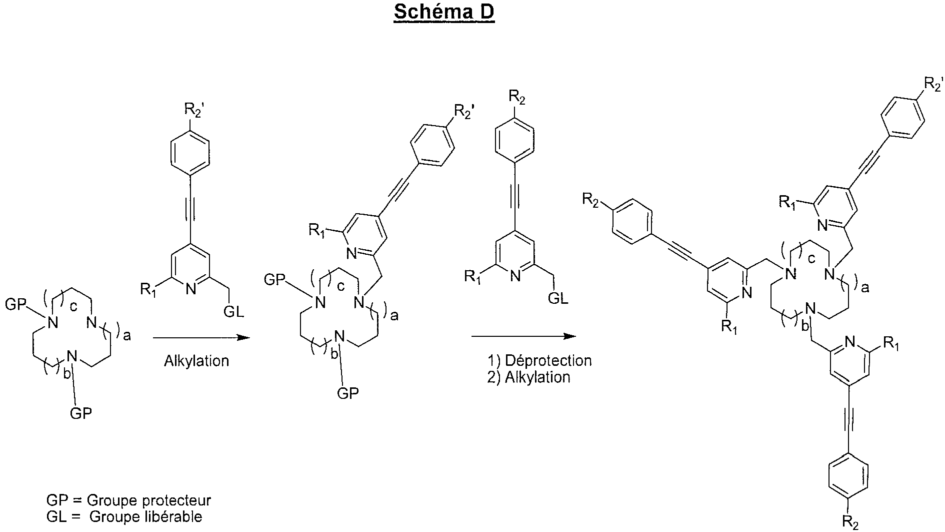 (R'₂ est tel que défini pour R₂ ci-dessus).

La synthèse des composés selon l'invention est détaillée de manière précise dans la partie expérimentale suivante.

### Description des Figures

La Figure 1 représente la luminescence des composés de l'invention en présence de cations divalents ou d'EDTA.
La Figure 2 représente le spectre d'excitation des composés de l'invention dans du tampon HEPES.

### Abréviations utilisées :

Ms : Mésyl
Boc : *tert*-Butyloxycarbonyle
PEG : polyéthylène glycol
NHS : N-hydroxysuccinimide
RMN : résonance magnétique nucléaire
CCM : chromatographie en couche mince
DMF : diméthylformamide
THF : tétrahydrofurane
DCM : dichlorométhane
HPLC : chromatographie liquide à haute performance
HRMS : spectroscopie de masse à haute résolution
TEA / Et₃N: triéthylamine
DIPEA: diisopropyléthylamine
NBS: N-bromosuccinimide
NIS : N-iodosuccinimide
TsCl : chlorure de tosyle
MeCN : acétonitrile
MeOH : méthanol
SM : spectrométrie de masse
ESI : ionisation par électronébulisation
*m*-CPBA : acide métachloroperbenzoïque
EtOH : éthanol
Moz-ON : ((2-(4-méthoxybenzyloxycarbonyloxy imino)-2-phénylacétonitrile)
Boc-OSu: N-(*tert*-butoxycarbonyloxy)succinimide
TFA : trifluoro acetic acid
Ph: phényl
TMS = triméthylsilyl
TSTU: tétrafluoroborate de O-(N-Succinimidyl)-1,1,3,3-tétraméthyluronium
DCC: dicyclohexylurée
PtF: point de fusion
δ: déplacement chimique
ppm : partie par million
Hz : Hertz
min : minute
h : heure
cAMP : adénosyl monophosphate cyclique
Mops : 3-(N-morpholino)propanesulfonic acid
HTRF: Fluorescence Homogène en Temps Résolu

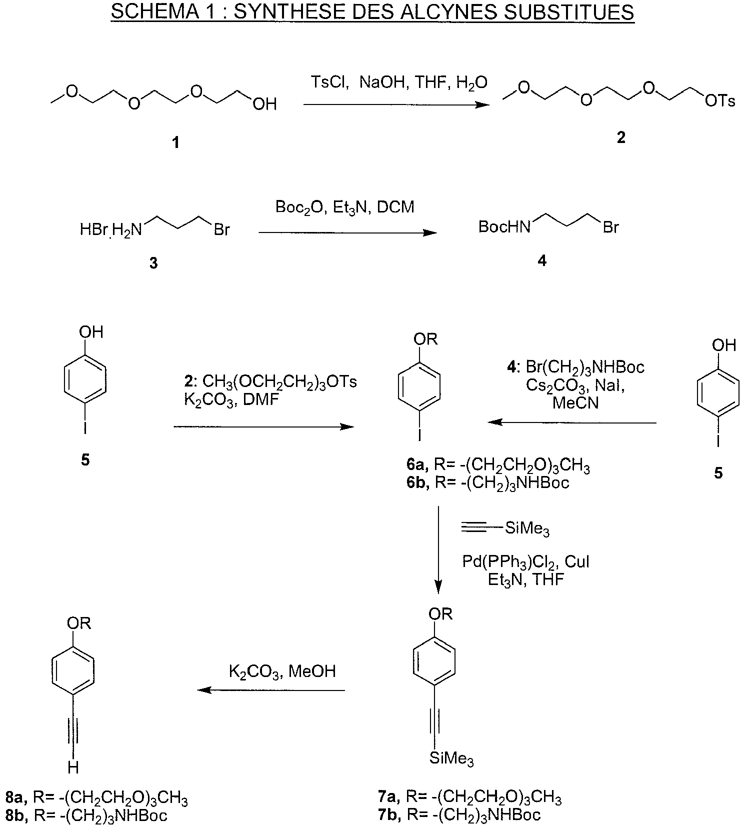

La stratégie générale pour synthétiser les chromophores implique un couplage de Sonogashira qui a largement était décrit dans la littérature (Sonogashira et al Tetrahedron Letters, 50 (1975) 4467-4470, Rossi et al Organic Préparation and Procédure International 27 (1995) 129-160. Cette réaction permet de coupler un alcyne vrai avec un halogénure aromatique (de préférence un dérivé iodé ou bromé) ou un tosyle. Les alcynes vrais (**8a** et **8b**) ne sont pas disponibles commercialement. Ils ont été préparés selon la synthèse décrite dans le schéma 1 et détaillés dans la partie expérimentale. Ainsi le dérivé polyéthylène glycol **1** a été activé sous forme de composé tosylé. La protection par un groupement protecteur Boc de l'amine **3** a été nécessaire pour éviter la polyalkylation. Les deux alkylations de l'iodophénol ont conduits aux deux éthers phénoliques **6a** et **6b** avec des rendements convenables de 51 et 75% respectivement. Les couplages de Sonogashira en utilisant le triméthylsilylacétylène suivis de la déprotection du groupement TMS aux deux alcynes vrais souhaités **8a** et **8b.**

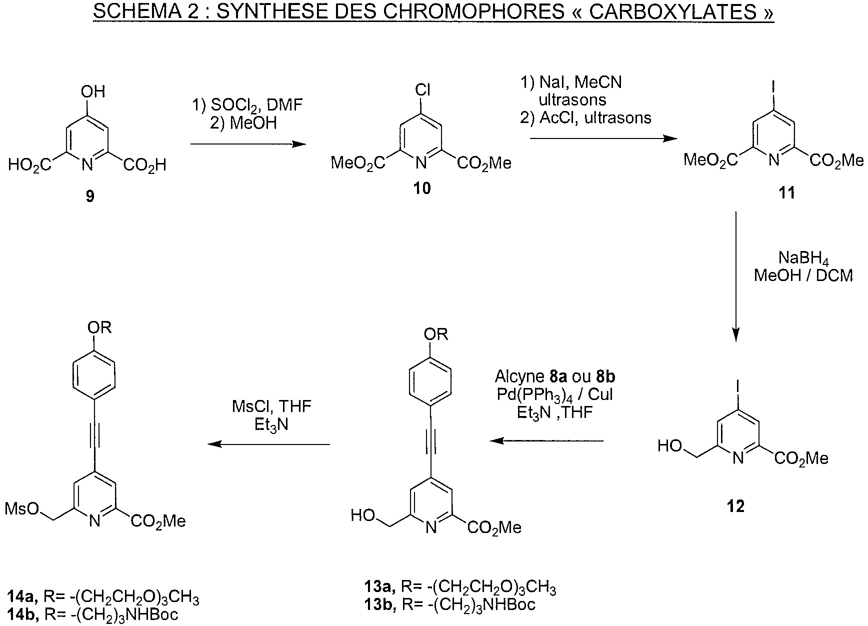

La synthèse des chromophores « carboxylates » est décrite dans le schéma 2. L'acide chélidamique **9** est converti en diester chloré **10** comme il a été décrit par Maury et al (Inorganic Chemistry 50 (2011) 4987-4999). L'échange chlore-iode est réalisé en présence d'iodure de sodium sous ultrasons permettant de conduire au composé **11.** L'étape clé de cette synthèse consiste à réduire sélectivement le diester **11** en présence de borohydrure de sodium à 0°C pour obtenir le monoalcool monoester avec un rendement de 60%. Finalement le squelette du chromophore est obtenu en utilisant une deuxième réaction de Sonogashira conduisant ainsi aux composés **13a** et **13b.** Enfin l'activation des alcools a été réalisée *via* une réaction de mésylation.

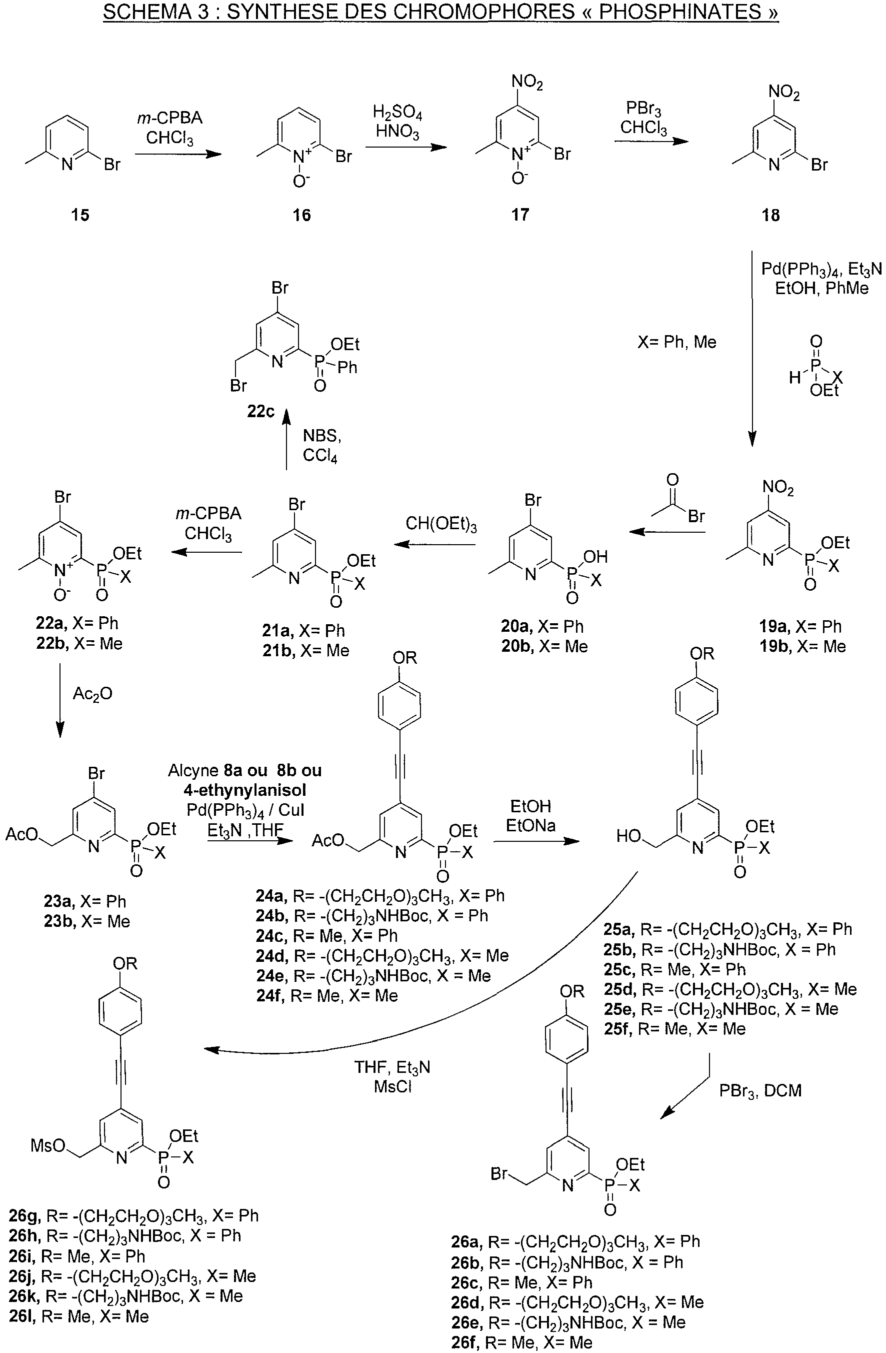

La préparation des chromophores « phosphinates » est décrite dans le schéma 3. L'objectif de la synthèse consiste à disposer d'un dérivé de la pyridine trisubstitué orthogonalement. Ainsi la 2-bromo-6-méthylpyridine disponible commercialement est oxydé en analogue N-oxyde **16** simplement par oxydation en présence d'acide métachloroperbenzoïque (m-CPBA). L'activation de cet hétérocycle azoté permet d'introduire facilement le groupement nitro en position 4, correspondant au troisième groupement fonctionnel. Le N-oxyde est alors supprimé en utilisant le tribromure de phosphore conduisant au dérivé pyridinyle libre. L'introduction du groupement phosphinate a été réalisée en utilisant un couplage au palladium en présence d'acide phényl phosphinique commercial. En ce qui concerne la pyridine méthylphosphinate, elle est obtenue par couplage du dérivé éthylméthyl phosphinate, lui-même obtenu par hydrolyse du diéthylméthyl phosphonite en utilisant 1 équivalent d'eau. La mise au point de ces couplages s'est avérée particulièrement difficile et finalement a permis d'obtenir les composés souhaités **19a** et **19b** en utilisant les micro-ondes comme source d'énergie mais également par voie thermique. La suite de la description de la synthèse a été réalisée par exemple sur la série des phényl phosphinates mais les résultats sont extrapolables aux méthyl phosphinates puisque ces deux types de composés présentent des réactivités identiques. La substitution du groupement nitro par un atome de brome a été réalisée en présence de bromure d'acétyle. Bien que ce réactif soit efficace dans cette réaction, il engendre la formation d'acide bromhydrique qui hydrolyse le groupement phosphinate. Celui-ci est réintroduit immédiatement après en utilisant l'orthoformiate d'éthyle. La fonctionnalisation du méthyle en position 6 de la pyridine est réalisée en utilisant un réarrangement décrit par Ginsburg et al (Journal American Chemical Society 79 (1957) 481-485). La pyridine est oxydée une seconde fois en utilisant les mêmes conditions décrites précédemment. Cette fonction N-oxyde est suffisamment nucléophile pour réagir avec l'anhydride acétique qui subit intermédiairement un réarrangement permettant ainsi d'introduire un groupement acétate en position 6 conduisant aux l'intermédiaires clés de la synthèse **23a** et **23b.** Le squelette du chromophore phosphinate est élaboré en utilisant la réaction de Sonogashira en présence des alcynes vrais décrits précédemment. L'ester (acétate) est hydrolysé en alcool correspondant qui est converti en dérivé bromé conduisant aux chromophores activés phosphinate **26a-f.** Alternativement il est également possible de préparer les dérivés mésylés correspondants comme décrit précédemment pour les chromophores « carboxylates » conduisant ainsi aux dérivés **26g-I.**

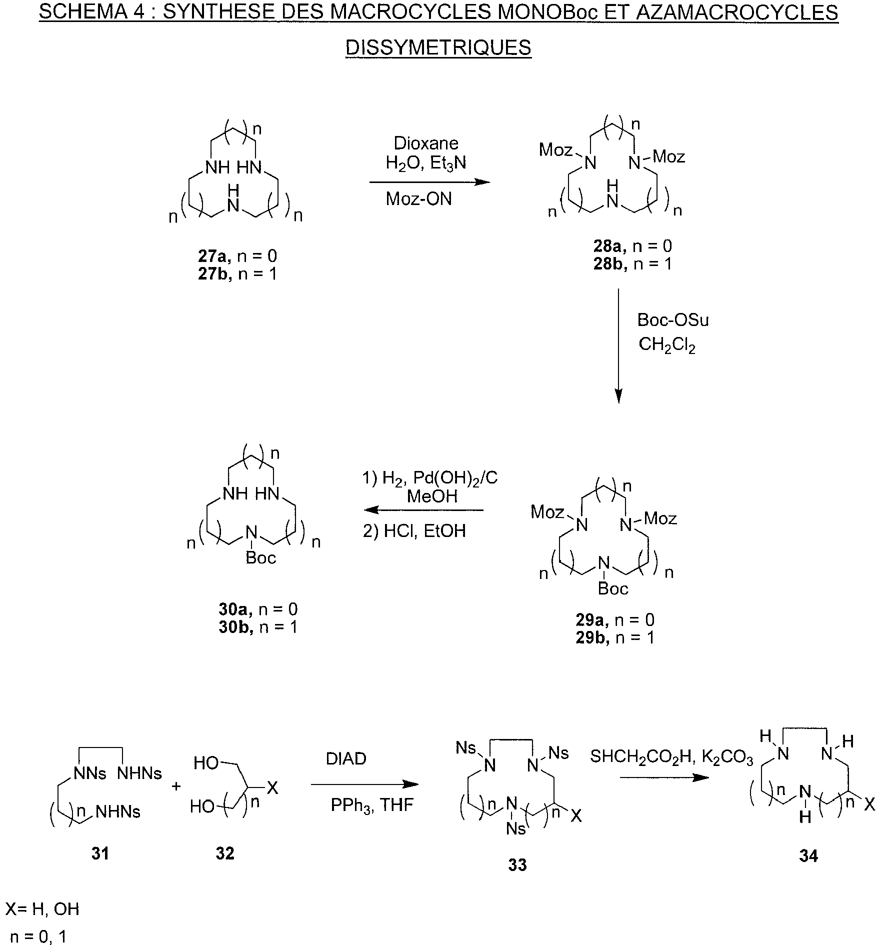

En ce qui concerne les azamacrocycles (série **34**) dissymétriques portant ou non un groupe fonctionnel X, ils ont été préparés par condensation du dérivé diol avec un composé dinosylé **31** en utilisant les conditions de Mitsunobu. La déprotection des groupements nosyles est effectuée classiquement en présence d'acide thioglycolique et de carbonate de potassium. Cette séquence réactionnelle permet d'obtenir par exemple toutes les déclinaisons des azamacrocycles (147TACN, 159TACD, 148TACD et 148TACU) substitués ou non.

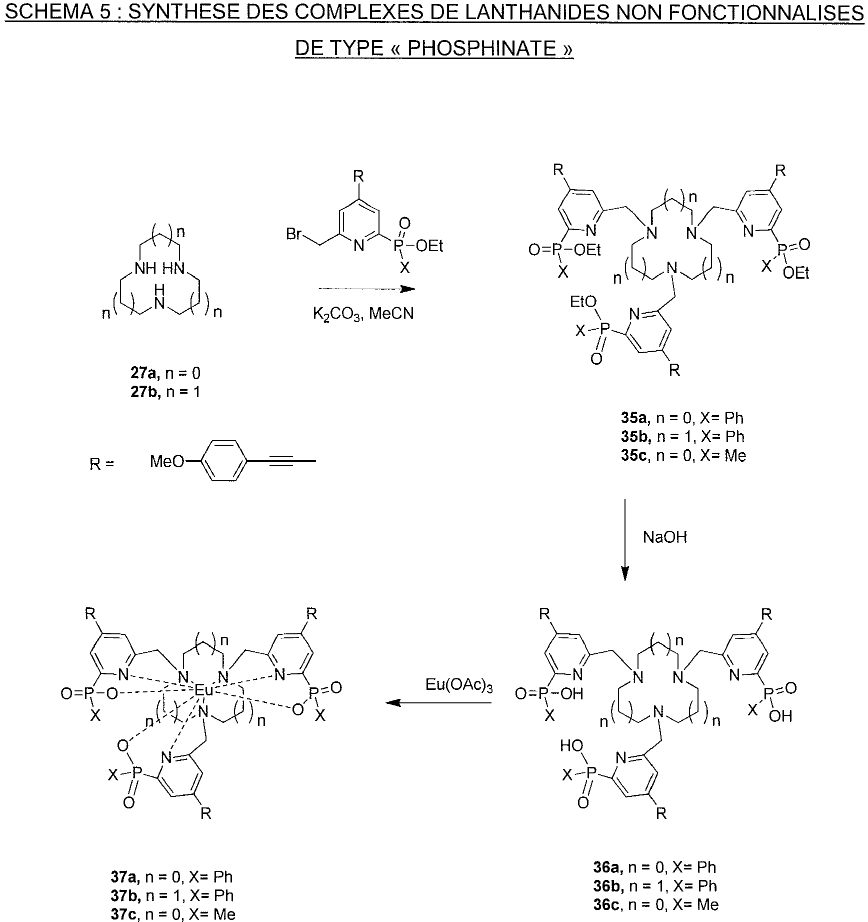

Un premier exemple de complexe phosphinate non fonctionnalisé est décrit dans le schéma 5. L'azamacrocycle est condensé sur le chromophore bromé phosphinate en présence de carbonate de potassium. Les esters de phosphinate sont hydrolysés avec de l'hydroxyde de potassium ou de lithium conduisant aux ligands qui sont ensuite utilisés pour former les complexes d'europium **37a-c.**

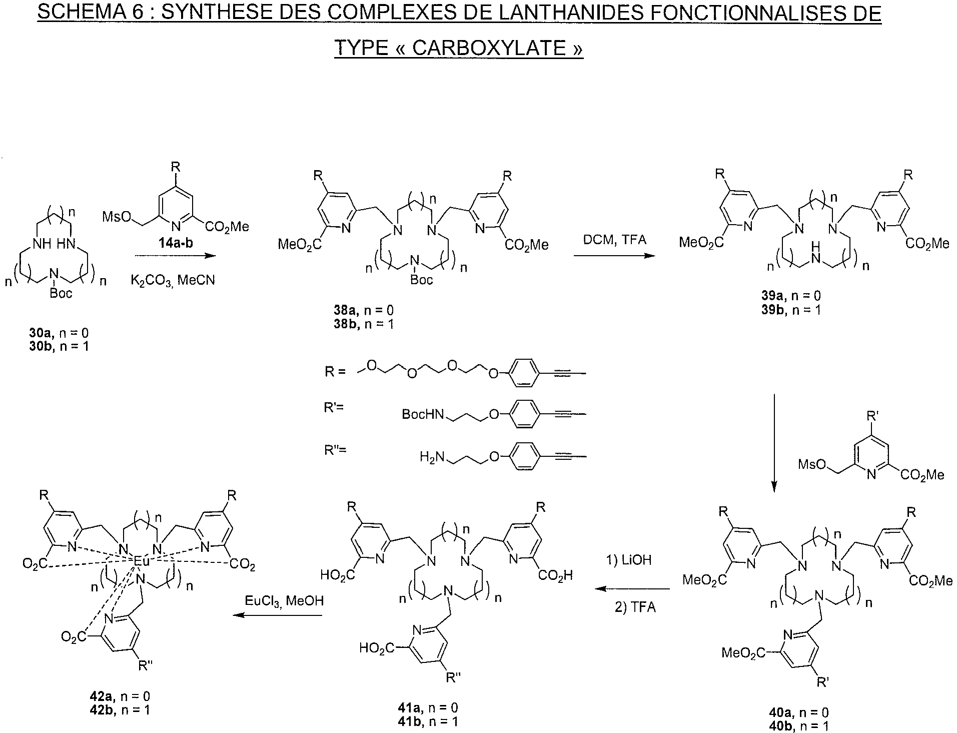

Les complexes d'europium sont préparés selon les schémas 6 et 7. A partir des macrocycles mono-substitués 147TACN et 159TACD, deux unités « chromophores carboxylates ou phosphinate « OPEG» sont condensés conduisant aux dérivés **42a-b** ou **47a-c.** En terme de rendement, des résultats comparables ont été obtenus avec les chromophores phosphinates bromés ou mésylés. Le groupement protecteur Boc est déprotégé en présence d'acide trifluoroacétique suivi de l'alkylation du troisième chromophore comportant un groupement NH₂ masqué, utile pour la conjugaison à une biomolécule. Les trois fonctions esters (carboxylate ou phosphinate) sont ensuite hydrolysées et le groupement Boc est éliminé en milieu acide. La formation du complexe de lanthanide est réalisée en faisant réagir les ligands **41** et **46** avec les sels de lanthanides correspondants et en l'occurrence le chlorure ou l'acétate d'europium.

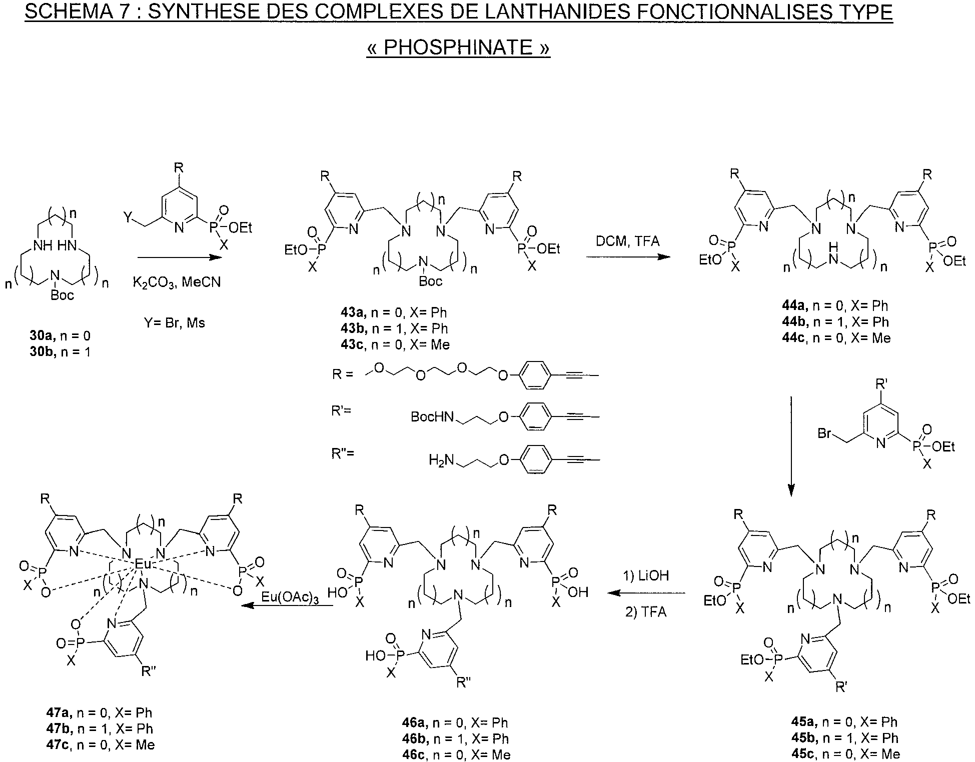

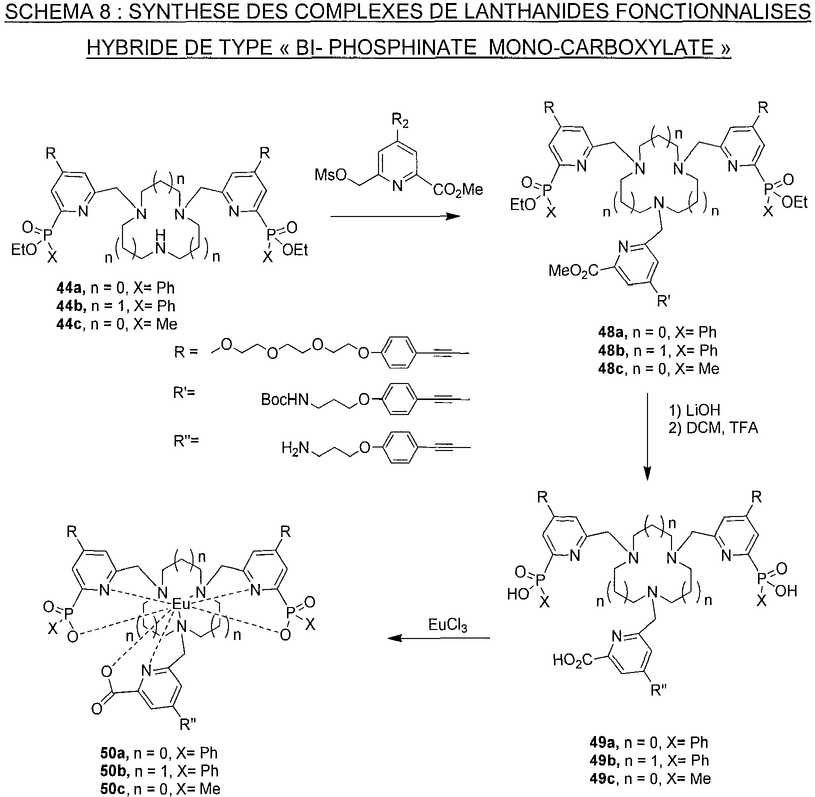

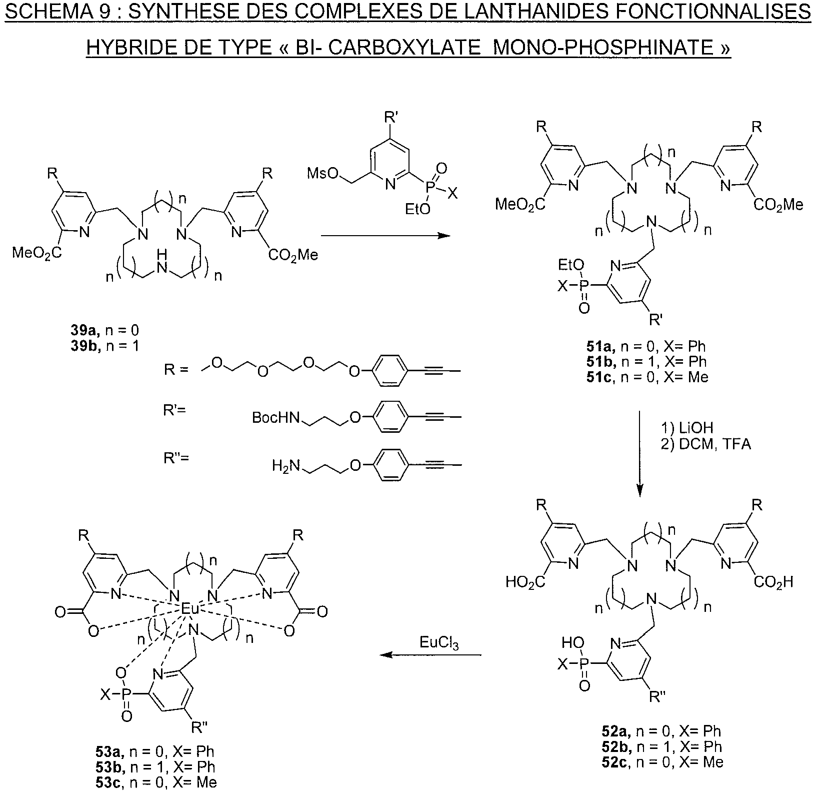

La méthodologie pour préparer les complexes hybrides est décrite sur les deux schémas 9 et 10. Elle consiste à utiliser des synthons préparés précédemment dans le cadre de la synthèse des complexes phosphinates ou carboxylates.

A partir des dérivés dialkylés (dicarboxylate -OPEG ou diphosphinate -OPEG) sont condensés les dérivés mésylés phosphinates (pour les dicarboxylates) ou bien les dérivés mésylés carboxylates (pour les diphosphinates). Ces réactions conduisent aux ligands hybrides qui sont ensuite hydrolysés et complexés avec la terre rare et en particulier avec l'europium en utilisant le chlorure ou l'acétate d'europium.

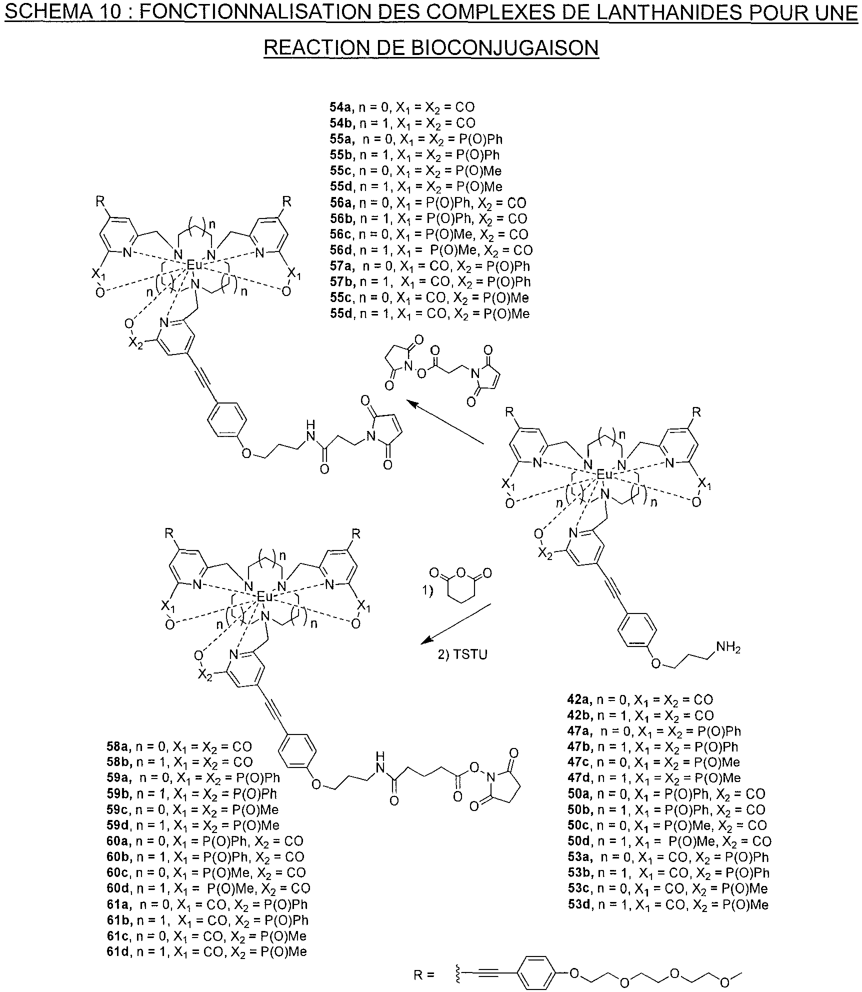

Le complexe fonctionnalisé NH₂ n'est pas utilisable en l'état pour être bioconjugué à une biomolécule : protéines, anticorps, peptides, sucres... Pour réaliser ces bioconjugaisons, il est nécessaire de convertir ce groupement NH₂ en un groupe fonctionnel biocompatible. Ci-dessous est décrite la méthodologie :

Pour les complexes maléimides, ils sont directement préparés en utilisant un réactif commercial bifonctionnel : le N-[β-maléimidopropyloxy] succinimidyle, ce qui conduit à la fonctionnalisation souhaitée. En ce qui concerne l'obtention des complexes fonctionnalisés sous forme d'ester de NHS, il est nécessaire de convertir préalablement la fonction amine en dérivé d'acide carboxylique (condensation de l'anhydride glutarique sur le groupement amino), puis d'activer cette fonction sous forme d'ester de NHS en utilisant soit du TSTU, soit les conditions classiques DCC, NHS.

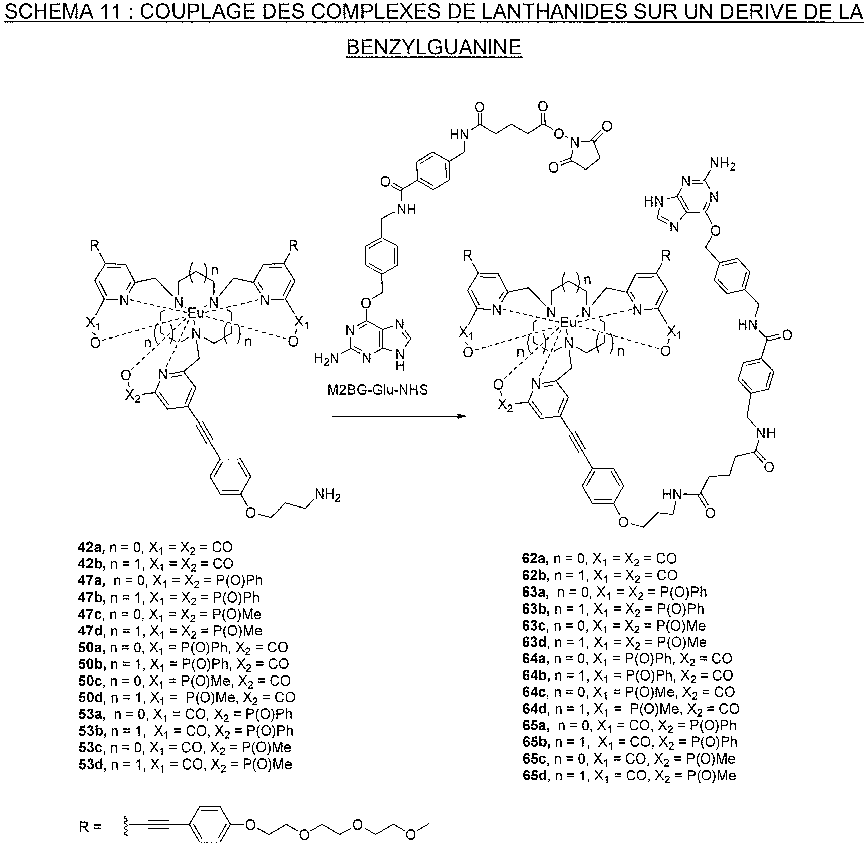

La condensation des complexes sur le dérivé M2BG-Glu-NHS dont la synthèse est décrite dans la demande de brevet WO2010034931, conduit aux complexes conjugués qui peuvent être utilisés dans une réaction de marquage de la protéine Snap.

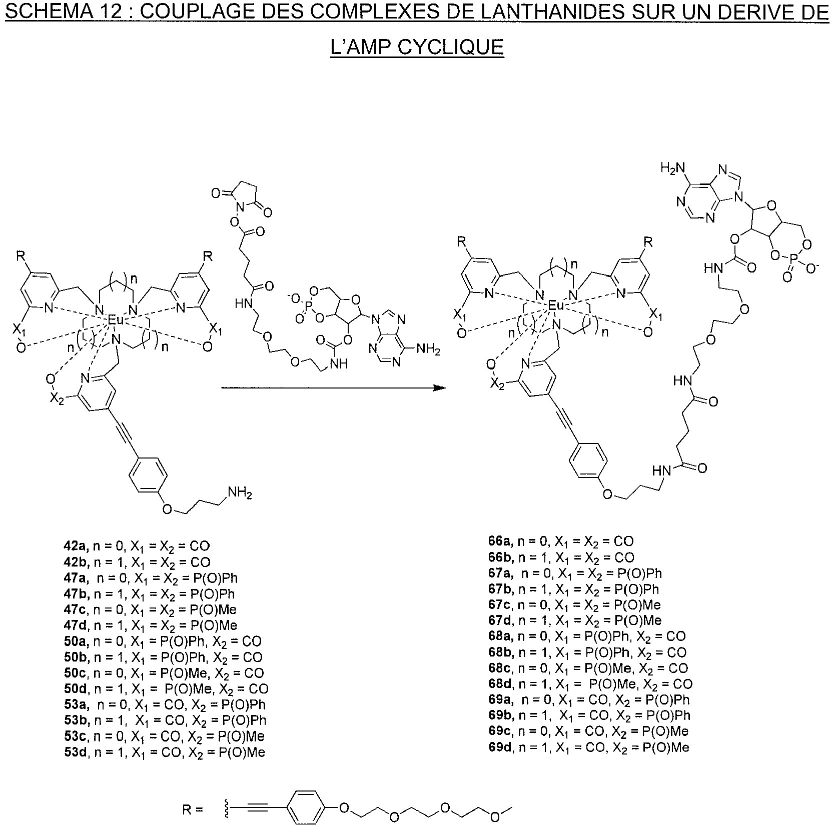

La condensation des complexes sur le dérivé cAMP permet d'obtenir toute une série de complexes conjugués au cAMP. Ces complexes présentent tous les mêmes propriétés photophysiques et peuvent être utilisés dans les tests HTRF développés par Cisbio Bioassays.

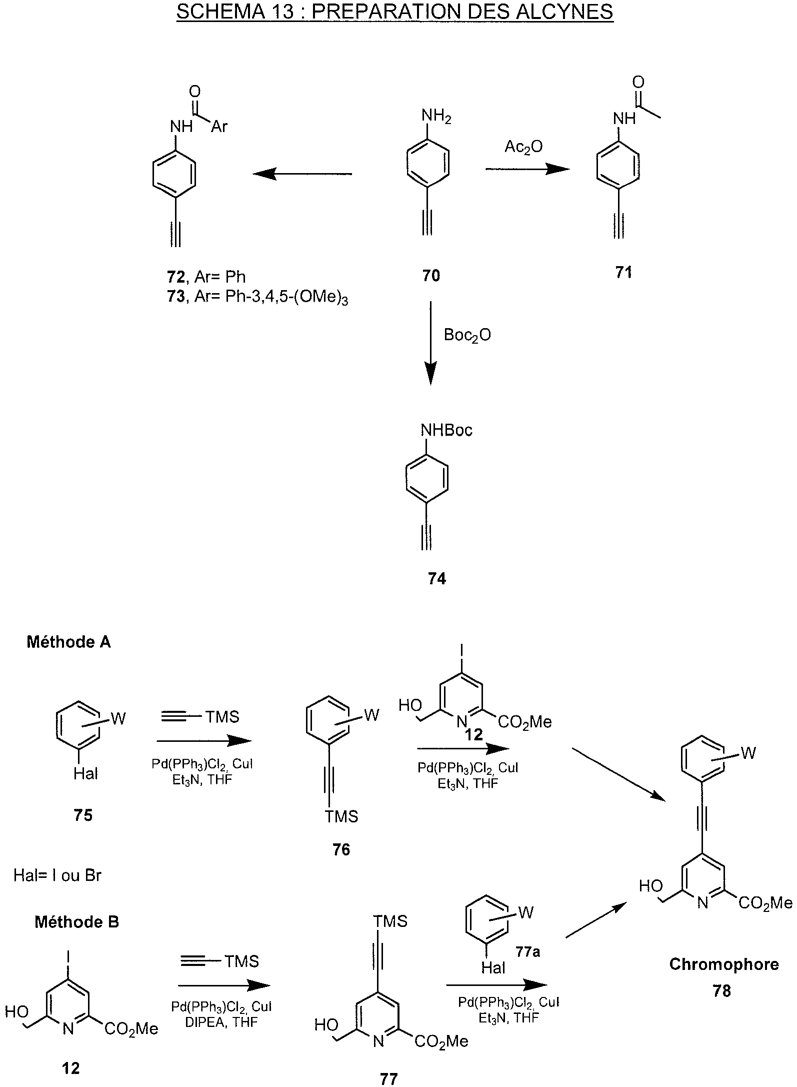

La nature du groupe W dépend du chromophore à synthétiser et n'a pas d'influence sur la synthèse des chromophores.

En ce qui concerne le dérivé acétylénique acétylé **71,** il a été obtenu par simple réaction d'acétylation en présence d'anhydride acétique en utilisant le précurseur correspondant commercial.

Les chromophores qui ne sont pas disponibles dans le commerce ont été préparés simplement selon la méthode A ou B.

La méthode A se décompose en trois étapes : halogénation du dérivé aromatique commercial suivi d'un couplage de Sonogashira avec le triméthylsilylacétylène conduisant au dérivé **76,** la déprotection du groupement protecteur triméthylsilyle peut être réalisée *in situ* ou non lors de la deuxième réaction de Sonogashira qui permet d'obtenir le chromophore **78.** Selon la méthode B, la séquence réactionnelle est réalisée dans l'ordre inverse, à savoir : réaction de Sonogashira sur le dérivé iodopyridine **12** conduisant au dérivé **77** qui est déprotégé *in situ* lors du deuxième couplage conduisant ainsi au chromophore **78.**

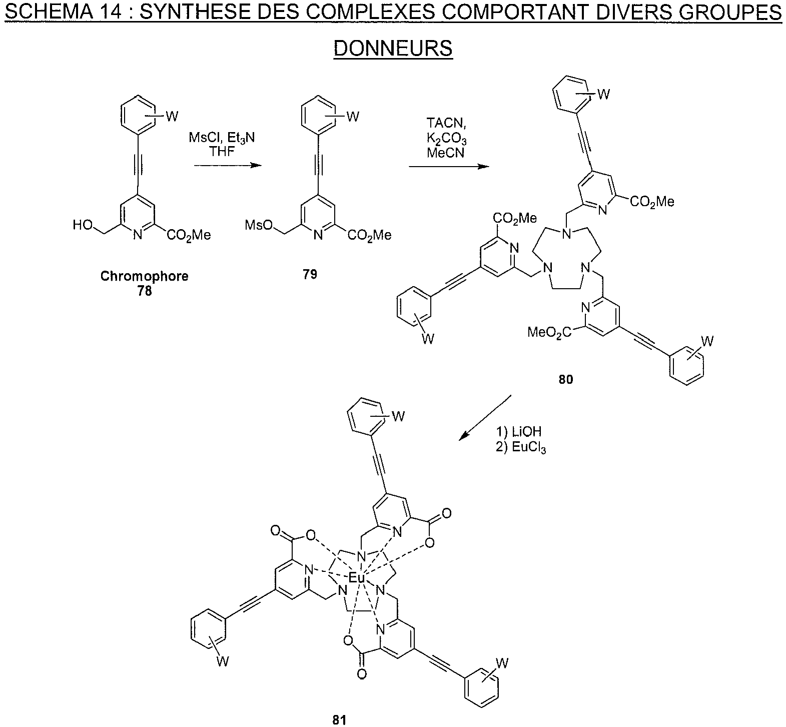

La méthodologie générale pour l'obtention de complexes symétriques est décrite dans le schéma 14: les chromophores sont mésylés puis introduits sur le macrocycle azoté (ici par exemple le triazacyclononane). L'hydrolyse des trois fonctions esters en acides carboxyliques puis la complexation avec l'europium conduit au complexe correspondant. Cette approche permet rapidement de discriminer un certain nombre de complexes présentant les propriétés photophysiques recherchées et évite la préparation plus fastidieuse de complexes dissymétriques fonctionnalisés. En effet, il a été déterminé que les propriétés photophysiques d'un complexe d'europium (complexe TACN ou TACD) symétrique (trois chromophores identiques) étaient les mêmes que celles d'un complexe d'europium dissymétrique (deux chromophores identiques et un chromophore portant un groupe -LG permettant la conjuguaison avec une biomolécule).

### PARTIE EXPERIMENTALE

### Informations générales

### Chromatographie

Les chromatographies sur couches minces ont été effectuées sur des plaques de gel de silice Merck 60 F₂₅₄ sur feuille d'aluminium ou sur des plaques d'oxyde d'aluminium neutre Merck 60 F₂₅₄ (type E) sur feuille d'aluminium.

Les chromatographies liquides à haute performance (HPLC) analytiques et préparatives ont été effectuées sur deux appareils :
- HPLC Analytique : ThermoScientific, pompe quaternaire P4000, Détecteur UV 1000 à lampe au deutérium (190-350 nm), colonne analytique Waters XBridge C18, 3.5 µm, 4.6 × 100 mm.
- HPLC Préparative : Shimadzu, 2 pompes LC-8A, détecteur UV à barrette de diodes Varian ProStar, colonne préparative Waters XBridge prép. C18, 5 µm: 19 × 100 mm ou 50 × 150mm.

Les chromatographies sur colonne de silice ont été réalisées sur gel de silice Merck 60 (0.040-0.063 mm). Les chromatographies sur colonne d'alumine ont été réalisées sur oxyde d'aluminium Sigma-Aldrich, neutre, activé, Brochmann I.

### Spectroscopie

### a. Résonance magnétique nucléaire

Les spectres RMN (¹H, ¹³C et ³¹P) ont été réalisés à l'aide d'un spectromètre Bruker Avance 400 MHz NanoBay (aimant de 9,4 Teslas), muni d'une sonde de mesure BBFO, multi noyaux de diamètre de 5 mm, de gradient Z et de lock ²H. Les déplacements chimiques (δ) sont exprimés en partie par million (ppm). Les abréviations suivantes sont utilisées :
s : singulet, se : singulet élargi, d : doublet, t : triplet, q : quadruplet, m : multiplet, dd : doublet dédoublé, td : triplet dédoublé, qd : quadruplet dédoublé, ddd : doublet de doublet dédoublé.

### b. Spectrométrie de masse

Les spectres de masse (LC-MS) ont été réalisés à l'aide d'un spectromètre Waters ZQ 2000 simple quadipôle à source multimode ESI/APCI équipé de colonne Waters XBridge C18, 3.5µm, 4.6 × 100mm.

### c. Spectrométrie de masse haute résolution

Les analyses ont été effectuées avec un spectromètre de masse QStar Elite (Applied Biosystems SCIEX) équipé d'une source d'ionisation à pression atmosphérique (API) assistée pneumatiquement. L'échantillon a été ionisé en mode electrospray positif dans les conditions suivantes : tension electrospray (ISV) : 5500 V ; tension d'orifice (OR) : 20 V ; pression du gaz de nébulisation (air) : 20 psi. Le spectre de masse haute résolution (MS) a été obtenu avec un analyseur temps de vol (TOF). La mesure de masse exacte a été effectuée en triplicat avec un double étalonnage interne.

### Divers

Appareil à point de fusion : les points de fusion ont été réalisés en utilisant un appareil BUCHI melting point B-540.

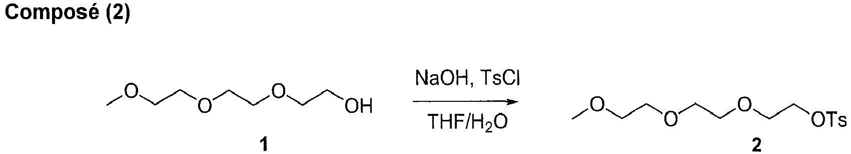

A une solution de triéthylèneglycolmonométhyléther **1** (33,2 g, 0,2 mol) dans du tétrahydrofurane (50 mL) a été ajoutée au goutte à goutte une solution d'hydroxyde de sodium (14,56 g d'hydroxyde de sodium dans 60 mL d'eau, 0,36 mol). Le milieu réactionnel a été refroidi à 0°C et une solution de chlorure de toluène-4-sulfonyle dans du tétrahydrofurane (38,5 g dans 60 mL, 0,2 mol) a été ajoutée au goutte à goutte. A la fin de l'ajout, le milieu réactionnel a été agité à température ambiante pendant 12 h. L'avancement de la réaction a été suivi par CCM. Après cette période, la réaction était totale. Le milieu réactionnel a été extrait avec de l'éther diéthylique (3 x 100 mL). Les phases organiques ont été regroupées, séchées sur sulfate de sodium et filtrées. Le solvant a été éliminé sous pression réduite pour conduire au composé **2** sous forme d'une huile légèrement jaune. Le composé était suffisamment pur pour être utilisé dans la suite de la synthèse sans purification supplémentaire (57,22 g, 89%). RMN ¹H (200 MHz, CDCl₃): δ: 7,77 (d, *J* = 8,3 Hz, 2H), 7,31 (d, *J* = 8,3 Hz, 2H), 4,13 (m, 2H), 3,57 (m, 10H), 3,33 (s, 3H), 2,41 (s, 3H).

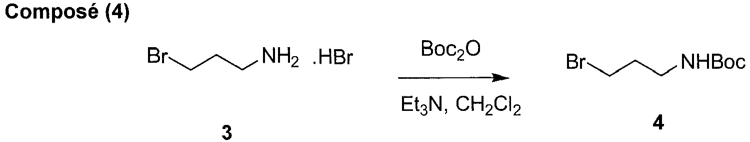

A une solution d'hydrobromure de 3-bromopropylamine **3** (3,72 g, 16,9 mmol) dans du dichlorométhane (100 mL) ont été ajoutés du dicarbonate de di-*tert*-butyle (3,71 g, 16,9 mmol) et de la triéthylamine (10 mL). Le mélange réactionnel a été agité à température ambiante pendant 12 h. L'avancement de la réaction a été suivi par CCM. Après cette période la réaction était totale. Le solvant a été éliminé sous pression réduite. A ce résidu a été additionnée une solution saturée de chlorure de sodium (100 mL) et le mélange a été extrait avec de l'éther diéthylique (2 x 50 mL). Les phases organiques ont été réunies, lavées par une solution saturée de chlorure de sodium (3 x 50 mL) et séchées sur sulfate de sodium. Après filtration, le solvant a été éliminé sous pression réduite pour conduire au composé **4** sous forme d'un solide légèrement brun. Le composé était suffisamment pur pour être utilisé dans la suite de la synthèse sans purification supplémentaire (3,50 g, 87%). RMN ¹H (200 MHz, CDCl₃) δ: 4,63 (s, 1 H), 3,42 (t, *J* = 6,5 Hz, 2H), 3,26 (td, *J* = 6,5; 6,5 Hz, 2H), 2,03 *(m, J* = 6,5 Hz, 2H), 1,43 (s, 9H). HMRS (ESI) calculée pour C₈H₁₆NO₂Br [M+H⁺], *m*/*z* 255,0703, trouvée: 255,0695.

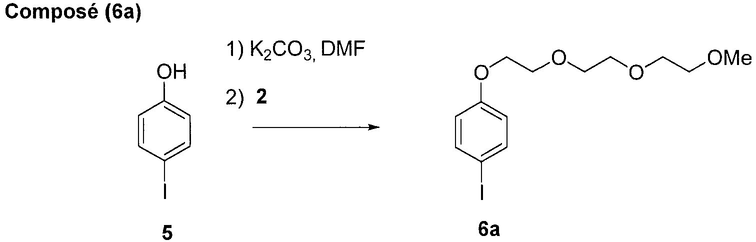

A une suspension de 4-iodophénol **5** (4,0 g, 18 mmol) et de carbonate de potassium (12,4 g, 90 mmol) dans du diméthylformamide (50 mL) a été ajouté le dérivé tosylé **2** (8,7 g, 27 mmol). Le mélange réactionnel a été chauffé à reflux pendant 36 h. L'avancement de la réaction a été suivi par CCM. Après cette période la réaction était totale. Le mélange réactionnel a été refroidi à température ambiante et a été filtré sur fritté. Le filtrat a été concentré sous pression réduite et le résidu a été purifié par chromatographie sur colonne de silice (cyclohexane / acétate d'éthyle 95/5 jusqu'à 50/50 par incrément de 5%) pour conduire au composé **6a** sous forme d'une huile jaune (3,38 g, 51%). RMN ¹H (200 MHz, CDCl₃) δ: 7,52 (d, *J* = 9,0 Hz, 2H), 6,68 (d, *J* = 9,0 Hz, 2H), 4,07 (t, *J* = 6,0 Hz, 2H), 3,82 (t, *J* = 5,2 Hz, 2H), 3,62 (m, 6H), 3,54 (m, 2H), 3,36 (s, 3H). RMN ¹³C (50 MHz, CDCl₃) δ: 158,7; 138,2; 117,1; 82,9; 71,9; 70,9; 70,7; 70,6; 69,6; 67,6; 50,1. HMRS (ESI) calculée pour C₁₃H₁₉O₄I [M+H⁺], *m*/*z* 384,0666, trouvée: 384,0668.

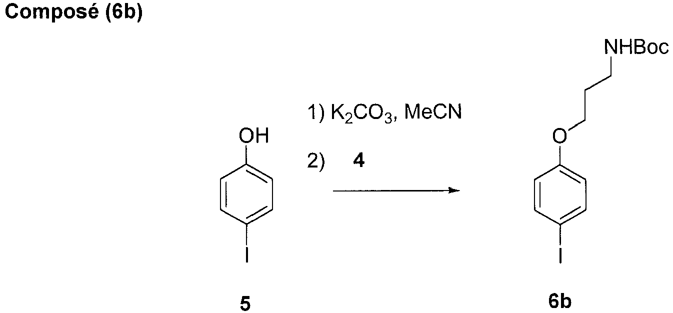

A une solution de 4-iodophénol **5** (3,00 g, 13,6 mmol) dans de l'acétonitrile anhydre (100 mL) a été ajouté le carbonate de sodium (5,65 g, 40,9 mmol). La réaction a été chauffée à reflux pendant 1 h puis à cette suspension a été ajouté le dérivé bromé **4** (2,60 g, 10,9 mmol). Le mélange réactionnel a été chauffé à reflux pendant 12 h. L'avancement de la réaction a été suivi par CCM. Après cette période la réaction était totale. Le mélange réactionnel a été refroidi à température ambiante et le solvant a été éliminé sous pression réduite. A ce résidu a été additionnée de l'eau (100 mL) et le mélange a été extrait avec du dichlorométhane (2 x 50 mL). Les phases organiques ont été réunies, séchées sur sulfate de sodium et filtrées. Le solvant a été éliminé sous pression réduite et le résidu a été purifié par chromatographie sur colonne de silice (dichlorométhane) pour conduire au composé **6b** sous forme d'un solide blanc (3,10 g, 75%). PtF : 79-80°C. RMN ¹H (500 MHz, CDCl₃) δ: 7,52 (d, *J* = 8,9 Hz, 2H), 6,64 (d, *J* = 8,9 Hz, 2H), 4,69 (s, 1 H), 3,95 (t, *J* = 6,2 Hz, 2H), 3,29 (td, *J* = 6,2; 6,2 Hz, 2H), 1,94 (m, *J* = 6,2 Hz, 2H), 1,41 (s, 9H), RMN ¹³C (125 MHz, CDCl₃) δ: 158,7; 155,9; 138,2; 116,9; 82,9; 65,9; 37,9. HMRS (ESI) calculée pour C₁₄H₂₀NO₃I [M+H⁺], *m*/*z* 378,0561, trouvée: 378,0559.

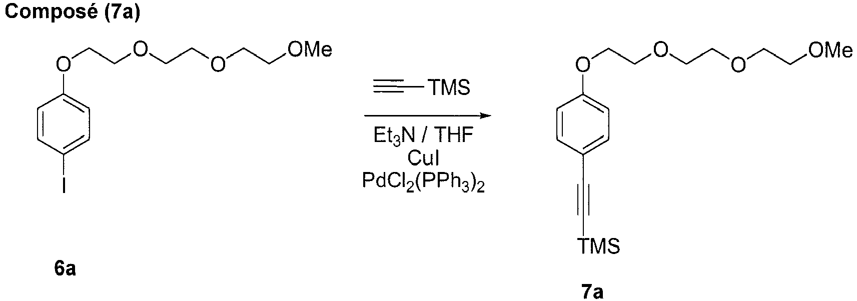

Une solution de composé iodé **6a** (1,00 g, 2,7 mmol) dans un mélange de tétrahydrofurane (20 mL) et de triéthylamine (20 mL) a été dégazée sous agitation pendant 20 min. A cette solution ont été additionnés du triméthylsilylacétylène (0,8 g, 8,2 mmol) suivi par du bis-chlorure bis-triphénylphosphine de palladium (II) (19 mg, 0,02 mmol) et de l'iodure de cuivre (I) (10 mg, 0,054 mmol). Le mélange réactionnel a été agité à température ambiante pendant 12 h. L'avancement de la réaction a été suivi par CCM. Après cette période, la réaction était totale. Les solvants ont été éliminés sous pression réduite. Au résidu a été additionnée une solution de chlorure d'ammonium saturée (50 mL) et le mélange a été extrait avec du dichlorométhane (2 x 25 mL). Les phases organiques ont été réunies et lavées par une solution de chlorure d'ammonium saturée (50 mL) puis par une solution de chlorure de sodium (2 x 50 mL) et ont été enfin séchées sur sulfate de sodium. Après filtration le solvant a été éliminé sous pression réduite et le résidu a été purifié par chromatographie sur silice (dichlorométhane/acétate d'éthyle 95/5 90/10) pour conduire au composé **7a** sous forme d'une huile légèrement jaune (0,64 g, 70%). RMN ¹H (200 MHz, CDCl₃) δ: 7,36 (d, *J* = 8,8 Hz, 2H), 6,82 (d, *J* = 8,8 Hz, 2H), 4,11 (t, *J* = 5,8Hz, 2H), 3,83 (t, *J =* 5,1 Hz, 2H), 3,64 (m, 6H), 3,53 (m, 2H), 3,34 (s, 3H), 0,24 (s, 9H). RMN ¹³C (50 MHz, CDCl₃) δ: 159,0; 133,3; 115,4; 114,4; 105,2; 92,3; 71,9; 70,8; 70,6; 70,5; 69,6; 67,4; 58,9; 0,1. HMRS (ESI) calculée pour C₁₈H₂₈O₄Si [M+H⁺], *m*/*z* 337,1830, trouvée: 337,1828.

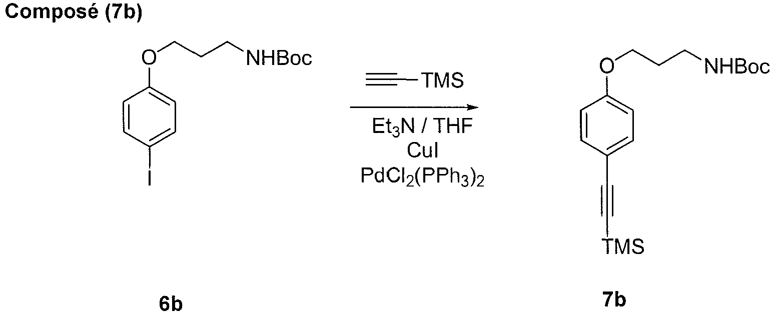

Une solution de dérivé iodé **6b** (2,50 g, 6,6 mmol) dans un mélange de tétrahydrofurane (20 mL) et de triéthylamine (20 mL) a été dégazée sous agitation pendant 20 min. A cette solution ont été additionnés du triméthylsilylacétylène (1,95 g, 19,8 mmol) suivi du bis-chlorure bis-triphénylphosphine de palladium (II) (46 mg, 0,06 mmol) et de l'iodure de cuivre (I) (25 mg, 0,13 mmol). La réaction a été agitée à température ambiante pendant 12 h. L'avancement de la réaction a été suivi par CCM. Après cette période la réaction était totale. Les solvants ont été éliminés sous pression réduite. Au résidu a été additionnée une solution de chlorure d'ammonium saturée (50 mL) et le mélange a été extrait avec du dichlorométhane (2 x 25 mL). Les phases organiques ont été réunies et lavées par une solution de chlorure d'ammonium saturée (50 mL) puis par une solution de saturée de chlorure de sodium (2 x 50 mL) et ensuite séchées sur sulfate de sodium. Après filtration, le solvant a été éliminé sous pression réduite et le résidu a été purifié par chromatographie sur colonne de silice (dichlorométhane / pentane 1/1 puis dichlorométhane) pour conduire au composé **7b** sous forme d'un solide blanc (1,50 g, 65%). PtF : 92-93°C. RMN ¹H (200 MHz, CDCl₃) δ: 7,37 (d, *J* = 8,7 Hz, 2H), 6,78 (d, *J* = 8,7 Hz, 2H), 4,69 (s, 1 H), 3,99 (t, *J* = 6,2 Hz, 2H), 3,29 (td, *J* = 6,2 Hz et *J* = 6,2 Hz, 2H), 1,95 (m, *J* = 6,2 Hz, 2H), 1,42 (s, 9H), 0,22 (s, 9H). RMN ¹³C (100 MHz, CDCl₃) δ: 159,08; 156,13; 133,61; 115,54; 114,44; 105,28; 92,63; 79,41; 65,92; 38,06; 29,66; 28,54; 0,20. HMRS (ESI) calculée pour C₁₉H₂₉NO₃Si[M+H⁺], *m*/*z* 348,1989, trouvée: 348,1993.

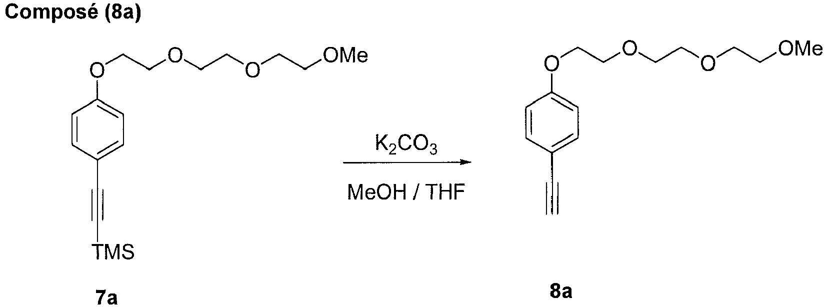

A une solution de dérivé silylé **7a** (5,7 g, 17 mmol) dans un mélange de tétrahydrofurane (60 mL) et de méthanol (60 mL) a été ajouté du carbonate de potassium (7,0 g, 58 mmol). Le milieu réactionnel a été agité à température ambiante pendant 2 h. L'avancement de la réaction a été suivi par CCM. Après cette période la réaction était totale. Le mélange réactionnel a été filtré et le filtrat a été concentré sous pression réduite. Le résidu a été purifié par chromatographie sur colonne de silice (dichlorométhane / acétate d'éthyle 95/5 puis 90/10) pour conduire au composé **8a** sous forme d'une huile jaune (3,9 g, 87%). RMN ¹H (200 MHz, CDCl₃) δ: 7,40 (d, *J* = 8,8 Hz, 2H), 6,84 (d, *J* = 8,8 Hz, 2H), 4,12 (t, *J* = 5,1 Hz, 2H), 3,84 (t, *J* = 4,5 Hz, 2H), 3,64 (m, 6H), 3,54 (m, 2H), 3,36 (s, 3H), 2,98 (s, 1H). RMN ¹³C (50 MHz, CDCl₃) δ: 159,4; 133,7; 114,8; 114,5; 83,9; 76,5; 72,1; 71,0; 70,8; 70,7; 69,8; 67,7; 59,1.

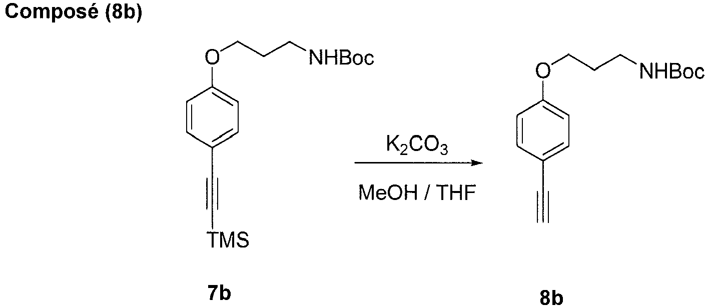

A une solution de dérivé silylé **7b** (1,27 g, 3,7 mmol) dans un mélange de tétrahydrofurane (30 mL) et de méthanol (30 mL) a été ajouté du carbonate de potassium (1,52 g, 11,0 mmol). Le milieu réactionnel a été agité à température ambiante pendant 2 h. L'avancement de la réaction a été suivi par CCM. Après cette période la réaction était totale. Le mélange réactionnel a été filtré et le filtrat a été concentré sous pression réduite. Le résidu a été purifié par chromatographie sur colonne de silice (pentane / dichlorométhane 50/50 jusqu'à 100% par incréments de 10%) pour conduire au composé **8b** sous forme d'un solide blanc (0,86 g, 85%). RMN ¹H (200 MHz, CDCl₃) δ: 7,39 (d, *J* = 8,7 Hz, 2H), 6,81 (d, *J* = 8,7 Hz, 2H), 4,69 (s, 1H), 3,99 (t, *J* = 6,2 Hz, 2H), 3,29 (td, *J* = 6,2 Hz et *J* = 6,2 Hz, 2H), 2,97 (s, 1 H), 1,95 (m, *J* = 6,2 Hz, 2H), 1,42 (s, 9H).

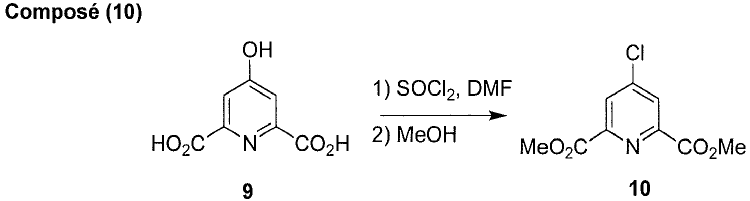

A une solution d'acide chélidamique **9** (17 g, 93 mmol) dans du chlorure de thionyle (95 mL) ont été additionnées quelques gouttes de diméthylformamide. Cette solution a été chauffée à 100°C pendant 48 h. Le chlorure de thionyle a été éliminé sous pression réduite. A ce résidu a été additionné du dichlorométhane (50 mL) et le mélange a été refroidi à 0°C. A ce mélange, est additionné goutte à goutte sur une période de 10 min du méthanol anhydre (40 mL) et le mélange a été laissé revenir à température ambiante pendant une nuit. Les solvants ont été éliminés sous pression réduite et à ce résidu a été additionnée une solution saturée de bicarbonate de sodium (100 mL). Le mélange a été filtré et le filtrat a été extrait avec du dichlorométhane (3 x 50 mL), les phases organiques ont été regroupées, séchées sur sultate de sodium, filtrées et évaporées sous pression réduite pour donner un solide blanc identifié comme le composé **10**. Le produit était suffisament pur pour être utilisé dans la suite de la synthèse sans purification supplémentaire (13,3 g, 63%). RMN ¹H (300 MHz, CDCl₃) δ: 8,32 (s, 2H), 4,06 (s, 6H).

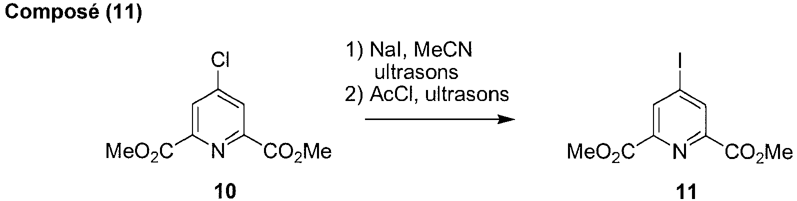

A une solution du composé **10** (6,0 g, 26,2 mmol) dans de l'acétonitrile anhydre (140 mL) a été additionné de l'iodure de sodium (39 g, 262 mmol) et le mélange a été ultrasonifié pendant 20 min. A ce mélange a été additionné du chlorure d'acétyle (5,55 mL, 78,6 mmol) et le mélange a été ultrasonifié pendant 5 h. A cette solution refroidie à 0°C a été additionnée une solution saturée de bicarbonate de sodium (75 mL) suivie par une addition d'eau (100 mL). Le mélange a été extrait avec de l'acétate d'éthyle (2 x 50 mL), et les phases organiques ont été réunies, lavées avec une solution de thiosufalte 0,2 M, et enfin séchées sur sulfate de sodium, filtrées puis concentrées sous pression réduite. A ce résidu a été additionné du méthanol (40 mL), le mélange a été agité pendant 20 min puis a été filtré pour donner un solide blanc identifié comme le composé **11**. Le produit était suffisament pur pour être utilisé dans la suite de la synthèse sans purification supplémentaire (6,9 g, 82%). RMN ¹H (300 MHz, CDCl₃) δ: 8,68 (s, 2 H), 4,05 (s, 6H).

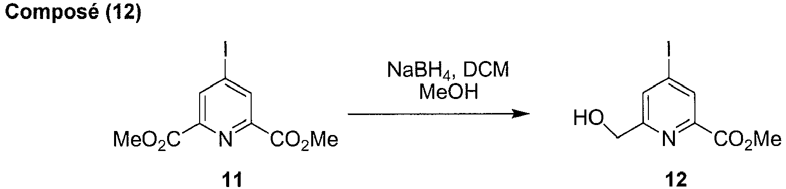

A une solution de diester **11** (7,0 g, 21,8 mmol) dans du dichlorométhane (50 mL) refroidie à 0°C a été additionné du méthanol (35 mL) suivie de l'addition de borohydrure de sodium (540 mg, 14,2 mmol). Le mélange a été agité pendant 2 h à 0°C puis a été additionnée une solution d'acide chlorhydrique 1 M (20 mL). Le solvant a été éliminé sous pression réduite et au résidu a été additionnée une solution saturée de bicarbonate de sodium (100 mL), puis le mélange a été extrait avec de l'acétate d'éthyle (4 x 30 mL). Les phases organiques ont été réunies, séchées sur sulfate de sodium, filtrées et concentrées sous pression réduite. Le résidu a été purifié par chromatographie sur colonne de silice (cyclohexane / acétate d'éthyle 60/40 jusqu'à 0/100 par incrément de 10%) pour donner le composé **12** comme un solide blanc (3.8 g, 60%). RMN ¹H (300 MHz, CDCl₃) δ: 8,41 (s, 1H), 7,99 (s, 1H), 4,85 (s, 2H), 4,02 (s, 3H). RMN ¹³C (75 MHz, CDCl₃) δ: 164,44; 161,44; 147,31; 133,34; 133,06; 106,97; 64,31; 53,29.

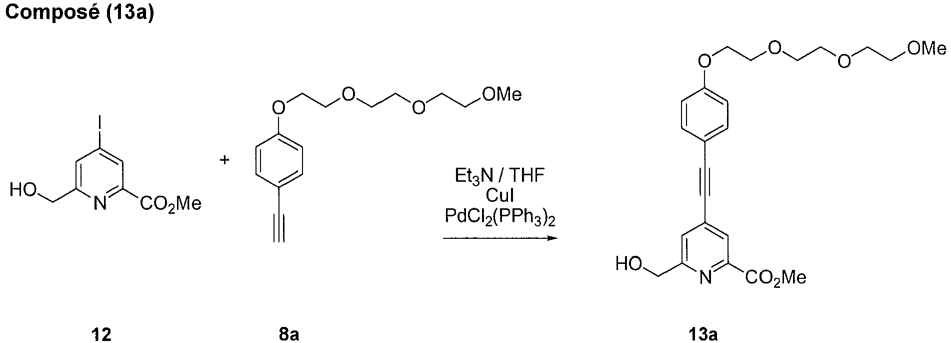

Une solution de dérivé acétylènique **8a** (3,9 g, 15 mmol) et de dérivé iodé **12** (2,8 g, 9,6 mmol) dans un mélange de tétrahydrofurane (20 mL) et de triéthylamine (20 mL) a été dégazée sous agitation pendant 20 min. A cette solution ont été additionnés du bis-chlorure bis-triphénylphosphine de palladium (II) (104 mg, 0,15 mmol) et de l'iodure de cuivre (I) (56 mg, 0,29 mmol). La réaction a été agitée à température ambiante pendant 12 h. L'avancement de la réaction a été suivi par CCM. Après cette période, la réaction était totale. Les solvants ont été éliminés sous pression réduite. Au résidu a été additionné une solution de chlorure d'ammonium saturée (50 mL) et le mélange a été extrait avec du dichlorométhane (2 x 25 mL). Les phases organiques ont été réunies, lavées par une solution de chlorure d'ammonium saturée (50 mL) puis par une solution de chlorure de sodium saturée (2 x 50 mL) et ensuite séchées sur sulfate de sodium. Après filtration, le solvant a été éliminé sous pression réduite et le résidu a été purifié par chromatographie sur colonne de silice (dichlorométhane / méthanol 1/1) pour conduire au produit **13a** sous forme d'un solide jaune (3,2 g, 78%). PtF : 47°C. RMN ¹H (500 MHz, CDCl₃) δ: 8,04 (s, 1 H), 7,56 (s, 1 H), 7,45 (d, *J* = 8,8 Hz, 2H), 6,89 (d, *J* = 8,8 Hz, 2H), 4,82 (s, 2H), 4,13 (m, 2H), 3,97 (s, 3H), 3,84 (m, 3H), 3,71 (m, 2H), 3,64 (m, 4H), 3,52 (m, 2H), 3,35 (s, 3H), 1,89 (s, 1H). RMN ¹³C (50 MHz, CDCl₃) δ: 165,4; 160,7; 160,1; 147,3; 134,1; 133,8; 125,8; 125,5; 115,1; 114,1; 95,9; 85,4; 72,1. HMRS (ESI) calculée pour C₂₃H₂₇NO₇ [M+H⁺], *m*/*z* 430,1860, trouvée: 430,1858.

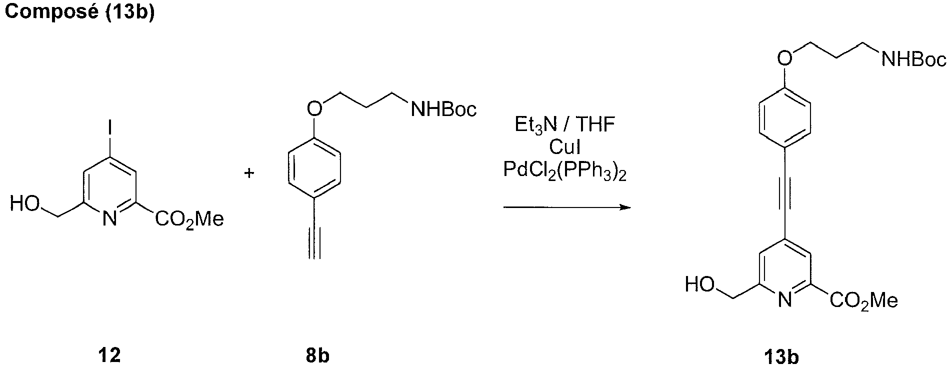

Une solution de dérivé acétylènique **8b** (0,864 g, 3,1 mmol) et de dérivé iodé **12** (0,735 g, 2,5 mmol) dans un mélange de tétrahydrofurane (20 mL) et de triéthylamine (20 mL) a été dégazée sous agitation pendant 20 min. A cette solution ont été additionnés du bis-chlorure bis-triphénylphosphine de palladium (II) (22 mg, 0,031 mmol) et de l'iodure de cuivre (I) (12 mg, 0,063 mmol). La réaction a été agitée à température ambiante pendant 12 h. L'avancement de la réaction a été suivi par CCM. Après cette période la réaction était totale. Les solvants ont été éliminés sous pression réduite. Au résidu a été additionnée une solution de chlorure d'ammonium saturée (50 mL) et le mélange a été extrait à l'aide de dichlorométhane (2 x 25 mL). Les phases organiques ont été réunies, lavées par une solution de chlorure d'ammonium saturée (50 mL) puis avec une solution saturée de chlorure de sodium (2 x 50 mL) et ensuite séchées sur sulfate de sodium. Après filtration le solvant a été éliminé sous pression réduite et le résidu a été purifié par chromatographie sur colonne de silice (dichlorométhane/méthanol 98/2) pour conduire au composé **13b** sous forme d'un solide blanc (0,910 g, 82%). PtF : 143-144°C. RMN ¹H (500 MHz, CDCl₃) δ: 8,04 (s, 1 H), 7,58 (s, 1 H), 7,45 (d, *J* = 8,7 Hz, 2H), 6,86 (d, *J* = 8,7 Hz, 2H), 4,83 (d, *J* = 5,4 Hz, 2H), 4,75 (s, 1 H), 4,01 (t, *J* = 6,1 Hz, 2H), 3,97 (s, 3H), 3,31 (td, *J* = 6,1; 6,1 Hz, 2H), 1,96 (m, *J* = 6,1 Hz, 2H), 1,41 (s, 9H). RMN ¹³C (125 MHz, CDCl₃) δ: 165,4; 160,8; 160,1; 156,2; 147,3; 134,1; 133,8; 125,8; 125,4; 114,9; 113,9; 95,9; 85,4; 66,1; 64,8; 53,2; 38,1; 29,7; 28,6. HMRS (ESI) calculée pour C₂₄H₂₈N₂O₆ [M+H⁺], *m*/*z* 441.2020, trouvée: 441.2021.

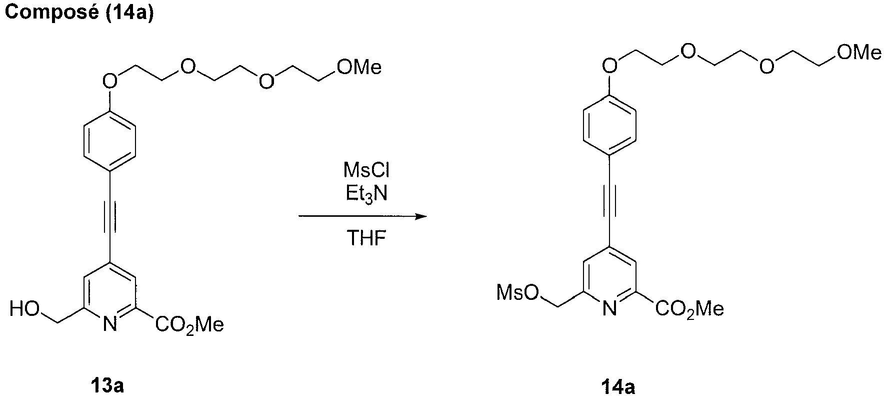

A une solution refroidie à 5°C d'alcool **13a** (300 mg, 0,7 mmol) dans du tétrahydrofurane (20 mL) ont été ajoutés de la triéthylamine (212 mg, 0,21 mmol) puis du chlorure de mésyle (88 mg, 0,77 mmol). Le milieu réactionnel a été agité à 0°C pendant 30 min puis à température ambiante pendant 12 h. L'avancement de la réaction a été suivi par CCM. Après cette période, la réaction était totale. Le solvant a été éliminé sous pression réduite. Au résidu a été additionnée de l'eau (30 mL) et le mélange a été extrait avec du dichlorométhane (2 x 30 mL). Les phases organiques ont été regroupées, lavées avec de l'eau (30 mL) et séchées sur sulfate de sodium. Après filtration le solvant a été éliminé sous pression réduite et le résidu a été purifié par chromatographie sur colonne de silice (dichlorométhane / acétate d'éthyle 8/2) pour conduire au composé **14a** sous forme d'un solide légèrement jaune (330 mg, 93%). RMN ¹H (500 MHz, CDCl₃) δ: 8,09 (s, 1 H), 7,66 (s, 1 H), 7,45 (d, *J =* 8,7 Hz, 2H), 6,88 (d, *J* = 8,7 Hz, 2H), 5,37 (s, 2H), 4,12 (m, 2H), 3,97 (s, 3H), 3,83 (m, 2H), 3,69 (m, 2H), 3,62 (m, 4H), 3,51 (m, 2H), 3,33 (s, 3H), 3,13 (s, 3H), RMN ¹³C (50 MHz, CDCl₃) δ: 164,9; 160,1; 154,6; 147,8; 134,6; 133,7; 126,6; 126,3; 114,9; 113,6; 96,8; 84,9; 71,9; 70,9; 70,7; 70,6; 69,6; 67,6; 59,1; 53,1; 38,1. HMRS (ESI) calculée pour C₂₄H₂₉NO₉S [M+H⁺], *m*/*z* 508,1636, trouvée: 508,1637.

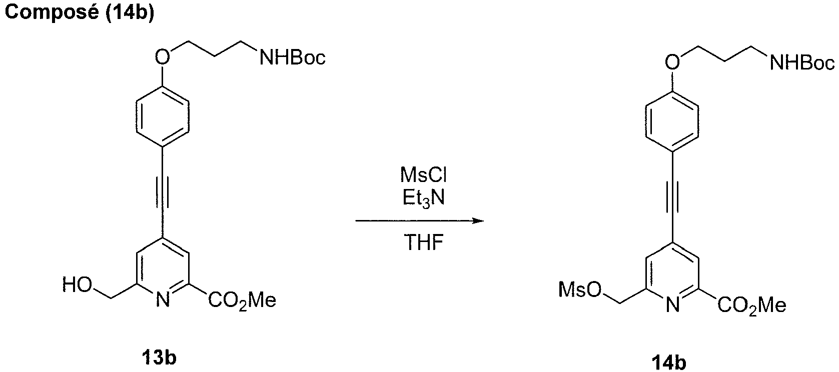

A une solution d'alcool **13b** (21,9 mg, 50 µmol) dans du tétrahydrofurane anhydre (1 mL), a été ajoutée goutte à goutte de la triéthylamine (18,1 mg, 150 µmol) sous atmosphère inerte. A ce mélange refroidi à 4°C, a été ajoutée goutte à goutte une solution de chlorure de mésyle (6 µL, 75 µmol) dans du tétrahydrofurane anhydre (0,2 mL). L'avancement de la réaction a été suivi par CCM. Après 20 min, la réaction était totale. Le solvant a été éliminé sous pression réduite. Le résidu a été dissous dans du dichlorométhane (4 mL) et cette solution a été lavée avec de l'eau (3 × 5 mL). La phase organique a été séchée sur sulfate de magnésium, filtrée et concentrée sous pression réduite pour conduire à une huile de couleur jaune-verte (26,9 mg, quantitatif). Le produit **14b** était suffisamment pur pour être utilisé dans la suite de la synthèse sans purification supplémentaire.

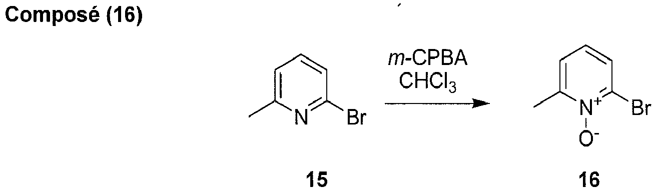

A une solution de 2-bromo-6-méthylpyridine **15** (30 g, 174 mmol) dans du chloroforme (300 mL) a été additionné à température ambiante de l'acide *m*-chloroperbenzoïque (62 g, 261 mmol). Le mélange a été chauffé à 65°C pendant 20 h puis a été refroidi à 0°C pendant 3 h. Après filtration du précipité, le filtrat a été concentré sous pression reduite. A ce résidu a été additionnée une solution aqueuse d'hydroxyde de sodium (2N, 75 mL) et cette solution a été extraite avec du dicloromethane (3 x 100 mL). Les phases organiques ont été regroupées, séchées sur sulfate de sodium, filtrées et concentrées sous pression reduite pour conduire au composé **16** sous forme d'un solide jaune utilisé dans la suite de la synthèse sans purification supplémentaire (15 g, 67%); PtF: 48-55 °C; RMN ¹H (400 MHz, CDCl₃) δ: 7,55 (dd, *J* = 7,9 Hz, 1,6 Hz, 1 H), 7,23 (dd, *J* = 7,9 Hz, 1,6 Hz, 1 H), 7,01 (t, *J* = 7,9 Hz, 1 H), 2,57 (s, 3H); RMN ¹³C (100 MHz, CDCl₃) δ: 151,2; 133,5; 128,7; 125,3; 125,2; 19,3. HMRS (ESI) calculée pour C₆H₇NOBr [M+H⁺], *m*/*z* 187,9711, trouvée: 187,9698.

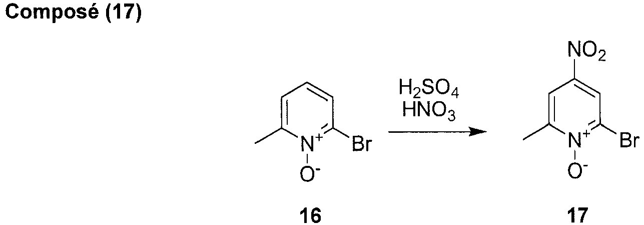

A une solution de composé **16** (10 g, 53 mmol) dans de l'acide sulfurique concentré (14,1 mL, 265 mmol) refroidie à 0°C a été additionné goutte à goutte de l'acide nitrique fumant (10,4 mL, 252 mmol). Le mélange a été agité à température ambiante pendant 15 min puis a été chauffé à 85°C pendant 16 h. La solution a été ensuite refroidie à température ambiante et a été versée dans de la glace pilée (60 g). Après 15 min d'agitation, le précipité a été filtré et a été lavé avec de l'eau. Le solide jaune a été dissous dans du dichlorométhane et la solution a été séchée sur sulfate de sodium, filtrée et concentrée sous pression réduite pour conduire au composé **17** sous forme d'un solide jaune qui a été utilisé dans la suite de la synthèse sans purification supplémentaire. PtF: 137-138°C.

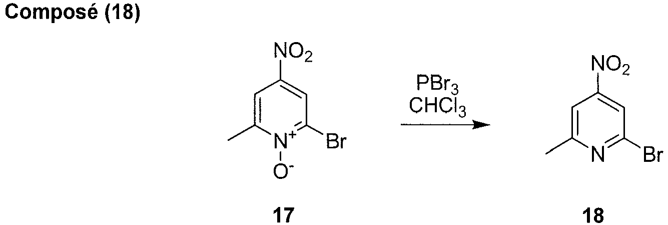

A une solution de composé N-oxyde **17** (11,7 g, 50 mmol) dans le chloroforme (300 mL), a été additionné du tribromure de phosphore (14,2 mL, 150 mmol) et le mélange a été chauffé à 60°C sous argon pendant 16 h. La solution a été refroidie à température ambiante et a été concentrée sous pression réduite puis a été versée dans une solution glacée d'hydroxyde de sodium 2M (260 mL). Le mélange a été extrait avec du dichlorométhane (3 x 100 mL), les phases organiques ont été regroupées, séchées sur du sulfate de sodium et concentrées sous pression réduite. L'huile résultante a été purifiée par chromatographie sur colonne de silice en utilisant le dichlorométhane comme éluant conduisant ainsi au composé **18** sous forme d'un solide jaune (8,4 g, 78%). PtF 51-52°C.

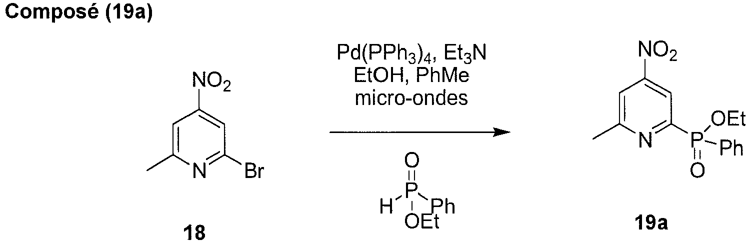

A une solution de 2-bromo-6-méthyl-4-nitropyridine **18** (1,01 g, 4,68 mmol) dans du toluène anhydre (10 mL), ont été additionnés du phénylphosphinate d'éthyle (0,95 g, 5,60 mmol) et de la triéthylamine (2,6 mL, 19,0 mmol). Le mélange a été dégazé par trois cycles de congélation-décongélation. A cette solution a été ajouté du tétrakis(triphénylphosphine)palladium(0) (83 mg, 0,07 mmole) et le mélange a été à nouveau dégazé trois fois avant d'être agité à reflux pendant 16 h sous argon. L'avancement de la réaction a été suivi par CCM. Après cette période, la réaction était totale. La solution a été refroidie et a été diluée avec du dichlorométhane (20 mL). Le mélange a été lavé avec une solution aqueuse d'acide chlorhydrique (1 M, 2 × 15 mL) suivi d'un lavage avec de l'eau (3 × 15 mL). La phase organique a été séchée sur du carbonate de potassium, filtrée et le solvant a été éliminé sous pression réduite pour obtenir un résidu sombre qui a été purifié par chromatographie sur colonne sur silice (dichlorométhane / méthanol 0,5%) conduisant ainsi au composé **19a** sous la forme d'une huile jaune (645 mg, 45 %). RMN ¹H (400 MHz, CDCl₃) δ: 8,55 (dd, *J* = 5,6 Hz, *J* = 1,4 Hz, 1H), 7,97 (ddd, *J =* 11,2 Hz, 7,7 Hz, 1,4 Hz, 2H), 7,90 (d, *J =* 1,4 Hz, 1 H), 7,55 (td, *J* = 7,7 Hz, 1,4 Hz, 1 H), 7,46 (td, *J* =7,7 Hz, 3,5 Hz, 2H), 4,15 (qd, *J* = 7,0 Hz, 4,2 Hz, 2H), 2,72 (s, 3H), 1,38 (t, *J* = 7,0 Hz, 3H) ; RMN ¹³C (100 MHz, CDCl₃) δ: 163,0 (d, *J* = 21 Hz), 158,1 (d, *J* = 167 Hz), 154,0 (d, *J* =13 Hz), 132,9 (d, *J* = 3 Hz), 132,5 (d, *J =* 10 Hz), 129,1 (d, *J* = 140 Hz), 128,5 (d, *J =* 13 Hz), 117,6 (d, *J* = 24 Hz), 117,5 (d, *J* = 3 Hz), 62,2 (d, *J* = 6 Hz), 24,9, 16,4; RMN ³¹P (162 MHz, CDCl₃) δ: 23,7. HMRS (ESI) calculée pour C₁₄H₁₆N₂O₄P [M+H⁺], *m*/*z* 307,0848, trouvée: 307,0851.

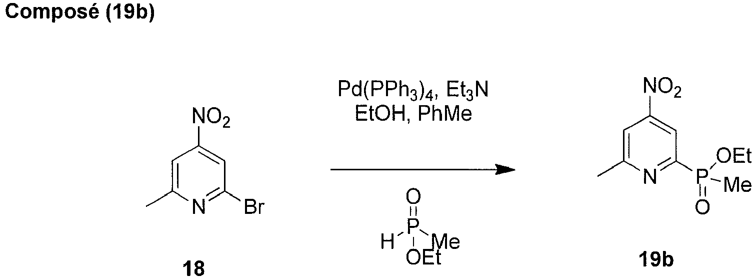

A du diéthylméthyl phosphonite (2 g, 14,7 mmol) a été ajoutée à 0°C de l'eau (264 µL, 14,7 mmol) sans précaution particulière. Le mélange réactionnel a été réchauffé à température ambiante en 1 h puis laissé sous agitation pendant 16 h. A ce mélange a été ajoutée une solution de 2-bromo-6-méthyl-4-nitropyridine **18** (2,1 g, 12,3 mmol) dans du toluène anhydre (20 mL), et de la triéthylamine (6 mL, 43,0 mmol). Le mélange a été dégazé par trois cycles de congélation-décongélation. A cette solution a été ajouté du tétrakis(triphénylphosphine)palladium(0) (320 mg, 0,27 mmole) et le mélange a été à nouveau dégazé trois fois avant d'être agité à reflux pendant 16 h sous argon. L'avancement de la réaction a été suivi par CCM. Après cette période, la réaction était totale. La solution a été refroidie et a été diluée avec du dichlorométhane (20 mL). Le mélange a été lavé avec une solution aqueuse d'acide chlorhydrique (1 M, 2 × 15 mL) suivi d'un lavage avec de l'eau (3 × 15 mL). La phase organique a été séchée sur du carbonate de potassium, filtrée et le solvant a été éliminé sous pression réduite pour obtenir un résidu sombre qui a été purifié par chromatographie sur colonne sur silice (dichlorométhane / méthanol 1,6% avec un incrément de 0,1%) conduisant ainsi au composé **19b** sous la forme d'une huile incolore (700 mg, 29 %). RMN ¹H (400 MHz, CDCl₃) δ 7,75 (dd, *J* = 7,0 Hz ³*J*_{H-P} 10 Hz, 1 H), 7,59 (td, ³*J* = 7,0 Hz *J* = 4,8 Hz, 1 H), 7,16 (d, *J* = 7,0 Hz, 1 H), 3,86 (dqd, *J* = 80 Hz *J* = 7,0 Hz *J* = 4,5 Hz, 2H), 2,50 (s, 3H), 1,66 (d, *J* = 15 Hz, 3H), 1,61 (t, *J* = 7,0 Hz, 3H); δ_{C} (CDCl₃) 159,4 (d, *J* 20 Hz), 153,7 (d, *J* = 158 Hz), 136,0 (d, *J* = 10 Hz), 125,6 (d, *J* = 3 Hz), 124,6 (d, *J* = 22 Hz), 60,8 (d, *J* = 6 Hz), 24,5, 16,3, 13,3 (d, *J* = 103 Hz); δ_{P} (CDCl₃) +41.2; *m*/*z* HMRS (ESI) calculée pour C₉H₁₄NO₂P [M+H⁺], *m*/*z* 200.0858, trouvée: 200.0862.

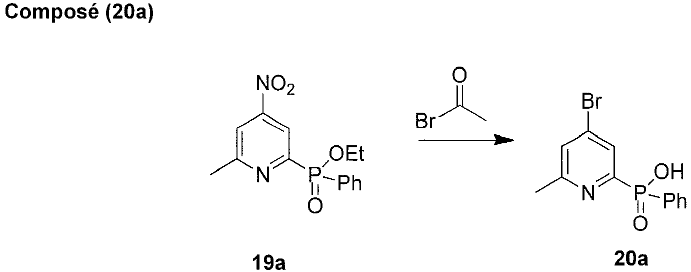

Au dérivé nitré **19a** (2,00 g, 6,54 mmol) a été ajouté du bromure d'acétyle (15 mL, 0,2 mol) et le mélange a été agité à 70 C pendant 16 h sous argon. Au cours de cette période un précipité brun pâle s'était formé. Le précipité et la solution ont été ajoutés goutte à goutte avec précaution dans du méthanol (100 mL) refroidi à 0°C. Le solvant a été éliminé sous pression réduite pour conduire au composé souhaité sous la forme d'un solide brun pâle qui a été utilisé directement dans la suite de la synthèse sans purification (1,81 g, 90 %) ; RMN ¹H (400 MHz, CD₃OD) δ: 8,33 (dd, *J* = 7,2 Hz, 2,0 Hz, 1 H), 8,23 (d, *J* = 2,0 Hz, 1 H), 7,95 (ddd, *J* =13,2 Hz, 7,6 Hz, 1,6 Hz, 2H), 7,63 (1 H, td, *J* = 7,6 Hz, 1,6 Hz, 1 H), 7,55 (2H, td, *J* = 7,6 Hz, 3,6 Hz), 2,77 (3H, s) ; RMN ¹³C (100 MHz, CD₃OD): δ 159,4 (d, *J* = 20 Hz), 151,7 (d, *J* = 160 Hz), 145,1 (d, *J* = 10 Hz), 134,8 (d, *J* = 3 Hz), 133,3 (d, *J =* 10 Hz), 131,0 (d, *J* = 24 Hz), 130,6 (d, *J* = 3 Hz), 130,2 (d, *J* = 140 Hz), 129,6 *(d, J* = 12 Hz), 20,4 ; RMN ³¹P (162 MHz, CD₃OD) δ: 14,3. HMRS (ESI) calculée pour C₁₂H₁₀NO₂P [M+H⁺], *m*/*z* 309,9633, trouvée: 309,9648.

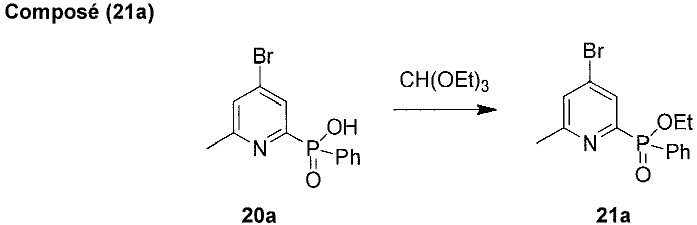

A l'acide phosphinique **20a** (1,80 g, 5,80 mmol) a été ajouté de l'orthoformiate d'éthyle (50 mL) et le mélange a été agité à 140°C pendant 72 h sous argon. L'avancement de la réaction a été suivi par CCM. Après cette période, la réaction était totale. La solution a été refroidie à température ambiante et le solvant a été éliminé sous pression réduite. Le résidu a été purifié par chromatographie sur colonne sur silice (dichlorométhane / méthanol 0,5 %) pour conduire au composé **21a** sous la forme d'une huile jaune (1,08 g, 55 %) ; RMN ¹H (400 MHz, CDCl₃) δ: 8,04 (dd, *J* = 6,3 Hz, 1,4 Hz, 1H), 7,95 (ddd, *J* = 11,2 Hz, 7,0 Hz, 1,4 Hz, 2H), 7,51 (1H, td, 7,0 Hz, 1,4 Hz, 1 H), 7,43 (td, *J* = 7,0 Hz, 3,5 Hz, 2H), 7,37 (d, *J* = 1,4 Hz, 1 H), 4,11 (qd, *J* = 7,0 Hz, 4,2 Hz, 2H), 2,52 (s, 3H), 1,34 (t, *J* = 7,0 Hz, 3H) ; RMN ¹³C (100 MHz, CDCl₃) δ: 161,2 (d, *J* = 22 Hz), 155,7 (d, *J* = 165 Hz), 133,5 (d, *J* = 15 Hz), 132,7 *(d, J* = 3 Hz), 132,6 (d, *J =* 10 Hz), 130,0 (d, *J* = 139 Hz), 128,5 (d, *J* = 3 Hz), 128,4 (d, *J* = 23 Hz), 128,3 (d, *J* = 13 Hz), 62,1 (d, *J* = 6 Hz), 24,5, 16,7 ; RMN ³¹P (162 MHz, CDCl₃) δ: 25,5. HMRS (ESI) calculée pour C₁₄H₁₆NO₂BrP [M+H⁺], *m*/*z* 340,0102, trouvée: 340,0102.

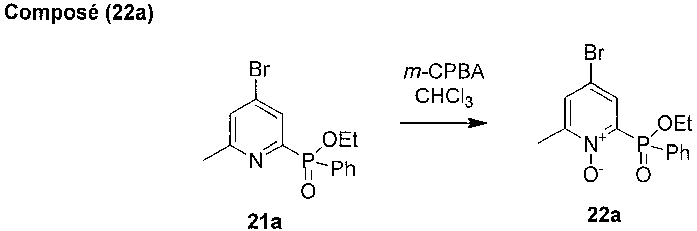

A une solution de dérivé pyridinyle **21a** (1,25 g, 3,68 mmol) dans du chloroforme (20 mL) a été ajouté de l'acide méta-chloroperbenzoïque (1,27 g, 7,35 mmol). La solution résultante a été agitée à 65°C pendant 16 h sous argon. L'avancement de la réaction a été suivi par CCM. Après cette période, la réaction était totale. Le mélange a été refroidi à température ambiante et le solvant a été éliminé sous pression réduite pour obtenir une huile jaune. Cette huile a été dissoute dans du dichlorométhane et a été lavée avec une solution de bicarbonate de sodium (0,5 M, 50 mL). Les phases ont été séparées et la phase aqueuse a été extraite avec du dichlorométhane (3 × 30 mL). Les phases organiques ont été réunies, ont été séchées sur sulfate de magnésium et le solvant a été éliminé sous pression réduite. L'huile jaune résultante a été purifiée par chromatographie sur colonne de silice (dichlorométhane / méthanol 0 à 2 % par incréments de 0,1 %) pour obtenir une huile jaune correspondant au composé **22a** (1,11 g, 75 %). RMN ¹H (400 MHz, CDCl₃) δ: 8,05 (dd, *J* = 7,7 Hz, 2,1 Hz, 1H), 7,98 (dd, *J* = 7,7 Hz, 13,3 Hz, 2H), 7,50 (t, 7,7 Hz, 1H), 7,44 (d, *J* = 2,1 Hz, 1H), 7,41 (td, *J* = 7,7 Hz, 4,2 Hz, 2H), 4,13 (qd, *J* = 5,6 Hz, 4,9 Hz, 2H), 2,32 (s, 3H), 1,34 (t, *J* = 5,6 Hz, 3H) ; RMN ¹³C (100 MHz, CDCl₃) δ: 151,0 (d, *J* = 4 Hz), 144,2 (d, *J* = 149 Hz), 133,2 (d, *J* = 11 Hz), 133,1 (d, *J* = 4 Hz), 133,0 (d, *J* = 11 Hz), 132,2 (d, *J* = 4 Hz), 129,0 (d, *J* = 152 Hz), 128,4 (d, *J* = 14 Hz), 117,4 (d, *J* = 12 Hz), 62,3 (d, *J* = 6 Hz), 17,5, 16,7 ; RMN ³¹P (162 MHz, CDCl₃) δ: +21,2. HMRS (ESI) calculée pour C₁₄H₁₆O₃BrNP [M+H⁺], *m*/*z* 356,0051, trouvée: 356,0061.

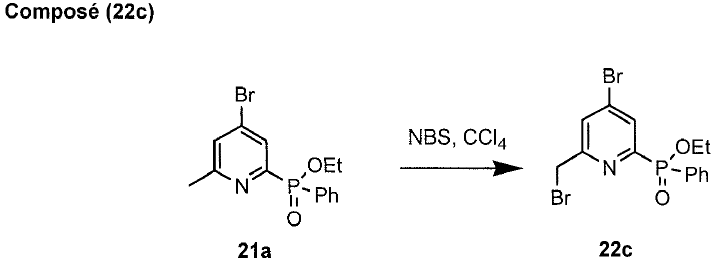

A une solution de dérivé méthylpyridinyle **21a** (1,18 g, 3,47 mmol) dans du tétrachlorure de carbone (50 mL) ont été additionnés de la N-bromosuccinimide (0,92 g, 5,21 mmol) puis du péroxyde de benzoyle (30 mg, 1,2 mmol). Le mélange a été chauffé à reflux sous atmosphère inerte et sous irradiation en utilisant une lampe de 100 W pendant 16 h. L'avancement de la réaction a été suivi par RMN ¹H. Après cette période, la réaction était totale. Le mélange réactionnel a été refroidi à température ambiante et le solvant a été éliminé sous pression réduite et le résidu a été solubilisé dans du dichlorométhane (30 mL) puis a été lavé avec une solution de carbonate de potassium (25 mL). La phase organique a été séchée sur sulfate de magnésium, filtrée et le solvant a été éliminé sous pression réduite. Le produit brut a été purifié par chromatographie sur colonne de silice (cyclohexane / acétate d'éthyle de 0% jusqu'à 30% par incrément de 2%) pour conduire au composé **22c** sous forme d'une huile incolore (466 mg, 32 %). RMN ¹H (400 MHz, CDCl₃) δ: 8,16 (dd, *J* = 6,0 Hz, *J* = 1,6 Hz, 1H), 7.96 (ddd, *J* = 12,4 Hz, *J* = 6,8 Hz, *J* = 1,6 Hz, 2H), 7,69 (d, *J* = 1,6 Hz, 1 H), 7,53 (td, *J* = 6,8 Hz, *J* = 1,6 Hz, 1 H), 7,44 (td, *J* = 6,8 Hz *J* = 3,6 Hz, 2H), 4,49 (s, 2H), 4,12 (qd, *J* = 6,8 Hz, *J* = 4,2 Hz, 2H), 1,36 (t, *J* = 6,8 Hz, 3H); RMN ¹³C (100 MHz, CDCl₃) δ: 159,2 (*J* = 21 Hz), 156,1 (d, *J =* 163 Hz), 134,5 (*J* = 14 Hz), 133,0 (*J* = 3 Hz), 132,7 (*J* = 10 Hz), 130,8 (*J* = 23 Hz), 129,4 (*J* = 139 Hz), 128,7 (*J* = 3 Hz), 128,6 (*J* = 13 Hz), 62,4 (d, *J* = 6 Hz), 32,5, 16,7; RMN ³¹P (162 MHz, CDCl₃) δ: 24,9; HMRS (ESI) calculée pour C₁₄H₁₅NO₂PBr₂ [M+H⁺], *m*/*z* 417,9207, trouvée: 417,9212.

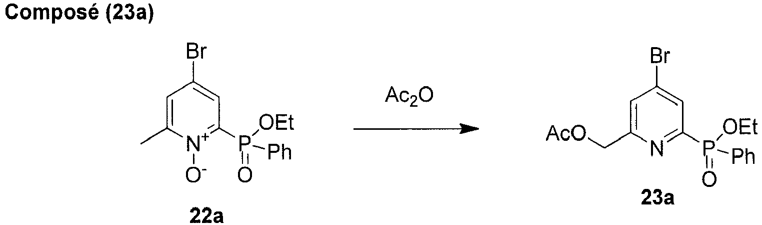

Au dérivé pyridinyl N-Oxyde **22a** (1,8 g, 5,1 mmol) a été ajouté de l'anhydride acétique (35 mL) et la solution a été chauffée à 120°C pendant 3 h sous agitation. L'avancement de la réaction a été suivi par RMN ³¹P. Après cette période, la réaction était totale. La solution a été refroidie à température ambiante et le solvant a été éliminé sous pression réduite. Le résidu a été purifié par chromatographie sur colonne de silice (dichlorométhane / méthanol de 0 à 1 % par incréments de 0,1 %) pour obtenir une huile jaune correspondant au composé **23a** (0,66 g, 33 %) ; RMN ¹H (400 MHz, CDCl₃) δ: 8,16 (dd, *J* = 7,7 Hz, 2,1 Hz, 1H), 7,93 (dd, *J* = 7,7 Hz, 13,3 Hz, 2H), 7,55 (d, *J =* 2,1 Hz, 1H), 7,51 (t, *J* = 7,7 Hz, 1H), 7,43 (td, *J* = 7,7 Hz, 4,2 Hz, 2H), 5,18 (s, 2H), 4,09 (qd, *J* = 5,6 Hz, 4,9 Hz, 2H), 2,12 (s, 3H), 1,33 (t, *J =* 5,6 Hz, 3H) ; RMN ¹³C (100 MHz, CDCl₃) δ: 170,5, 158,6 (d, *J* = 4 Hz), 156,0 (d, *J* = 149 Hz), 134,3 (d, *J* = 25 Hz), 132,9 (d, *J* = 5 Hz), 132,7 (d, *J* = 10 Hz), 130,5 (d, *J* = 23 Hz), 129,5 (d, *J* = 139 Hz), 128,6 (d, *J* = 13 Hz), 126,6 (d, *J =* 3 Hz), 66,0, 62,2 (d, *J* = 6 Hz), 21,0, 16,7 ; RMN ³¹P (162 MHz, CDCl₃) δ: +25,0 . HMRS (ESI) calculée pour C₁₆H₁₈O₄BrNP [M+H⁺], *m*/*z* 398,0151, trouvée: 398,0157.

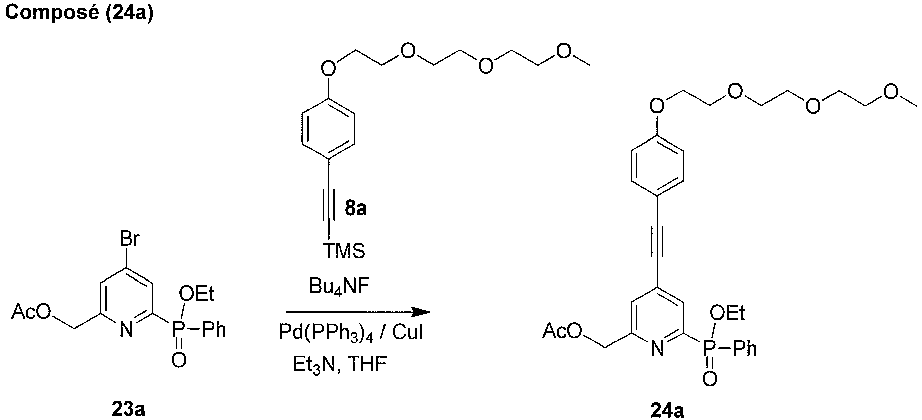

Au dérivé bromé **23a** (540 mg, 1,36 mmol) a été ajouté du tétrahydrofurane anhydre (8 mL) et la solution a été dégazée par trois cycles de congélation-décongélation. A cette solution a été ajouté du dérivé acétylénique **8a** (456 mg, 1,36 mmol) suivi par de la triéthylamine (4 mL) et la solution a été à nouveau dégazée. A cette solution ont été ajoutés du tétrakis(triphénylphosphine)palladium(0) (156 mg, 0,136 mmol) et de l'iodure de cuivre (26 mg, 0,136 mmol). Cette nouvelle solution a été à nouveau dégazée trois fois puis a été agitée sous argon et immédiatement après, à ce mélange a été additionné une solution de fluorure de tétrabutylammonium (1 M dans du tétrahydrofurane, 1,9 mL, 2,04 mmol). Un changement de couleur du jaune au bleu foncé était observé et le mélange a été agité à 65 °C sous argon. L'avancement de la réaction a été suivi par CCM. Après 3 h la réaction était totale. Le mélange réactionnel a été refroidi et le solvant a été éliminé sous pression réduite et le produit brut a été purifié par chromatographie sur colonne de silice (dichlorométhane / méthanol 0 à 1,5 % par incréments de 0,1 %) pour obtenir une huile jaune correspondant au composé **24a** (575 mg, 73 %). RMN ¹H (400 MHz, CDCl₃) δ: 8,05 (dd, *J* = 6,0 Hz, 1,6 Hz, 1H), 7,96 (dd, *J* = 8,4 Hz, 12,4 Hz, 2H), 7,50 (t, *J* = 8,4 Hz, 1 H), 7,44 (d, *J* = 8,8 Hz, 2H), 7,43 (2H, td, *J* = 8,4 Hz, 4,2 Hz, 2H), 7,42 (d, *J* = 1,6 Hz, 1 H), 6,88 (d, *J* = 8,8 Hz, 2H), 5,21 (s, 2H), 4,12 (qd, *J* = 5,6 Hz, 4,8 Hz, 2H), 4,11 (t, *J* = 4,8 Hz, 2H), 3,83 (t, *J* = 4,8 Hz, 2H), 3,71 (t, *J* = 4,8 Hz, 2H), 3,65 (t, *J* = 4,8 Hz, 2H), 3,63 (t, *J* = 4,8 Hz, 2H), 3,61 (t, *J* = 4,8 Hz, 2H), 3,34 (s, 3H), 2,13 (s, 3H), 1,34 (t, *J =* 5,6 Hz, 3H) ; RMN ¹³C (100 MHz, CDCl₃) δ: 170,6; 160,1; 157,3 (d, *J* = 20 Hz); 154,7 (d, *J =* 165 Hz), 133,8; 133,2 (d, *J* =12 Hz), 132,7 (d, *J* = 5 Hz), 132,6 (d, *J* = 10 Hz), 130,1 *(d, J =* 139 Hz), 128,8 (d, *J* = 22 Hz), 128,6 (d, *J =* 12 Hz), 124,6 (d, *J* = 3 Hz), 115,1; 114,1; 96,2; 85,5 (d, *J* = 2 Hz), 72,2; 71,1; 70,9; 70,8; 69,8; 67,8; 66,5; 62,1 (d, *J* = 6 Hz), 59,3; 21,1; 16,7; RMN ³¹P (162 MHz, CDCl₃) δ:+26,0. HMRS (ESI) calculée pour C₃₁H₃₇O₈NP [M+H⁺], *m*/*z* 582,2257, trouvée: 582,2260.

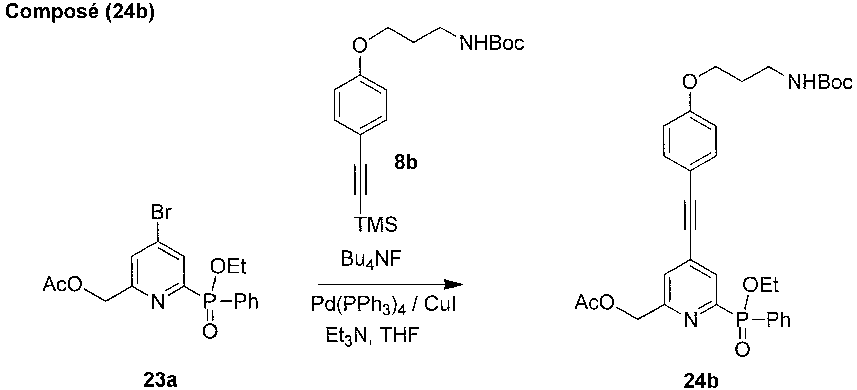

Au dérivé bromé **23a** (170 mg, 0,43 mmol) a été ajouté du tétrahydrofurane anhydre (3 mL) et la solution a été dégazée par trois cycles de congélation-décongélation. A cette solution ont été ajoutés du dérivé acétylénique **8b** (148 mg, 0,43 mmol) et de la triéthylamine (1,5 mL) et la solution a été à nouveau dégazée. A cette solution ont été ajoutés du tétrakis(triphénylphosphine)palladium(0) (49 mg, 0,043 mmol) et de l'iodure de cuivre (8 mg, 0,043 mmol). Cette nouvelle solution a été à nouveau dégazée trois fois puis a été agitée sous argon et immédiatement après, à ce mélange, a été additionnée une solution de fluorure de tétrabutylammonium (1 M dans du THF, 0,6 mL, 0,64 mmol). Un changement de couleur du jaune au bleu foncé était observé et le mélange a été agité à 65 °C sous argon. L'avancement de la réaction a été suivi par CCM. Après 3 h la réaction était totale. Le mélange réactionnel a été refroidi à température ambiante et le solvant a été éliminé sous pression réduite. Le produit brut a été purifié par chromatographie sur colonne de silice (dichlorométhane / méthanol 0 à 1,5 % par incréments de 0,1 %) pour obtenir une huile jaune correspondant au composé **24b** (174 mg, 69 %) ; RMN ¹H (400 MHz, CDCl₃) δ: 8,09 (dd, *J* = 6,0 Hz, 2,0 Hz, 1H), 7,99 (dd, *J =* 8,4 Hz *J* = 12,4 Hz, 2H), 7,54 (t, *J* = 8,4 Hz, 1 H), 7,47 (d, *J* = 8,4 Hz, 2H), 7,45 (td, *J* = 8,4 Hz, 4,2 Hz, 2H), 7,45 (d, *J* 2,0 Hz, 1H), 6,88 (d, *J* = 8,4 Hz, 2H), 5,24 (s, 2H), 4,75 (se, 1H), 4,14 (qd, *J* = 5,6 Hz; 4,8 Hz, 2H), 4,05 (t, *J* = 6 Hz, 2H), 3,33 (m, 2H), 2,17 (s, 3H), 1,92 (q, *J* = 6 Hz, 2H), 1,44 (s, 9H), 1,38 (t, *J =* 5,6 Hz, 3H); RMN¹³C (100 MHz, CDCl₃) δ: 170,7; 160,0; 157,3 (d, *J* = 20,3 Hz), 156,2; 154,6 *(d, J* = 167 Hz), 133,9; 133,2 (d, *J* = 12 Hz), 132,7 (d, *J* = 5 Hz), 132,6 (d, *J* = 10 Hz), 130,1 (d, *J* = 138 Hz), 129,1 (d, *J* = 23 Hz), 128,6 (d, *J =* 13 Hz), 124,6 (d, *J* = 3 Hz), 114,9; 114,1; 96,2; 85,5 (d, *J* = 2 Hz), 79,5; 66,5; 66,1; 62,1 (d, *J* = 6 Hz), 38,1; 29,7; 28,6; 21,1; 16,7; RMN ³¹P (162 MHz, CDCl₃) δ: +26,1. HMRS (ESI) calculée pour C₃₂H₃₈O₇N₂P [M+H⁺], *m*/*z* 593,2417 trouvée: 593,2435.

### Composés (24c), (24d), (24e), (24f)

Ces composés ont été obtenus en suivant la procédure utilisée pour les composés **24a** et 24b.

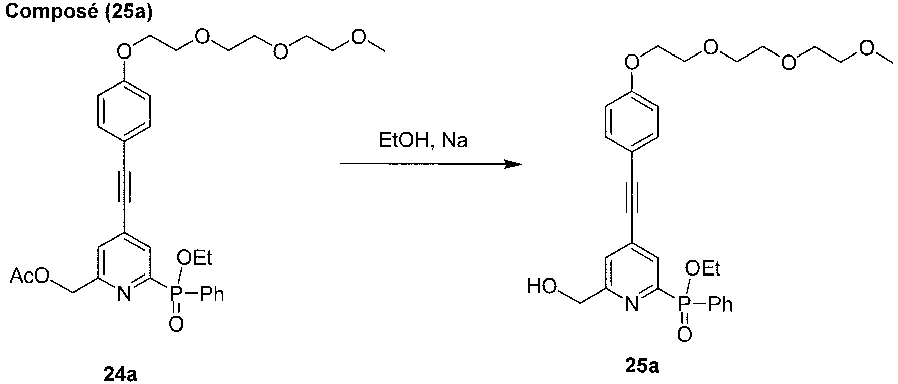

A une solution du dérivé acétylé **24a** (50 mg, 0,086 mmol) dans de l'éthanol absolu (2,5 mL) a été additionnée une quantité catalytique de sodium métal (~2 mg) et la solution a été chauffée sous agitation pendant 40 min sous atmosphère inerte. L'avancement de la réaction a été suivi par CCM. Après cette période, la réaction était totale. A ce mélange a été additionné du dichlorométhane (25 mL) et les sels de sodium ont été ensuite filtrés sur silice. La silice a été rincée avec du dichlorométhane / éthanol à 10% (300 mL). Le solvant a été éliminé sous pression réduite et le produit brut a été purifié par chromatographie sur colonne de silice (dichlorométhane / méthanol 0 à 2,4 % par incréments de 0,2 %) pour obtenir une huile incolore correspondant au composé **25a** (40 mg, 87 %). RMN ¹H (400 MHz, CDCl₃) δ: 8,06 (dd, *J =* 6,0 Hz, 1,6 Hz, 1 H), 7,95 (dd, *J* = 8,4 Hz; 12,4 Hz, 2H), 7,53 (t, *J* = 8,4 Hz, 1 H), 7,44 (d, *J =* 8,8 Hz, 2H), 7,43 (td, *J* = 8,4 Hz; 4,2 Hz, 2H), 7,39 (d, *J* = 1,6 Hz, 1 H), 6,90 (d, *J* = 8,8 Hz), 4,75 (s, 2H), 4,14 (qd, *J* = 5,6 Hz 4,8 Hz, 2H), 4,11 (t, *J* = 4,8 Hz, 2H), 3,86 (t, *J* = 4,8 Hz, 2H), 3,73 (t, *J* = 4,8 Hz, 2H), 3,68 (t, *J* = 4,8 Hz, 2H), 3,66 (t, *J* = 4,8 Hz, 2H), 3,63 (t, *J* = 4,8 Hz, 2H), 3,37 (s, 3H), 1,37 (t, *J* = 5,6 Hz, 3H) ; RMN ¹³C (100 MHz, CDCl₃) δ: 160,4 (d, *J* = 19 Hz), 159,9; 154,0 (d, *J* = 165 Hz), 133,6, 133,0 (d, *J* = 12 Hz), 132,6 (d, *J* = 5 Hz), 132,3 (d, *J* = 10 Hz), 129,7 (d, *J* = 128 Hz), 128,6 (d, *J =* 22 Hz), 128,4 *(d, J* = 12 Hz), 123,8 (d, *J* = 3 Hz), 114,9, 113,8, 96,0, 85,3 *(d, J* = 2 Hz), 71,9, 70,9, 70,7, 70,6, 69,6, 67,5, 63,9, 61,8 (d, *J* = 6 Hz), 59,1, 16,5 ; RMN ³¹P (162 MHz, CDCl₃) δ: +26,6. HMRS (ESI) calculée pour C₂₄H₂₉NO₉S [M+H⁺], *m*/*z* 540,2151 trouvée: 540,2142.

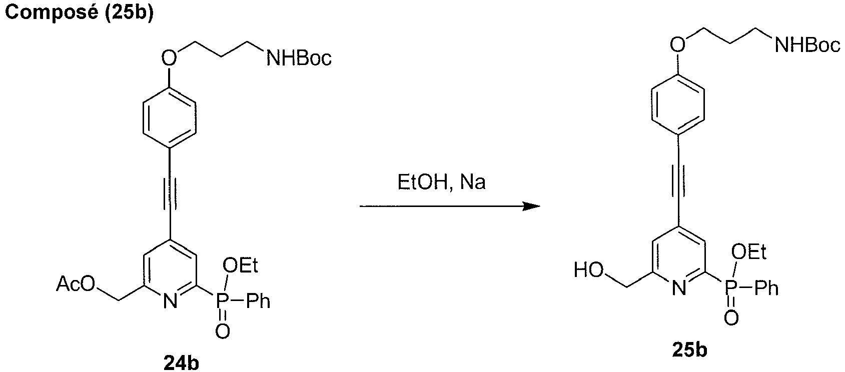

A une solution du dérivé acétylé **24b** (32 mg, 0,054 mmol) dans de l'éthanol absolu (2,5 mL) a été additionnée une quantité catalytique de sodium métal (~2 mg) et la solution a été chauffée sous agitation pendant 40 min sous atmosphère inerte. L'avancement de la réaction a été suivi par CCM. Après cette période, la réaction était totale. A ce mélange a été additionné du dichlorométhane (25 mL) et les sels de sodium ont été ensuite filtrés sur silice. La silice a été rincée avec du dichlorométhane / éthanol à 10% (300 mL). Le solvant a été éliminé sous pression réduite et le produit brut a été purifié par chromatographie sur colonne de silice (dichlorométhane / méthanol 0 à 2 % par incréments de 0,2 %) pour obtenir une huile incolore correspondant au composé **25b** (24 mg, 80 %). RMN ¹H (400 MHz, CDCl₃) δ: 8,01 (dd, *J* = 6,4 Hz, 2,0 Hz, 1 H), 7,89 (dd, *J* = 8,4 Hz, 12,4 Hz, 2H), 7,48 (t, *J* = 8,4 Hz, 1 H), 7,40 (d, *J =* 8,8 Hz, 2H), 7,39 (td, *J* = 8,4 Hz, 4,2 Hz, 2H), 7,33 (d, *J =* 2,0 Hz, 1 H), 6,81 (d, *J* = 8,8 Hz, 2H), 4,73 (s, 1 H), 4,69 (s, 2H), 4,08 (qd, *J* = 5,6 Hz, 4,8 Hz, 2H), 3,97 (t, *J* = 6 Hz, 2H), 3,26 (m, 2H), 1,92 (q, *J* = 6 Hz, 2H), 1,37 (s, 9H, 1,31 (t, *J* = 5,6 Hz, 3H) ; RMN ¹³C (100 MHz, CDCl₃) δ: 160,3 (d, *J* = 18 Hz), 159,8; 156,0; 153,2 (d, *J* = 164 Hz); 133,7; 133,0 (d, *J* = 11 Hz); 132,6 (d, *J* = 5 Hz); 132,3 (d, *J* = 10 Hz); 129,6 (d, *J =* 138 Hz); 128,6 (d, *J* = 18 Hz); 128,5 (d, *J* = 9 Hz); 123,8 (d, *J* = 3 Hz); 114,7; 113,8; 96,0; 85,3 (d, *J* = 2 Hz); 79,3; 65,9; 63,8; 61,9 (d, *J* = 6 Hz); 37,9; 29,5; 28,4; 16,6; RMN ³¹P (162 MHz, CDCl₃) δ: +25,6. HMRS (ESI) calculée pour C₃₀H₃₆O₆N₂P [M+H⁺], *m*/*z* 551,2311 trouvée: 551,2290.

### Composés (25c), (25d), (25e), (25f)

Ces composés ont été obtenus en suivant la procédure utilisée pour les exemples **25a** et **25b.**

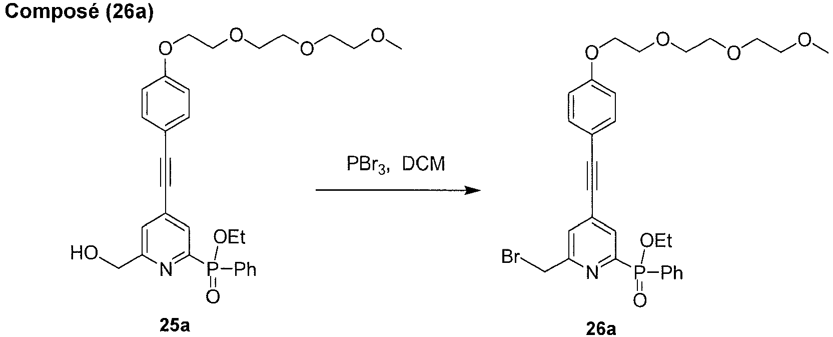

A une solution de l'alcool **25a** (116 mg, 0,22 mmol) dans du dichlorométhane anhydre (5 mL) refroidie à 0°C, a été additionné du tribromure de phosphore (30 µl, 0,33 mmol). La solution a été agitée pendant 1 h à 0 °C puis a été laissée revenir à température ambiante. L'avancement de la réaction a été suivi par CCM. Après 1 h la réaction était totale. Le mélange réactionnel a été immédiatement purifié par chromatographie sur colonne de silice, (dichlorométhane / méthanol, 1 %) pour conduire au composé souhaité **26a** sous la forme d'une huile jaune (95 mg, 73 %) ; RMN ¹H (400 MHz, CDCl₃) δ: 8,00 (dd, *J* = 6,0 Hz, 1,2 Hz, 1 H), 7,94 (dd, *J* = 8,8 Hz, 12,4 Hz, 2H), 7,51 (d, *J* = 1,2 Hz, 1 H), 7,48 (t, *J* = 8,8 Hz, 1 H), 7,41 (d, *J =* 8,8 Hz, 2H), 7,40 (td, *J =* 8,8 Hz, 4,2 Hz, 2H), 6,85 (d, *J* = 8,8 Hz, 2H), 4,48 (s, 2H), 4,09 (qd, *J =* 7,2 Hz, 4,8 Hz, 2H), 4,08 (t, *J* 4,8 Hz, 2H), 3,81 (t, *J* = 4,8 Hz, 2H), 3,67 (t, *J* = 4,8 Hz, 2H), 3,63 (t, *J* = 4,8 Hz, 2H), 3,59 (t, *J* = 4,8 Hz, 2H), 3,49 (t, *J* = 4,8 Hz), 3,31 (s, 3H), 1,32 (t, *J* = 7,2 Hz, 3H) ; RMN ¹³C (100 MHz, CDCl₃) δ: 160,1 (d, *J* = 19 Hz); 159,9; 154,3 (d, *J* = 165 Hz); 133,6; 133,3 (d, *J* = 12 Hz); 132,4 (d, *J* = 5 Hz); 132,3 (d, *J* = 10 Hz); 129,7 (d, *J* = 128 Hz); 128,6 (d, *J* = 22 Hz); 128,4 (d, *J* = 13 H); 126,7 (d, *J* = 3 Hz); 114,9; 113,8; 96,3; 84,9 (d, *J* = 2 Hz); 71,9; 70,9; 70,7; 70,6; 69,6; 67,5; 61,0 (d, *J* = 5 Hz); 59,1; 33,1; 16,6; RMN ³¹P (162 MHz, CDCl₃) δ: +24,8 ; HMRS (ESI) calculée pour C₂₉H₃₄O₆NPBr [M+H⁺], *m*/*z* 602,1302, trouvée: 602,1302.

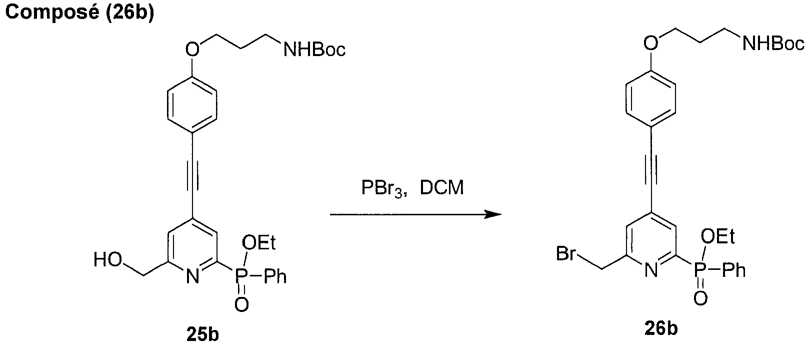

A une solution d'alcool **25b** (25 mg, 0,045 mmol) dans du dichlorométhane anhydre (3 mL) refroidie à 0°C, a été additionné du tribromure de phosphore (30 µl, 0,33 mmol). La solution a été agitée pendant 45 min à 0 °C puis a été laissée revenir à température ambiante. L'avancement de la réaction a été suivi par CCM. Après 30 min la réaction était totale. Le mélange réactionnel a été immédiatement purifié par chromatographie sur colonne de silice, (dichlorométhane / méthanol 1 %) pour conduire au composé souhaité **26b** sous la forme d'une huile jaune (13 mg, 47 %) ; RMN ¹H (400 MHz, CDCl₃) δ: 8,07 (dd, *J* = 6,0 Hz, 1,2 Hz, 1 H), 8,01 (dd, *J* = 8,4 Hz *J =* 12 Hz, 2H), 7,58 (d, *J* = 1,2 Hz, 1 H), 7,55 (t, *J* = 8,4 Hz, 1 H), 7,47 (td, *J* = 8,4 Hz, 4,2 Hz, 2H), 7,46 (d, *J* = 8,8 Hz, 2H), 6,89 (d, *J* = 8,8 Hz, 2H), 4,72 (s, 1 H), 4,55 (s, 2H), 4,15 (qd, *J* = 6,0 Hz, 4,8 Hz, 2H), 4,05 (t, *J* = 6 Hz, 2H), 3,33 (m, 2H), 2,00 (q, *J* = 6 Hz, 2H), 1,44 (s, 9H), 1,38 (t, *J* = 6,0 Hz, 3H) ; RMN ³¹P (162 MHz, CDCl₃) δ:+25,8. HMRS (ESI) calculée pour C₃₀H₃₄O₅N₂PBr [M+H⁺], *m*/*z* 613,1462, trouvée: 613,1462.

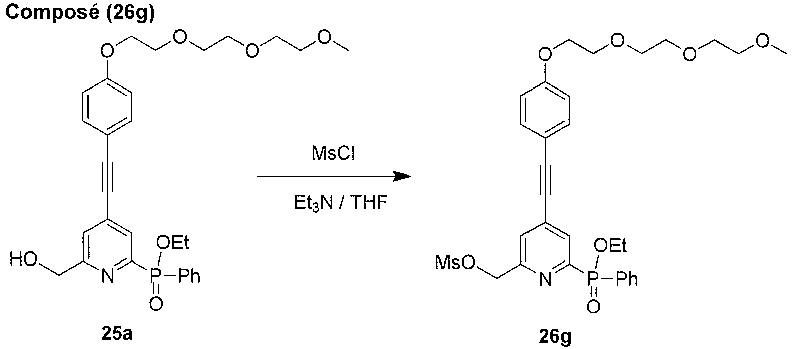

A une solution d'alcool **25a** (360 mg 0,66 mmol) dans du tétrahydrofurane anhydre (4 mL) refroidie à 5°C ont été ajoutés de la triéthylamine (0,3 mL, 1,98 mmol) puis du chlorure de mésyle (77 µL, 0,99 mmol) sous atmosphère inerte. Le milieu réactionnel a été agité à température ambiante pendant 15 min. L'avancement de la réaction a été suivi par CCM. Après cette période, la réaction était totale. Le solvant a été éliminé sous pression réduite. Au résidu a été additionnée de l'eau saturée en chlorure de sodium (30 mL) et le mélange a été extrait avec du dichlorométhane (2 x 30 mL). Les phases organiques ont été regroupées et séchées sur sulfate de magnésium. Après filtration, le solvant a été éliminé sous pression réduite pour conduire à une huile jaunâtre correspondant au composé **26g** (430 mg, 99%) qui était suffisamment pur pour être utilisé dans l'étape suivante sans purification complémentaire.

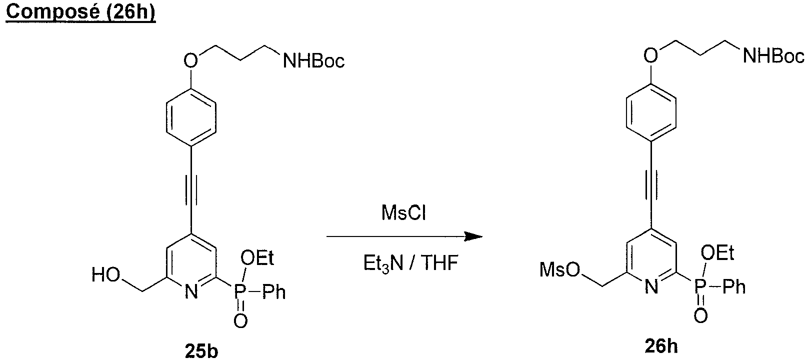

A une solution d'alcool **25b** (118 mg 0,21 mmol) dans du tétrahydrofurane anhydre (7 mL) refroidie à 5°C ont été ajoutés de la triéthylamine (87 µL, 0,64 mmol) puis du chlorure de mésyle (24 µL, 0,32 mmol) sous atmosphère inerte. Le milieu réactionnel a été agité à température ambiante pendant 15 min. L'avancement de la réaction a été suivi par CCM. Après cette période, la réaction était totale. Le solvant a été éliminé sous pression réduite. Au résidu a été additionnée de l'eau saturée en chlorure de sodium (30 mL) et le mélange a été extrait avec du dichlorométhane (2 x 30 mL). Les phases organiques ont été regroupées et séchées sur sulfate de magnésium. Après filtration, le solvant a été éliminé sous pression réduite pour conduire à un solide marron correspondant au composé **26h** (125 mg, 95%) qui était suffisamment pur pour être utilisé dans l'étape suivante sans purification complémentaire.

### Composés (26i), (26j), (26k), (26I)

Ces composés ont été obtenus en suivant la procédure utilisée pour les exemples **26g** et **26h.**

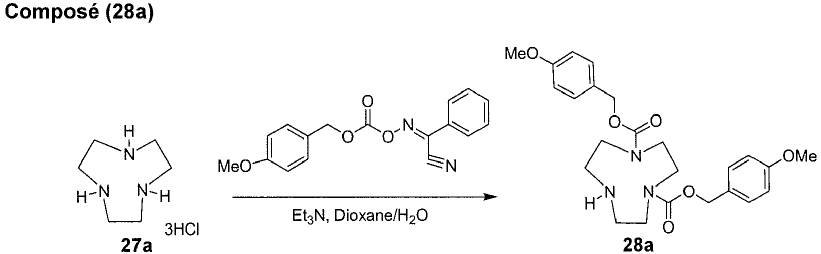

A une solution d'hydrochlorure de 1,4,7-triazacyclononane **27a** (188 mg, 0,78 mmol) dans un mélange dioxane/eau (10 mL, 8:2), a été ajouté le 2-(4-méthoxybenzyloxycarbonyloxyimino)-2-phénylacétonitrile (477 mg, 1,5 mmol). Le mélange réactionnel a été homogénéisé par une agitation vigoureuse puis a été ajoutée une solution de triéthylamine (540 µL, 3,8 mmol) dans un mélange dioxane/eau (10 mL, 8:2). L'avancement de la réaction a été suivi par CCM. Après 24 h, la réaction était totale. Le solvant a été éliminé sous pression réduite. Le résidu a été dissous dans du dichlorométhane. Cette solution a été lavée avec de l'eau saturée en chlorure de sodium (3 × 10 mL). La phase organique a été séchée sur sulfate de magnésium, filtrée et concentrée sous pression réduite. Le brut a été purifié par chromatographie sur colonne d'alumine (dichlorométane / méthanol 98:2 jusqu'à 90:10 par incrément de 1%) pour conduire au composé souhaité **28a** sous la forme d'une huile (266 mg, 74 %). RMN ¹H (400 MHz, CDCl₃) δ: 7,32-7,25 (m, 4H), 6,88-6,85 (m, 4H), 5,09 (m, 4H), 3,80 (s, 6H), 3,56-3,47 (m, 4H), 3,31-3,25 (m, 4H), 2,87 (m, 4H). RMN ¹³C (100 MHz, CDCl₃) δ: 159,68; 156,76; 130,05; 128,92; 114,03; 67,15; 55,41; 52,20; 51,98; 51,26; 50,31; 50,07; 49,19; 48,18; 47,73; 47,66.

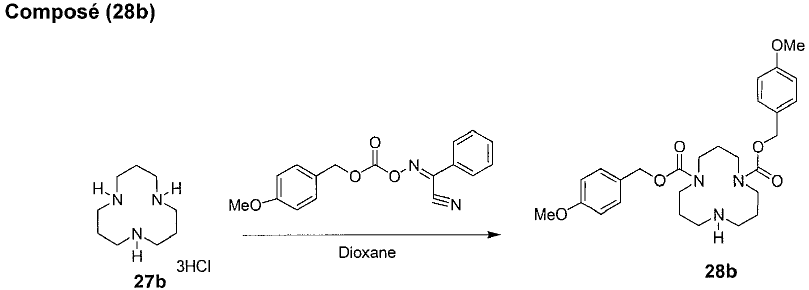

A une solution de 1,4,7-triazacyclododecane **27b** (1 g, 5,84 mmol) dans du dioxane (50 mL), a été ajouté le 2-(4-méthoxybenzyloxycarbonyloxyimino)-2-phénylacétonitrile (3,62 g, 11,7 mmol). Le mélange réactionnel a été homogénéisé par une agitation vigoureuse puis a été agité à température ambiante sous atmosphère inerte pendant 2 h. L'avancement de la réaction a été suivi par CCM. Après cette période, la réaction était totale. Le solvant a été éliminé sous pression réduite. Le résidu a été dissous dans du dichlorométhane (50 mL). Cette solution a été lavée avec de l'eau saturée en chlorure de sodium (2 × 20 mL). Les phases aqueuses ont été réunies et ont été extraites par du dichlorométhane (25 mL). Les phases organiques ont été réunies, séchées sur sulfate de magnésium, filtrées et concentrées sous pression réduite. Le brut a été purifié par chromatographie sur colonne d'alumine (dichlorométane / méthanol 100 : 0 jusqu'à 97:3 par incrément de 0,5%) pour conduire au composé souhaité **28b** sous la forme d'une huile (980 mg, 34 %). HMRS (ESI) calculée pour C₂₇H₃₇N₃O₆ [M+H⁺], *m*/*z* 500,2761, trouvée: 500,2758.

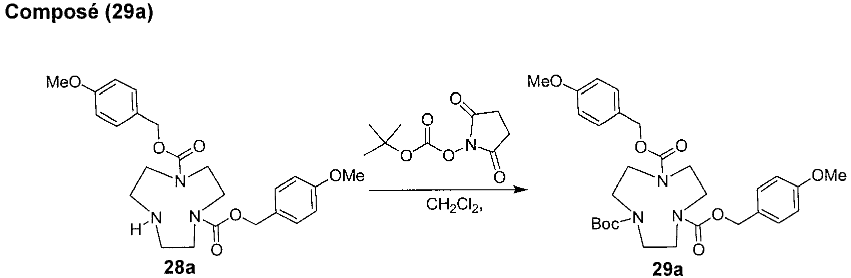

A une solution de macrocycle diprotégé **28a** (50 mg, 0,1 mmol) dans du dichlorométhane (7 mL), a été ajouté le N-(*tert*-butoxycarbonyloxy)succinimide (36 mg, 0,15 mmol). La solution a été agitée à température ambiante sous atmosphère inerte. L'avancement de la réaction a été suivi par CCM. Après 24 h, la réaction était totale. La solution a été directement lavée avec de l'eau saturée en chlorure de sodium (3 × 10 mL). La phase organique a été séchée sur sulfate de magnésium, filtrée et concentrée sous pression réduite. Le brut a été purifié par chromatographie sur colonne d'alumine (dichlorométhane/méthanol 90:10 jusqu'à 70:30 par incrément de 5%) pour conduire au composé souhaité **29a** sous la forme d'une huile de couleur brune (44 mg, 72%). RMN ¹H (400 MHz, CDCl₃) δ: 7,30-7,19 (m, 4H), 6,88-6,83 (m, 4H), 5,06-4,92 (m, 4H), 3,77 (s, 6H), 3,46-3,39 (m, 12H), 1,55 (s, 9H). RMN ¹³C (100 MHz, CDCl₃) δ: 159,65; 156,56; 155,76; 130,04; 128,98; 114,00; 80,01; 67,15; 55,38; 49,74; 49,64; 49,47;49,37; 49,12; 48,92; 48,80; 28,53; 27,75; 25,60. HMRS (ESI) calculée pour C₂₉H₃₉N₃O₈ [M+H⁺], *m*/*z* 558,2815, trouvée: 558,2808.

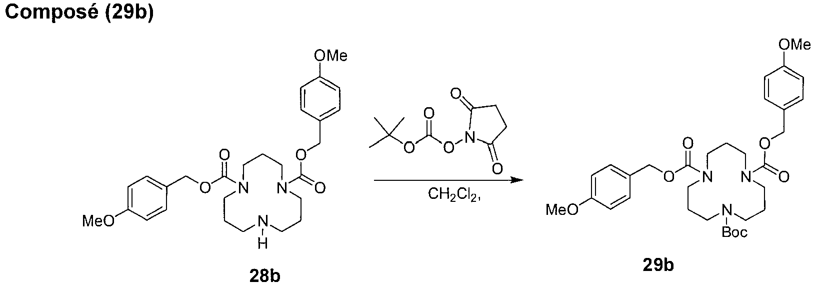

A une solution de macrocycle diMOZ **28b** (980 mg, 1,96 mmol) dans du dichlorométhane (40 mL), a été ajouté le N-(*tert*-butoxycarbonyloxy)succinimide (630 mg, 2,93 mmol). Le mélange réactionnel a été agité à température ambiante pendant 24 h. L'avancement de la réaction a été suivi par CCM. Après cette période, la réaction était totale. Cette solution a été lavée avec de l'eau saturée en chlorure de sodium (2 × 20 mL). Les phases aqueuses ont été réunies et ont été extraites par du dichlorométhane (2 × 30 mL). Les phases organiques ont été réunies, séchées sur sulfate de magnésium, filtrées et concentrées sous pression réduite. Le brut a été purifié par chromatographie sur colonne d'alumine (cyclohexane/acétate d'éthyle 100:0 jusqu'à 70:30 par incrément de 5 %) pour conduire au composé souhaité **29b** sous la forme d'une huile (507 mg, 42 %).

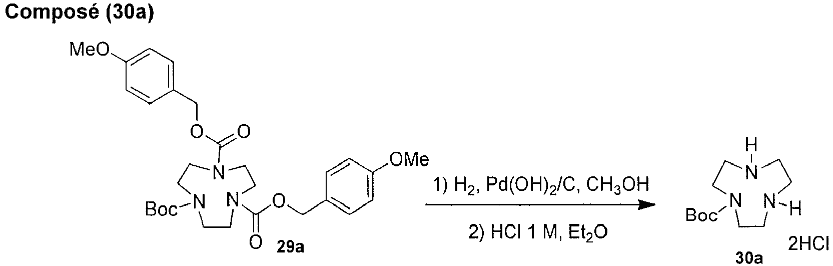

A une solution de macrocycle diMOZ-monoBoc **29a** (300 mg, 0,5 mmol) dans le méthanol (25 mL) a été ajouté de l'hydroxyde de palladium absorbé sur charbon (environ 100 mg). Le milieu réactionnel a été placé dans un hydrogénateur et a été maintenu sous une vive agitation et sous atmosphère d'hydrogène (3,45 bars - 50 PSI). L'avancement de la réaction a été suivi par CCM d'alumine. Après une nuit, la réaction était totale. Le milieu réactionnel a été filtré sur Celite puis concentré sous pression réduite. L'huile incolore obtenue a été dissoute dans du méthanol (3 mL). A cette solution a été ajoutée une solution aqueuse d'acide chlorhydrique à 1M (pH 2-3). Le mélange a été concentré sous pression réduite et a été redissous dans un minimum de méthanol (2 mL). A cette solution, a été ajouté de l'éther diéthylique (35 mL). Le précipité blanc obtenu a été recueilli par filtration pour donner un solide blanc correspondant à l'hydrochlorure **30a** (102 mg, 62%). PtF: 172-174°C. RMN ¹H (500 MHz, D₂O) δ: 3,74 (t, *J* = 4,5 Hz, 4H), 3,63 (s, 4H), 3,48 (t, *J* = 4,5 Hz, 4H), 1,47 (s, 9H). RMN ¹³C (125 MHz, CDCl₃) δ:136,0 ; 62,9; 25,9; 22,2; 21,7 ; 6,7. HMRS (ESI) calculée pour C₁₁H₂₃N₃O₂ [M+H⁺], *m*/*z* 230,1869, trouvée: 230,1863.

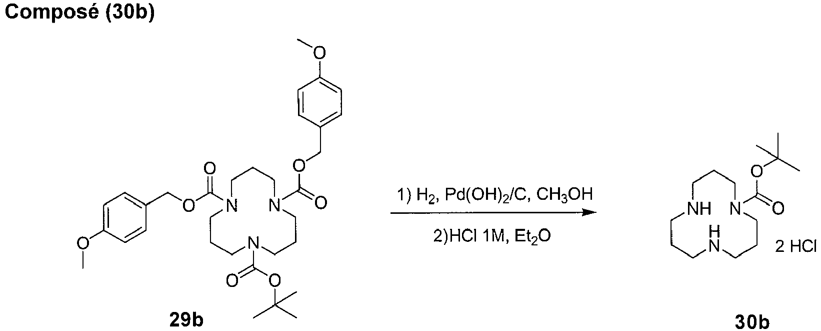

A une solution de macrocycle diMOZ-monoBoc **29b** (507 mg, 0,846 mmol) dans le méthanol (25 mL) a été ajouté de l'hydroxyde de palladium absorbé sur charbon (environ 100 mg). Le milieu réactionnel a été placé dans un hydrogénateur et a été maintenu sous une vive agitation et sous atmosphère d'hydrogène (3,45 bars - 50 PSI). L'avancement de la réaction a été suivi par LC-MS. Après une nuit, la réaction était totale. Le milieu réactionnel a été filtré sur Celite puis concentré sous pression réduite. L'huile incolore obtenue a été dissoute dans du méthanol (2 mL). A cette solution a été ajoutée une solution aqueuse d'acide chlorhydrique à 1 M (pH 2-3). Le mélange a été concentré sous pression réduite et a été redissous dans un minimum de méthanol (1 mL). A cette solution, a été ajouté de l'éther diéthylique (150 mL). Le précipité blanc obtenu a été recueilli par filtration sur fritté pour donner un solide blanc correspondant à l'hydrochlorure **30b** (125 mg, 43%). PtF: 149-168°C.

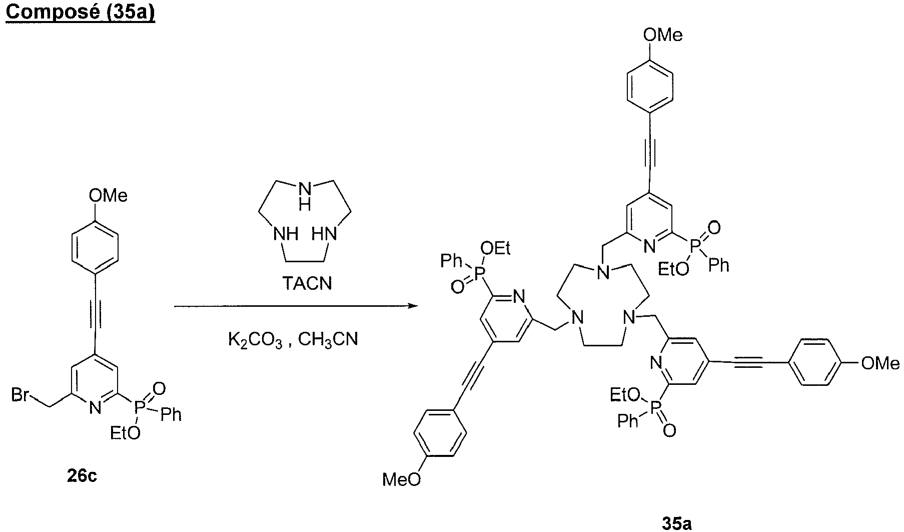

A une solution de dérivé bromé **26c** (110 mg, 0,234 mmol) dans de l'acétonitrile anhydre (5 mL), ont été ajoutés sous atmosphère inerte du carbonate de potassium (32 mg, 0,234 mmol) et du 1,4,7-triazacyclononane (10 mg, 0,078 mmol). Le milieu réactionnel a été chauffé à reflux pendant 16 h. L'avancement de la réaction a été suivi par CCM. Après cette période, la réaction était totale. Le milieu réactionnel a été refroidi à température ambiante et le solvant a été éliminé sous pression réduite. Au résidu a été additionnée de l'eau (10 mL) et le mélange a été extrait avec de l'acétate d'éthyle (2 x 20 mL). Les phases organiques ont été regroupées et séchées sur sulfate de magnésium. Après filtration, le solvant a été éliminé sous pression réduite. Le produit brut obtenu a été purifié par chromatographie sur colonne de silice (dichlorométhane/méthanol, 0 jusqu'à 30% par incrément de 1%) pour conduire au composé **35a** sous forme d'une huile jaunâtre (23 mg, 23%). RMN ¹H (400 MHz, CDCl₃) δ: 8,03 (dd, *J =* 6,0 Hz, 3H), 7,94 (dd, *J* = 12,4 Hz, *J* = 6,8 Hz, 6H), 7,58 (s, 3H), 7,45 (d, *J* = 8,8 Hz, 6H), 7,42 (t, *J* = 6,8 Hz, 3H), 7,37 (td, *J* = 6,8 Hz *J* = 3,6 Hz, 6H), 6,87 (d, *J* = 8,8 Hz, 6H), 4,10 (qd, *J* = 7,2 Hz, *J =* 4,2 Hz, 6H), 3,83 (s, 6H), 3,82 (s, 9H), 2,74 (s, 12H), 1,33 (t, *J* = 7,2 Hz, 9H); RMN ³¹P (162 MHz, CDCl₃) δ: +26.6; HMRS (ESI) calculée pour C₇₅H₇₆N₆O₉P₃ [M+H⁺], *m*/*z* 1297,4860, trouvée: 1297,4860.

### Composés (35b) et (35c)

Ces composés ont été obtenus en suivant la procédure utilisée pour l'exemple **35a.**

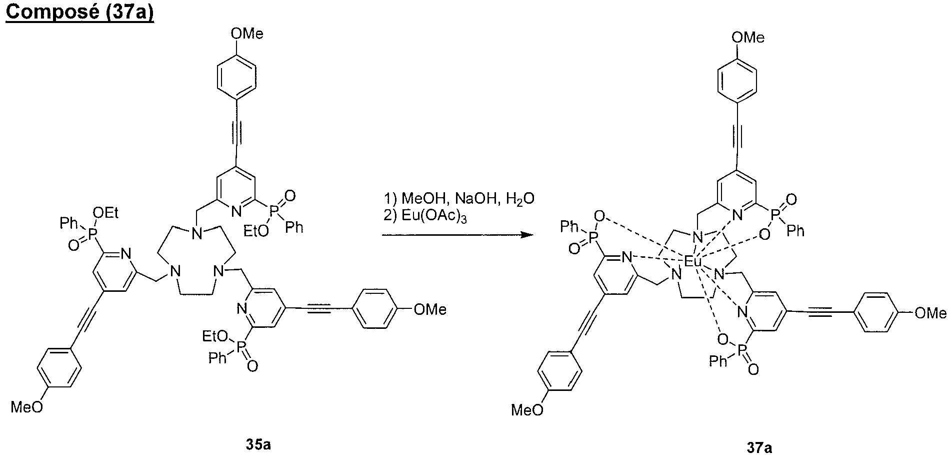

A une solution du ligand **35a** (10 mg, 7,7 µmol) dans le méthanol (3 mL) a été additionnée une solution aqueuse d'hydroxyde de sodium (1 mL, 0,1 M). La solution a été chauffée à 60°C pendant 16 h. L'avancement de la réaction a été suivi par RMN ¹H. Après cette période la réaction était totale. Le pH de la solution a été ajusté à 7 par addition d'acide chlorhydrique (1 M). A cette solution a été additionné de l'acétate d'europium (3,4 mg, 8,5 µmol) dissous préalablement dans une solution H₂O:CH₃OH (0,5 mL, 1:1 v/v). Le mélange réactionnel a été chauffé à 50°C pendant 24 h. Le mélange réactionnel a été refroidi à température ambiante et le solvant a été éliminé sous pression réduite et le produit brut a été purifié par chromatographie sur colonne de silice (dichlorométhane / méthanol 5%) pour donner le composé désiré **37a** sous la forme d'un solide blanc (2,5 mg, 24%). RMN ³¹P (162 MHz, CDCl₃) δ: +17.5. HMRS (ESI) calculée pour C₆₉H₆₁N₆O₉P₃Eu [M+H⁺], *m*/*z* 1361,2910, trouvée: 1361,2830.

### Composés (37b) et (37c)

Ces composés ont été obtenus en suivant la procédure utilisée pour l'exemple **37a.**

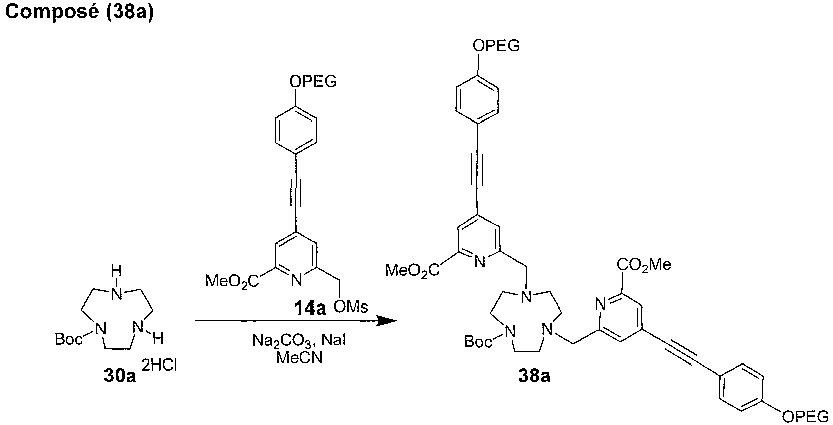

A une solution d'hydrochlorure de TACN monoBoc **30a** (20,2 mg, 67 µmol) dans l'acétonitrile anhydre (20 mL), a été ajouté du carbonate de potassium (55,5 mg, 0,40 mmol) sous atmosphère inerte. A cette suspension, a été ajoutée une solution de chromophore OPEG mésylé **14a** (70 mg, 0,14 mmol) dans l'acétonitrile anhydre (2 mL). L'avancement de la réaction a été suivi par HPLC analytique. Après agitation et chauffage à 60°C pendant 2h, le milieu réactionnel a été refroidi à température ambiante. Le solvant a été éliminé sous pression réduite. Le brut réactionnel a été purifié par chromatographie sur colonne de silice (dichlorométhane / méthanol 98 :2 jusqu'à 80 :20 par incréments de 2%) pour conduire au composé **38a** sous forme d'une huile (52.8 mg, 75%). HMRS (ESI) calculée pour C₅₇H₇₃N₅O₁₄ [M+H⁺], *m*/*z* 1052,5232, trouvée: 1052,5256.

### Composé (38b)

Ce composé a été obtenu en suivant la procédure utilisée pour l'exemple **38a.**

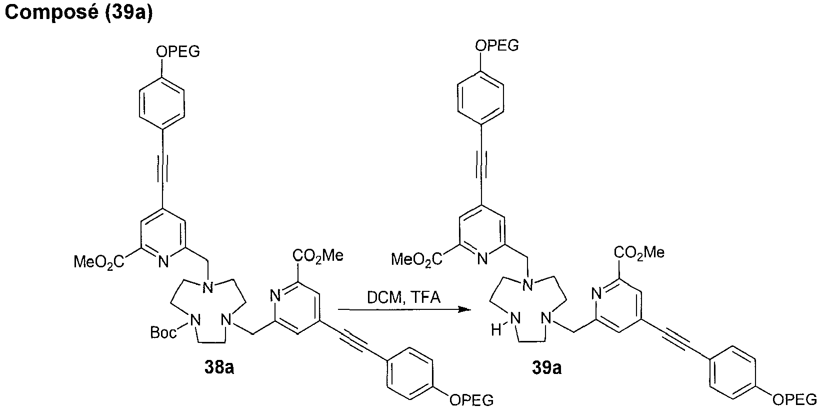

A une solution de TACN monoBoc-dibras OPEG **38a** (1,47 mg, 1,4 µmol) dans du dichlorométhane (950 µL) a été ajouté de l'acide trifluoroacétique (50 µL). L'avancement de la réaction a été suivi par HPLC. Après agitation à température ambiante pendant une nuit, le solvant a été éliminé sous pression réduite. Le milieu a été repris dans du méthanol (1 mL) et du toluène (3 mL) puis a été évaporé afin d'éliminer les traces d'acide trifluoroacétique. Le milieu réactionnel a été purifié par HPLC sur colonne préparative conduisant au composé **39a** sous la forme d'une huile jaunâtre (0,75 mg, 56%). HMRS (ESI) calculée pour C₅₂H₆₅N₅O₁₂ [M+H⁺], *m*/*z* 952,4708, trouvée: 952,4714.

### Composé (39b)

Ce composé a été obtenu en suivant la procédure utilisée pour l'exemple **39a.**

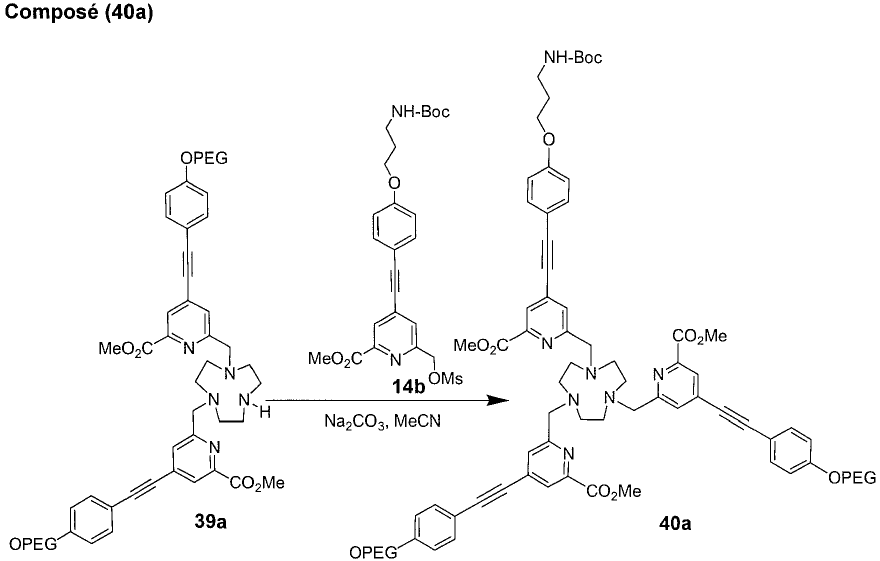

A une solution de TACN dibras OPEG **39a** (4,25 mg, 4,4 µmol) dans l'acétonitrile anhydre (4 mL), a été ajouté du carbonate de sodium (1,4 mg, 1,34 µmol) sous atmosphère inerte. A cette suspension, a été ajoutée une solution de chromophore NH-Boc mésylé **14b** (3,5 mg, 6,7 µmol) dans l'acétonitrile anhydre (2 mL). L'avancement de la réaction a été suivi par HPLC. Après agitation et chauffage à 50°C pendant 22 h, le milieu réactionnel a été refroidi à température ambiante. Le solvant a été éliminé sous pression réduite. Le milieu réactionnel a été purifié par HPLC sur colonne préparative. Le composé **40a** a été obtenu sous la forme d'une huile jaunâtre (1,25 mg, 21%).

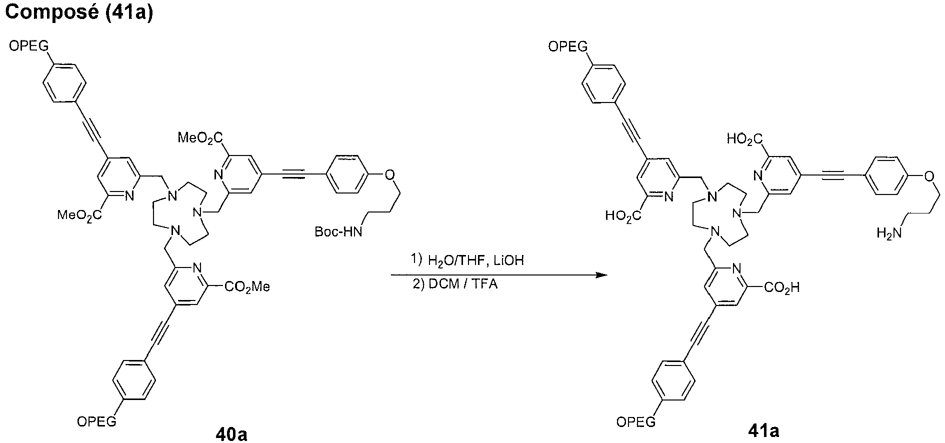

A une solution de ligand triester méthylique **40a** (1 mg, 727 nmol) dans du THF (150 µL), ont été ajoutés de l'hydroxyde de lithium à 1 M (50 µmol) et de l'eau pure (100 µL). A cette suspension blanchâtre, a été ajouté du THF (1 mL) pour parfaire la solubilisation. Le mélange réactionnel a été agité à température ambiante pendant 2 h. L'avancement de la réaction a été suivi par HPLC. Après cette période, la réaction était totale. Le solvant a été éliminé sous pression réduite. Le milieu réactionnel a été alors repris dans de l'eau pure (500 µL) et a été acidifié par l'addition d'une solution aqueuse d'acide chlorhydrique 1M pour obtenir le pH du mélange compris entre 2-3. Le solvant a été éliminé sous pression réduite. Le résidu a été purifié par HPLC préparative conduisant au produit souhaité sous la forme d'une huile (312 nmol, 43%). A l'huile obtenue précédemment et d'un deuxième lot (1 µmol) dans du dichlorométhane (500 µL) ont été ajoutés de l'acide trifluoroacétique (150 µL) puis un volume supplémentaire de dichlorométhane (2500 µL). Le mélange réactionnel a été agité à température ambiante pendant 10 min. L'avancement de la réaction a été suivi par HPLC. Après cette période, la réaction était totale. Le solvant a été éliminé sous pression réduite pour conduire au composé **41a** sous la forme d'une huile (530 nmol, 53%) qui a été utilisé dans l'étape suivante sans autre purification.

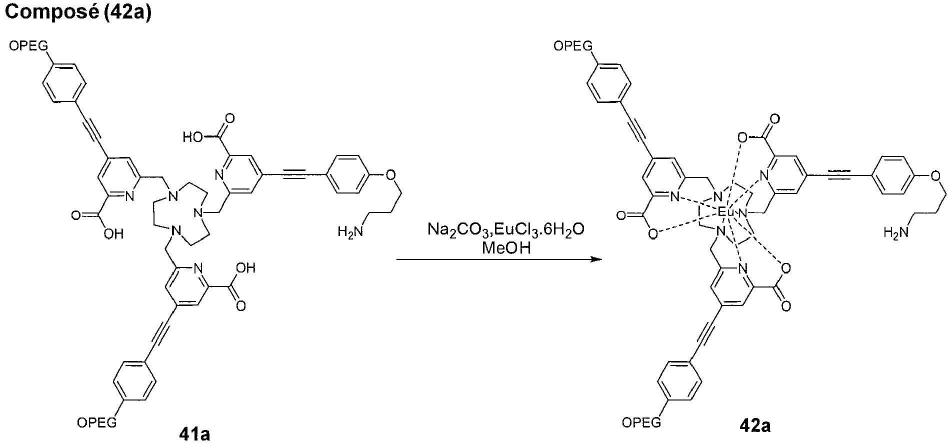

A une solution de TACN tribras asymétrique acides carboxyliques -NH₂ **41a** (16,4 µmol) dans du méthanol (5 mL) et de l'eau (2 mL), ont été ajoutés du carbonate de sodium (10,4 mg, 98,4 µmol) et du chlorure d'europium hexahydraté (16,6 mg, 32,8 µmol). Le mélange réactionnel a été chauffé à 50°C pendant une nuit. L'avancement de la réaction a été suivi par HPLC analytique. Après cette période la réaction était totale. Le milieu réactionnel a été refroidi à température ambiante puis le solvant a été éliminé sous pression réduite. Le résidu a été purifié par HPLC préparative conduisant au complexe **42a** sous la forme d'une huile incolore (9,15 µmol, 56%).

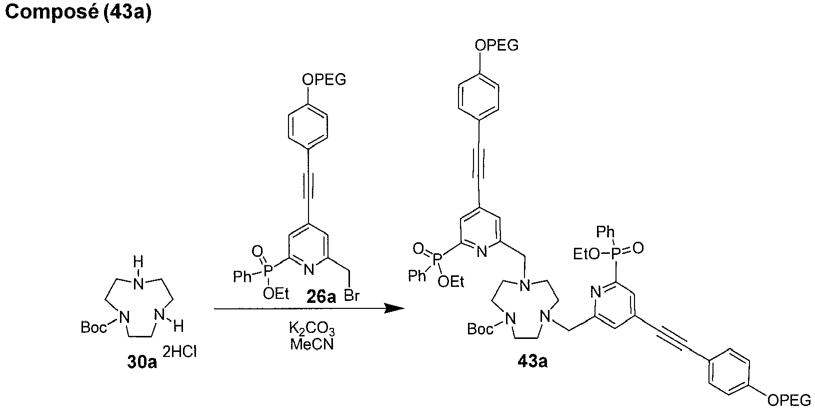

A une solution de 1,4,7-triazacyclononane-mono Boc **30a** (22 mg, 0,096 mmole) dans de l'acétonitrile (5 mL) ont été ajoutés le composé **26a** (95 mg, 0,16 mmole) et du carbonate de potassium (27 mg, 0,19 mmole). Le mélange a été agité sous argon à 50 °C pendant 1 h. L'avancement de la réaction a été suivi par CCM. Après cette période, la réaction était totale. Le mélange réactionnel a été refroidi à température ambiante puis le solvant a été éliminé sous pression réduite. Le résidu a été purifié par chromatographie sur colonne de silice (dichlorométhane / méthanol 0 à 5 % par incréments de 0,5 %) pour obtenir le composé souhaité **43a** sous la forme d'une huile jaune (58 mg, 59 %) : RMN ¹H (400 MHz, CDCl₃) δ: 8,01 (m, 2H), 7,95 (m, 4H), 7,56 (s, 2H), 7,45 (m, 2H), 7,41 (d, *J* = 8,8 Hz, 4H), 7,40 (m, 4H), 6,87 (d, *J* = 8,8 Hz, 4H), 4,13 (t, *J* = 4,8 Hz, 4H), 4,10 (m, 4H), 3,85 (s, 4H), 3,84 (t, *J* = 4,8 Hz, 4H), 3,72 (t, *J* = 4,8 Hz, 4H), 3,66 (t, *J* = 4,8 Hz, 4H), 3,63 (t, *J* = 4,8 Hz, 4H), 3,53 (t, *J* = 4,8 Hz, 4H), 3,35 (s, 6H), 3,24 (m, 4H), 2,97 (m, 4H), 2,53 (m, 4H), 1,45 (s, 9H), 1,33 (m, 6H) ; RMN ¹³C (100 MHz, CDCl₃) δ: 161,6; 161,4 *(d, J* = 19 Hz); 159,7; 159,6; 155,5; 153,8; 153,7 (d, *J* = 165 Hz); 133,6; 133,5; 133,3; 132,4; 132,3; 130,1 (d, *J* = 145 Hz); 128,1; 128,0; 126,2; 125,9 (d, *J =* 3 Hz); 114,9; 114,7; 114,1; 114,0; 95,4; 95,1; 85,7; 85,5; 71,9; 70,8; 70,6; 70,5; 69,6; 67,5; 63,0; 49,1; 61,6; 59,0; 53,4; 28,7; 28,6; 16,5; RMN ³¹P (162 MHz, CDCl₃) δ: +26,6, +26,5 HMRS (ESI) calculée pour C₆₉H₈₈N₅O₁₄P₂ [M+H⁺], *m*/*z* 1272,5840, trouvée: 1272,5800.

### Composé (43c)

Ce composé a été obtenu en suivant la procédure utilisée pour l'exemple **43a.**

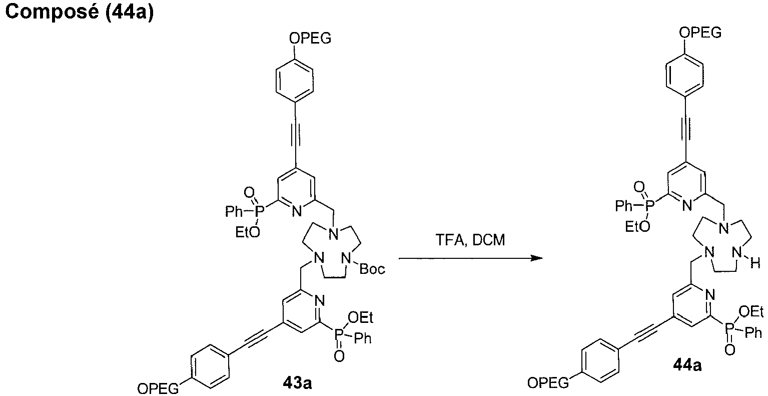

A une solution de **43a** (47 mg, 0,037 mmol) dans du dichlorométhane anhydre (1 mL) a été ajouté de l'acide trifluoroacétique (0,5 mL). La solution a été agitée sous argon à 23 °C pendant 30 min. L'avancement de la réaction a été suivi par CCM. Après cette période la réaction était totale. Le solvant a été éliminé sous pression réduite et le résidu a été redissous dans du dichlorométhane (1 mL), qui a été à nouveau éliminé sous pression réduite. Cette procédure a été répétée 5 fois pour éliminer l'acide trifluoroacétique en excès. Le résidu a été utilisé dans la suite de la synthèse sans purification supplémentaire. HMRS (ESI) calculée pour C₆₄H₈₀N₅O₁₂P₂ [M+H⁺], *m*/*z* 1172,5270, trouvée: 1172,5280.

### Composé (44c)

Ce composé a été obtenu en suivant la procédure utilisée pour l'exemple **44a.**

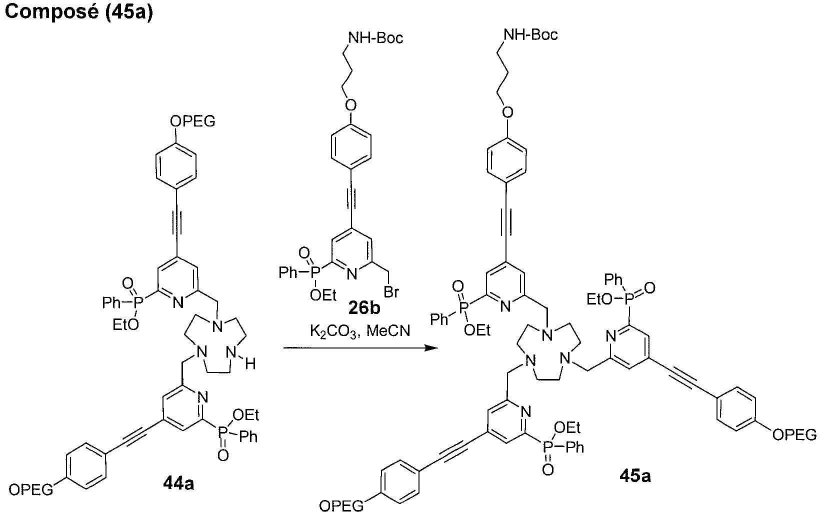

Au résidu **44a** obtenu dans l'étape précédente dissous dans de l'acétonitrile (4 mL) ont été ajoutés le dérivé bromé **26b** (18 mg, 0,029 mmol) et du carbonate de potassium (15 mg, 0,11 mmol). La suspension a été agitée à 50 °C pendant 30 min. L'avancement de la réaction a été suivi par CCM. Après cette période la réaction était totale. Le mélange réactionnel a été refroidi et le solvant a été éliminé sous pression réduite. Le résidu a été purifié par chromatographie sur colonne de silice (dichlorométhane / méthanol 0 à 10 % par incréments de 0,5 %) pour obtenir le composé souhaité **45a** sous la forme d'une huile jaune (25 mg, 50 %). RMN ¹H (400 MHz, CDCl₃) δ: 8,07 (d, *J* = 6,0 Hz, 3H), 7,85 (dd, *J* = 8,4, *J* = 12 Hz, 6H), 7,48 (m, 3H), 7,47 (m, 3H), 7,46 (d, *J* = 8,8 Hz, 6H), 7,39 (m, 6H), 6,90 (d, *J =* 8,8 Hz, 6H), 4,76 (s, 1H), 4,14 (m, 6H), 4,11 (t, *J* = 4,8 Hz, 4H), 4,04 (t, *J* = 6 Hz, 2H), 3,94 (s, 6H), 3,87 (t, *J =* 4,8 Hz, 4H), 3,73 (t, *J* = 4,8 Hz, 4H), 3,68 (t, *J* = 4,8 Hz, 4H), 3,65 (t, *J* = 4,8 Hz, 4H), 3,54 (t, *J =* 4,8 Hz, 4H), 3,37 (s, 6H), 3,34 (m, 2H), 2,81 (m, 12H), 1,99 (q, *J* = 6 Hz, 2H), 1,43 (s, 9H), 1,34 (t, *J* = 7,2 Hz, 9H) ; RMN ¹³C (100 MHz, CDCl₃) δ: 160,8 (d, *J* = 19 Hz), 160,2; 156,2; 154,6 (d, *J* = 165 Hz); 134,0; 133,9; 132,9; 132,5 (d, *J* = 10 Hz); 130,2 (d, *J* = 138 Hz); 128,8; 128,7; 127,5; 115,1; 113,9; 97,0; 85,3; 79,6; 72,1; 71,1; 70,9; 70,8; 69,8; 67,8; 65,9; 62,0 (d, *J* = 6 Hz); 59,3; 52,3; 53,0 à 46,0; 37,9; 29,7; 28,6; 16,8; RMN ³¹P (162 MHz, CDCl₃) δ: +26,4. HMRS (ESI) calculée pour C₉₄H₁₁₃N₇O₁₇P₃ [M+H⁺], *m*/*z* 1704,7400, trouvée: 1704,7410.

### Composé (45c)

Ce composé a été obtenu en suivant la procédure utilisée pour l'exemple **45a.**

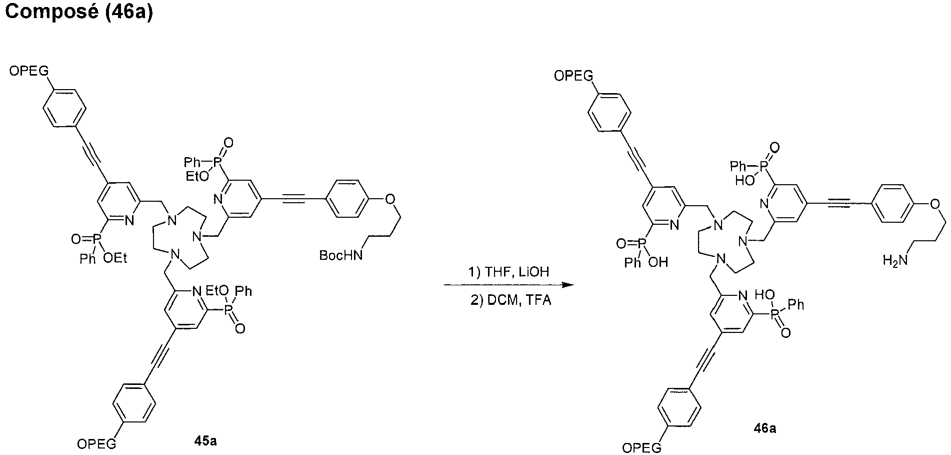

Au ligand **45a** (52 mg, 30,5 µmol) ont été additionnés du tétrahydrofurane (6 mL) et une solution aqueuse d'hydroxyde de lithium (4 mL, 1 M). La solution a été agitée à température ambiante pendant 24 h. L'avancement de la réaction a été suivi par HPLC. Après cette période, la réaction était totale. Le mélange réactionnel a été concentré sous pression réduite et utilisé dans la suite de la synthèse sans purification complémentaire. MS (ESI) calculée pour C₈₈H₁₀₃N₇O₁₇P₃ [M+3H⁺], *m*/*z* 1622. A une solution du composé obtenu précédemment dans du dichlorométhane (6 mL) a été ajouté de l'acide trifluoroacétique (3 mL). La solution a été agitée à température ambiante pendant 2 h. L'avancement de la réaction a été suivi par HPLC. Après cette période la réaction était totale. Le solvant a été éliminé sous pression réduite et le résidu a été redissous dans du dichlorométhane (6 mL). Le solvant a été à nouveau éliminé sous pression réduite. Cette procédure a été répétée 5 fois pour éliminer l'acide trifluoroacétique en excès. Le produit brut a été purifié par HPLC préparative pour conduire à une huile qui a été utilisée directement dans l'étape de complexation. MS (ESI) calculée pour C₈₃H₉₇N₇O₁₅P₃ [M+3H⁺], *m*/*z* 1622.

### Composé (46c)

Ce composé a été obtenu en suivant la procédure utilisée pour l'exemple **46a.**

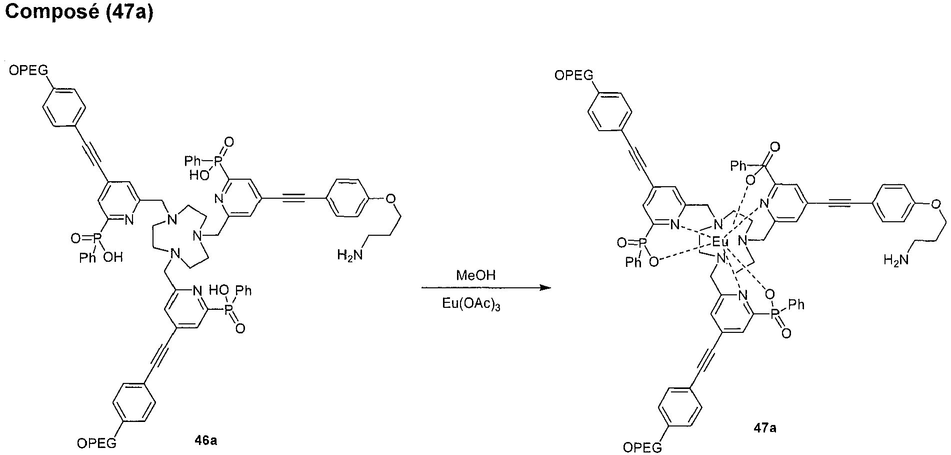

A une solution de composé **46a** (0,016 mmol) dans du méthanol (1 mL) a été ajouté une solution d'acétate d'europium (7 mg, 0,016 mmole) dissous dans un mélange eau : méthanol (0,5 mL, 1:1 v/v). La solution a été agitée à 50 °C pendant 14 h. L'avancement de la réaction a été suivi par HPLC. Après cette période, la réaction était totale. Le mélange réactionnel a été refroidi et le solvant a été éliminé sous pression réduite. Le résidu a été purifié par chromatographie sur colonne de silice (CH₂Cl₂ / CH₃OH, 10% : NH₃ 0 à 1 % par incréments de 0,1 %) pour obtenir le complexe d'europium sous la forme d'un solide blanc **47a** (9,1 mg, 34 %) ; RMN ³¹P (162 MHz, CD₃OD) δ: +17,5. HMRS (ESI) calculée pour C₈₃H₉₀N₇O₁₅P₃Eu [M-CF₃CO₂]⁺, *m*/*z* 1668,4900, trouvée: 1668,4980.

### Composé (47c)

Ce composé a été obtenu en suivant la procédure utilisée pour l'exemple 47a.

### Composés 50a, 50c, 53a et 53c

Ces composés ont été obtenus en suivant la procédure utilisée pour les exemples **47a** et **47c.**

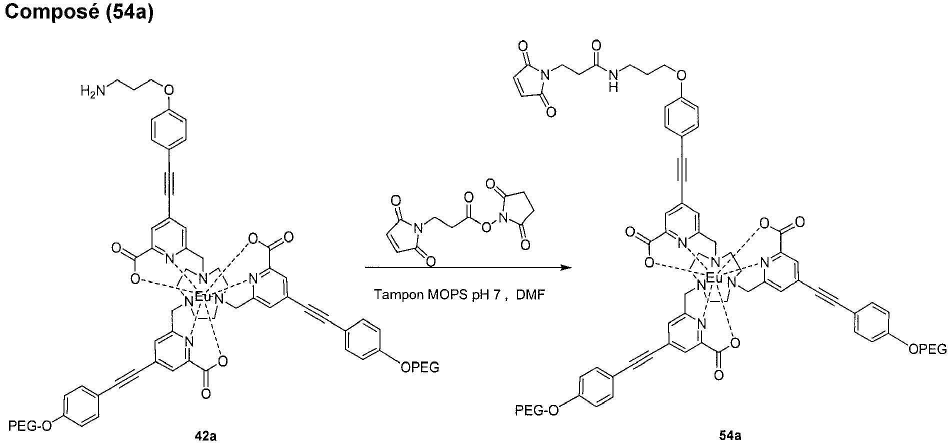

A une solution de complexe d'europium NH₂ **42a** (200 nmol) dans du tampon MOPS pH 7 (200 µL) a été ajoutée une solution d'ester de (N-[β-maléimidopropyloxy] succinimidyle (41 µL, 300 nmol) dans du diméthylformamide préparée à partir d'une solution stock (1,92 mg N-[β-maléimidopropyloxy] succinimidyle dans 1000 µL de diméthylformamide). Le milieu réactionnel a été agité à température ambiante pendant une nuit. L'avancement de la réaction a été suivi par HPLC. Après cette période la réaction était totale. Le milieu réactionnel a été purifié par HPLC préparative pour conduire au composé **54a** sous la forme d'une huile (105 nmol, 52%).

### Composés (55a)-(55c), (56a)-(56c) et (57a)-(57c)

Ces composés ont été obtenus en suivant la procédure utilisée pour l'exemple **54a.**

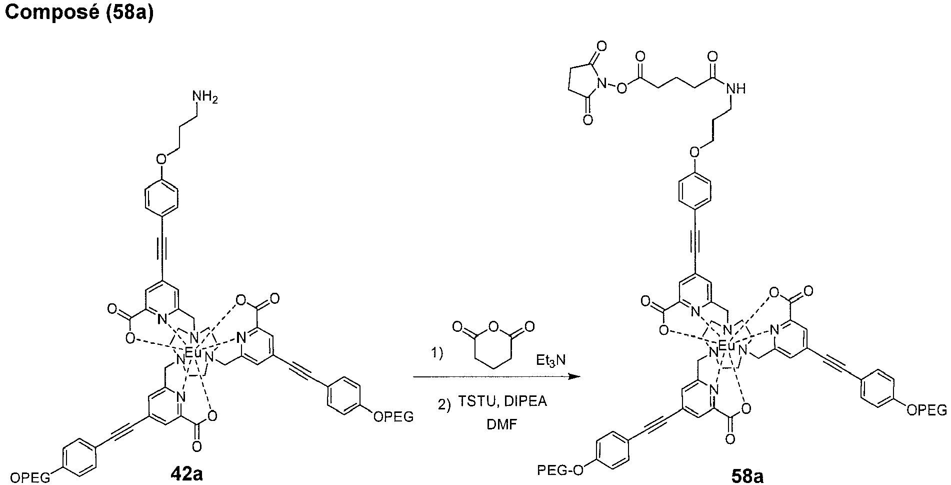

A une solution de complexe d'europium NH₂ **42a** (200 nmol) dans du DMF anhydre (100 µL) a été ajoutée de la triéthylamine (1 µL). A ce mélange a été additionnée une solution d'anhydride glutarique (236 nmol) dans du diméthylformamide (27,4 µL). Le mélange a été agité à température ambiante pendant une nuit. L'avancement de la réaction a été suivi par HPLC. Après cette période, la réaction était totale. Le milieu réactionnel a été purifié par HPLC préparative pour conduire au composé souhaité sous la forme d'une huile (62,7 nmol, 31%). ESI- MS m/z calculée pour C₇₃H₈₀N₇O₁₈Eu+H⁺ = 1382,45. A une solution de complexe acide (550 nmol) dans du diméthylformamide anhydre (200 µL) ont été additionnés de la diisopropyléthylamine (0,8 µL) puis du O-(N-Succinimidyl)-1,1,3,3-tétraméthyluronium tétrafluoroborate (0,3 mg, 1 µmol). Le mélange réactionnel a été agité à température ambiante pendant 15 min. L'avancement de la réaction a été suivi par HPLC. Après cette période, la réaction était totale. Le milieu réactionnel a été purifié directement par HPLC préparative pour conduire au composé NHS **58a** (370 nmol, 74%). ESI- MS m/z calculée pour C₇₃H₈₀N₇O₁₈Eu+H⁺ = 1382,45.

### Composés (59a)-(59c), (60a)-(60c) et (61a)-(61c)

Ces composés ont été obtenus en suivant la procédure utilisée pour l'exemple **58a.**

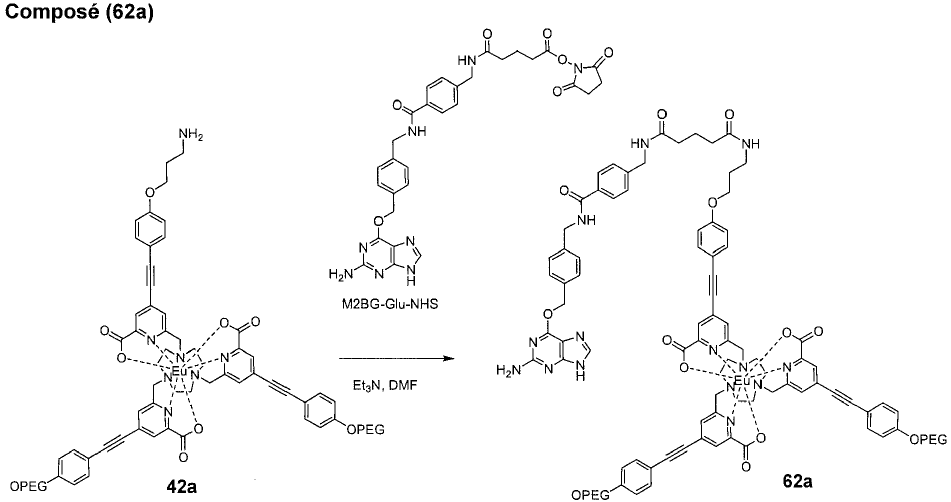

A une solution de complexe d'europium NH₂ **42a** (100 nmol) dans du diméthylformamide (50 µL) ont été ajoutée de la triéthylamine (1,5 µL) et une solution de M2BG-glu-NHS (100 nmol) dans du diméthylformamide (50 µL). La réaction est agitée à température ambiante pendant 2 h. L'avancement de la réaction a été suivi par HPLC. Après cette période un deuxième ajout équivalent au premier a été réalisé (triéthylamine et une solution de M2BG-glu-NHS dans le diméthylformamide). La réaction a été agitée à température ambiante pendant une nuit. Après cette période, la réaction était totale. Le mélange réactionnel a été purifié par HPLC préparative pour conduire au composé **62a** sous forme d'une huile (68 nmoles, 68%). ESI- MS m/z calculée pour C₆₈H₇₄N₇O₁₅Eu+H⁺ = 1382,45.

### Composés (63a), (63c), (63d), (64a), (64c), (64d), (65a), (65c) et (65d)

Ces composés ont été obtenus en suivant la procédure utilisée pour l'exemple **62a.**

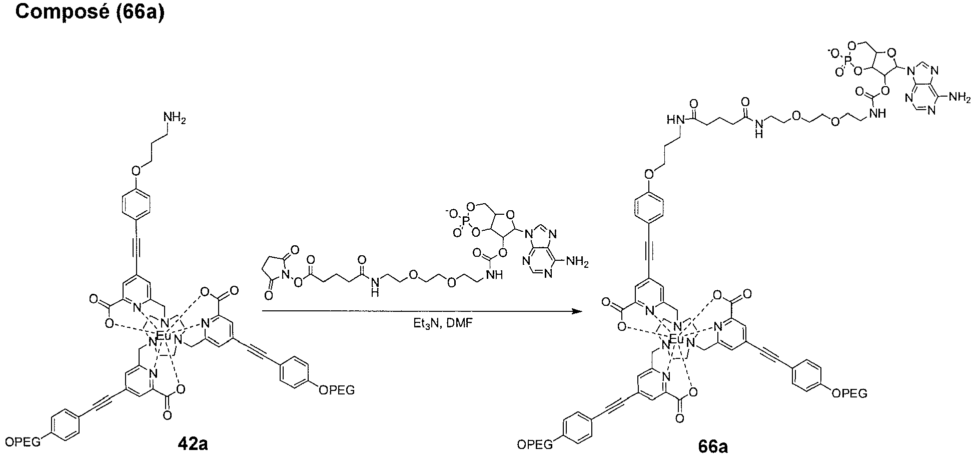

A une solution de complexe d'europium -NH₂ **42a** (200 nmol) dans du diméthylformamide (100 µL) a été ajouté de la triéthylamine (5 µL). Une solution stock d'AMPc-NHS a été préparée contenant AMPc-NHS (200 nmol) dans du diméthylformamide (200 µL). Cette solution stock a été ajoutée à la solution du complexe. Le milieu réactionnel a été agité pendant une nuit. L'avancement de la réaction a été suivi par HPLC. Le milieu réactionnel a été purifié par HPLC préparative pour conduire au composé **66a** sous la forme d'une huile (62 nmol, 31%).

### Composés (67c), (67d), (68c), (68d), (69c) et (69d)

Ces composés ont été obtenus en suivant la procédure utilisée pour l'exemple **66a.**

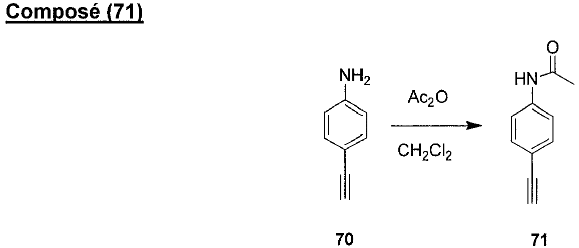

A une solution de 4-éthynylaniline **70** (2,34 g, 20 mmol) dans du dichlorométhane (30 mL) a été ajouté de l'anhydride acétique (2,5 mL, 40 mmol). Le milieu réactionnel a été agité à température ambiante pendant 16 h. L'avancement de la réaction a été suivi par CCM. Après cette période la réaction était totale. Le solvant a été éliminé sous pression réduite. Au résidu couleur noir a été ajoutée une solution saturée de bicarbonate de sodium (15 mL) et le mélange a été extrait avec du dichlorométhane (2 x 15 mL). Les phases organiques ont été rassemblées puis séchées sur sulfate de magnésium. Après filtration, le solvant a été éliminé sous pression réduite. Le produit brut obtenu a été purifié par chromatographie flash sur colonne de silice (acétate d'éthyle / cyclohexane 98/2 jusqu'à 60/40 par incrément de 5%) pour conduire au composé **71** sous forme d'un solide légèrement rosâtre (2,05 g, 64 %). PtF:118,4-125,3°C.

### Composés (72) et (73)

Ces composés ont été obtenus en utilisant la méthode décrite dans la littérature (Inorganic Chemistry-2005-44 (18) 6284).

### Composé (74)

Ce composé a été obtenu en utilisant la méthode décrite dans la littérature (Journal of American Chemical Society 2009-131 (35) 12560).

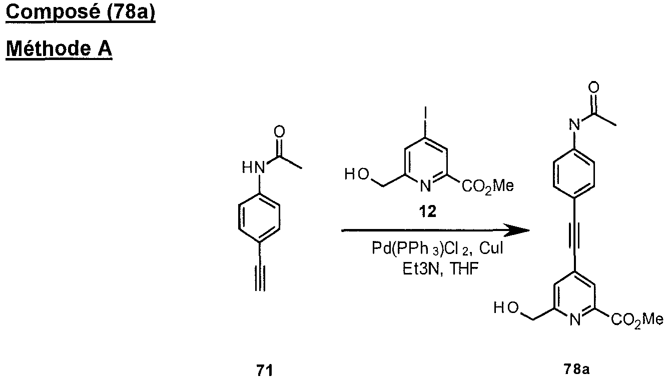

A une solution de dérivé acétylénique **71** (174 mg, 1,1 mmol) dans du tétrahydrofurane anhydre (20 mL) et de la triéthylamine anhydre (10 mL) a été additionnée de la pyridine iodée **12** (296 mg, 1 mmol). Le mélange a été dégazé sous agitation pendant 1 h. A cette solution ont été additionnés du bis-chlorure bis-triphénylphosphine de palladium (II) (70 mg, 0,2 mmol) et de l'iodure de cuivre (I) (38 mg, 0,4 mmol). Le milieu réactionnel a été agité à 50 °C pendant 18 h à l'abri de la lumière. L'avancement de la réaction a été suivi par CCM. Après cette période la réaction était totale. Les solvants ont été éliminés sous pression réduite. Au résidu noir a été additionnée de l'eau (10 mL) et le mélange a été extrait avec de l'acétate d'éthyle (3 x 20 mL). Les phases organiques ont été réunies et séchées sur sulfate de magnésium. Après filtration, le solvant a été éliminé sous pression réduite. Le produit brut obtenu a été purifié par chromatographie flash sur colonne de silice (dichlorométhane / méthanol 0,5% jusqu'à 20 % par incrément de 1%) pour conduire au composé **78a** sous forme d'un solide marron (200 mg, 61%).

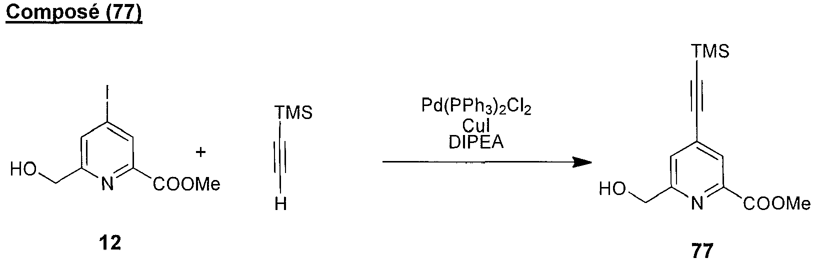

A la pyridine iodée **12** (3.22 g, 11 mmol) a été ajouté du tétrahydrofurane anhydre (10 mL) et la solution a été dégazée par trois cycles de congélation-décongélation. A cette solution ont été ajoutés le triméthylsilyl acétylène (7,8 mL, 55 mmol) et de la diisopropylethylamine (9,6 mL, 50 mmol) et la solution a été à nouveau dégazée. A cette solution ont été ajoutés du tétrakis(triphénylphosphine)palladium(0) (385 mg, 0,55 mmol) et de l'iodure de cuivre (209 mg, 0,11 mmol). Cette nouvelle solution a été à nouveau dégazée trois fois puis a été agitée sous argon. Un changement de couleur du jaune très clair au marron foncé a été observé et le mélange a été agité à 65 °C sous argon. L'avancement de la réaction a été suivi par CCM. Après 3 h la réaction était totale. Le mélange réactionnel a été refroidi à température ambiante et le solvant a été éliminé sous pression réduite. Le produit brut a été purifié par chromatographie sur colonne de silice (dichlorométhane /méthanol 0 à 4 % par incréments de 0,5 %) pour obtenir le composé **77** (2.61 g, 90 %) ; RMN ¹H (400,13 MHz, CDCl₃) δ : 8,01 (s, 1 H), 7,56 (s, 1 H), 4,82 (s, 2H), 3.97 (s, 3H), 0,25 (s, 9H); RMN ¹³C (100 MHz, CDCl₃) δ: 165,2 ; 160,8; 147,2; 133,4; 126,2; 126,0; 101,9; 101,3; 64,6; 53,1 ;- 0,3. HMRS (ESI) calculée pour C₁₃H₁₈O₃NSi [M+H⁺], *m*/*z* 264,1056 trouvée: 264,1050.

### Composé (77a)

Ce composé a été obtenu en utilisant la méthode décrite dans la littérature sur le 4-nitrophénol (Bioorganic & Medicinal Chemistry Letters 2007-17-5428).

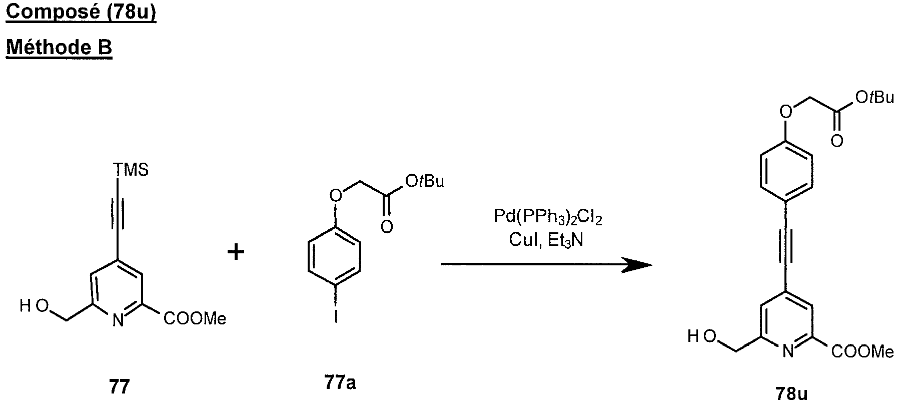

A une solution de dérivé acétylénique **77** (263 mg, 1 mmol) dans du tétrahydrofurane anhydre (10 mL) et de la triéthylamine anhydre (5 mL) a été additionné du dérivé iodé **77a** (370 mg, 1 mmol). Le mélange a été dégazé sous agitation pendant 1 h. A cette solution ont été additionnés une solution 1 M de fluorure de tétrabutylammonium dans du tétrahydrofurane (1,5 mL, 1,5 mmol), du bis-chlorure bis-triphénylphosphine de palladium (II) (72 mg, 0,1 mmol) et de l'iodure de cuivre (I) (40 mg, 0,2 mmol). Le milieu réactionnel a été agité à 75 °C pendant 3 h à l'abri de la lumière. L'avancement de la réaction a été suivi par CCM. Après cette période la réaction était totale. Les solvants ont été éliminés sous pression réduite. Au résidu noir a été additionnée de l'eau (10 mL) et le mélange a été extrait avec du dichlorométhane (3 x 20 mL). Les phases organiques ont été réunies et séchées sur sulfate de magnésium. Après filtration, le solvant a été éliminé sous pression réduite. Le produit brut obtenu a été purifié par chromatographie flash sur colonne de silice (dichlorométhane / méthanol 0,5% jusqu'à 5 % par incrément de 1%) pour conduire au composé **78u** sous forme d'une huile jaunâtre (203 mg, 54%).

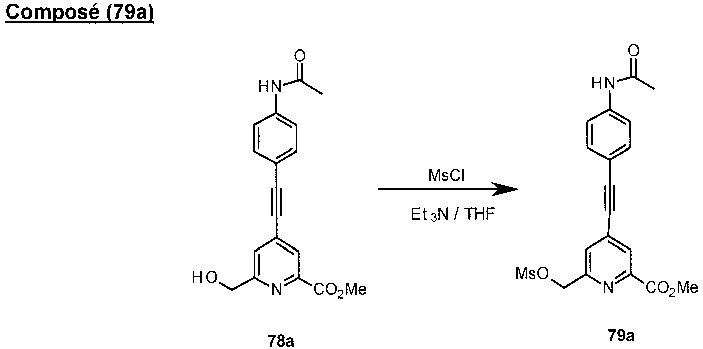

A une solution d'alcool **78a** (195 mg 0,6 mmol) dans du tétrahydrofurane anhydre (14 mL) refroidie à 5°C ont été ajoutés de la triéthylamine (0,3 mL, 1,8 mmol) puis du chlorure de mésyle (70 µL, 1,8 mmol) sous atmosphère inerte. Le milieu réactionnel a été agité à température ambiante pendant 15 min. L'avancement de la réaction a été suivi par CCM. Après cette période, la réaction était totale. Le solvant a été éliminé sous pression réduite. Au résidu a été additionnée de l'eau saturée en chlorure de sodium (30 mL) et le mélange a été extrait avec du dichlorométhane (2 x 30 mL). Les phases organiques ont été regroupées et séchées sur sulfate de magnésium. Après filtration, le solvant a été éliminé sous pression réduite pour conduire à un solide marron correspondant au composé **79a** (231 mg, 95%) qui était suffisamment pur pour être utilisé dans l'étape suivante sans purification complémentaire.

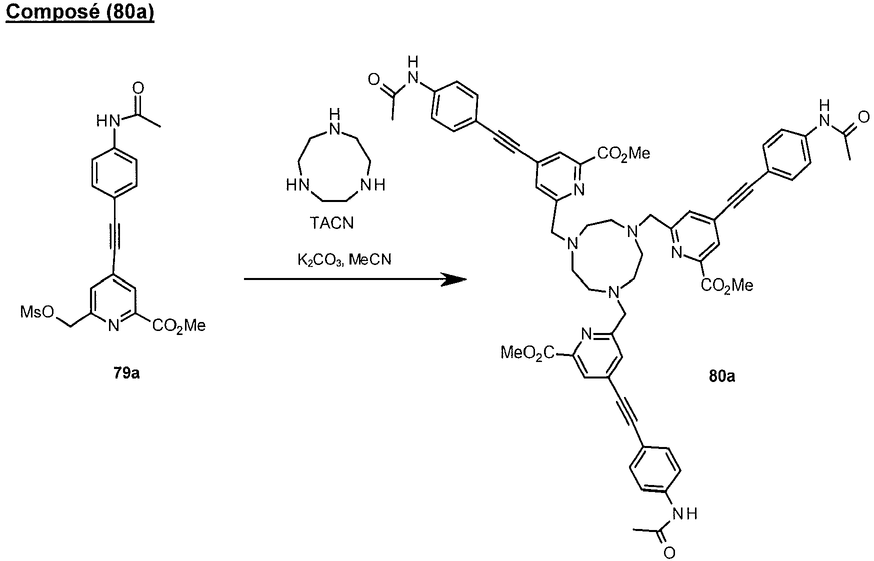

A une solution de dérivé mésylé **79a** (120 mg, 0,3 mmol) dans de l'acétonitrile anhydre (4 mL), ont été ajoutés sous atmosphère inerte du carbonate de potassium (82 mg, 0,6 mmol) et du 1,4,7-triazacyclononane (12,9 mg, 0,1 mmol). Le milieu réactionnel a été chauffé à reflux pendant 24 h. L'avancement de la réaction a été suivi par CCM. Après cette période, la réaction était totale. Le milieu réactionnel a été refroidi à température ambiante et le solvant a été éliminé sous pression réduite. Au résidu a été additionnée de l'eau (10 mL) et le mélange a été extrait avec de l'acétate d'éthyle (2 x 20 mL). Les phases organiques ont été regroupées et séchées sur sulfate de magnésium. Après filtration, le solvant a été éliminé sous pression réduite. Le produit brut obtenu a été purifié par HPLC préparative pour conduire au composé **80a** sous forme d'un solide blanc (12 mg, 12%).

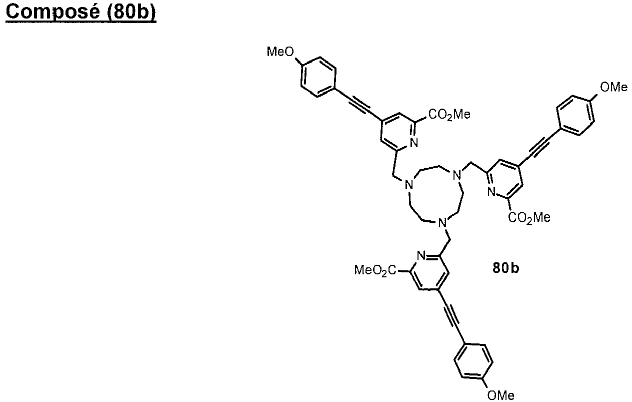

Ce composé a été obtenu suivant la procédure utilisée pour l'exemple **80a**.

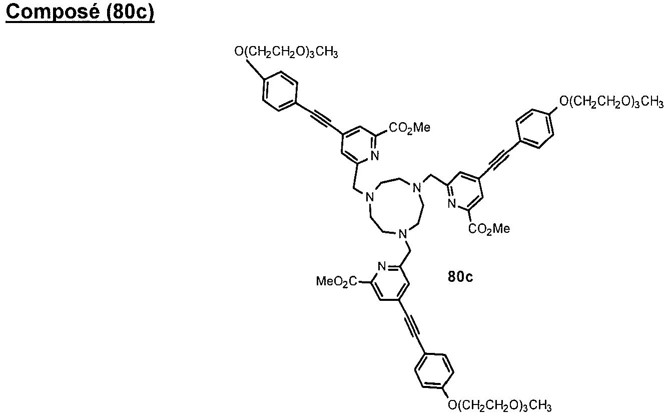

Ce composé a été obtenu suivant la procédure utilisée pour l'exemple **80a**.

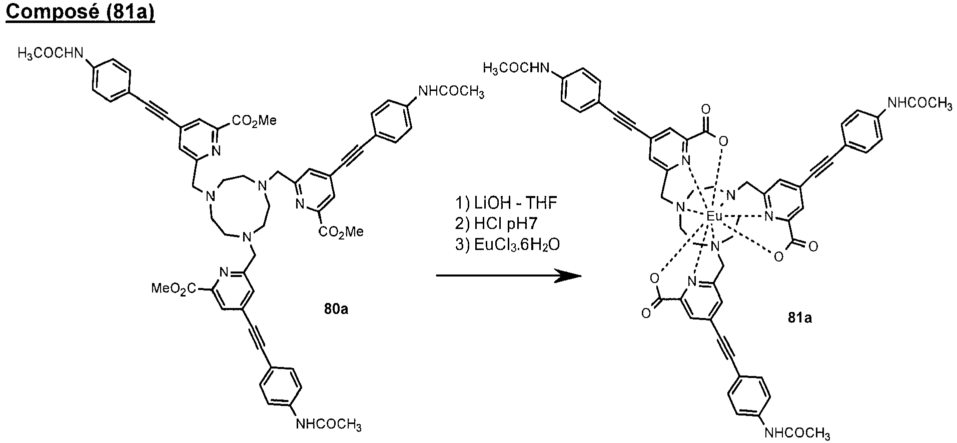

Ce composé a été obtenu suivant la procédure utilisée pour l'exemple **81b** ci-après.

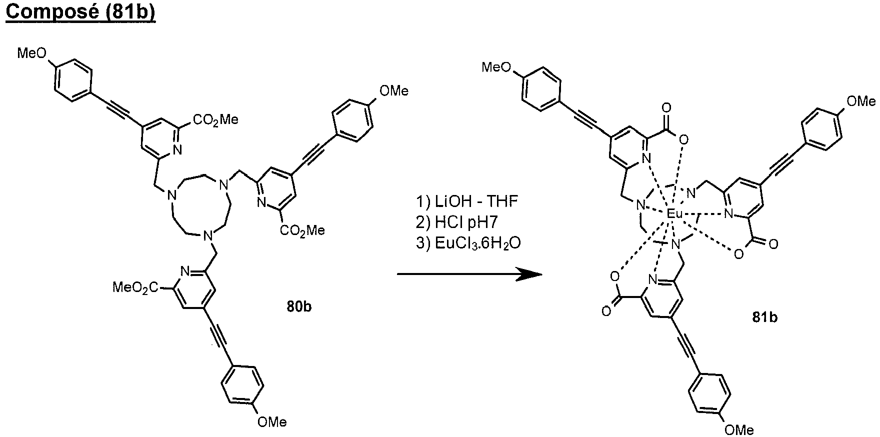

A une solution du ligand triester **80b** (33 mg, 34 µmol) dans du tétrahydrofurane (3 mL) a été additionnée une solution aqueuse d'hydroxyde de lithium (1 mL, 1 M). La solution a été agitée à température ambiante pendant 30 min. L'avancement de la réaction a été suivi par LC-MS. Après cette période la réaction était totale. Le pH de la solution a été ajusté à 7 par addition d'acide chlorhydrique (1 M). A cette solution a été additionné du chlorure d'europium hexahydraté (37 mg, 102 µmol). Le mélange réactionnel a été agité à température ambiante pendant 30 min. Le solvant a été éliminé sous pression réduite et le produit brut a été dissous dans de l'acétonitrile (4 mL) puis purifié par HPLC préparative pour donner le composé désiré **81b** sous la forme d'un solide blanc (23 mg, 63%).

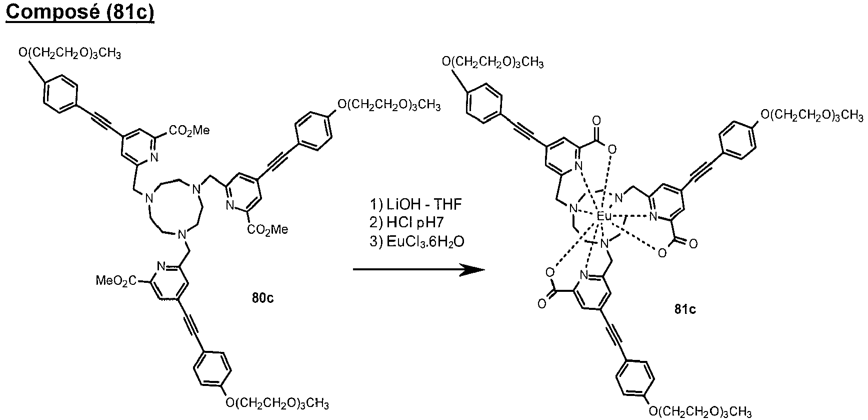

Ce composé a été obtenu suivant la procédure utilisée pour l'exemple **81b.**

### Exemple 1 : Stabilité d'intensité de luminescence des composés de l'invention

La stabilité de la luminescence de différents composés selon l'invention **(37a, 47a, 53a, 62a, 66a, 67a, 69a, 81a, 81c**), ainsi que celle d'un composé de l'art antérieur (composé 39 décrit par Latva et al dans Journal of Luminescence, Volume 75, n° 2, Septembre 1997, Pages 149-169, de formule ci-dessous) ont été mesurées dans différent milieux.

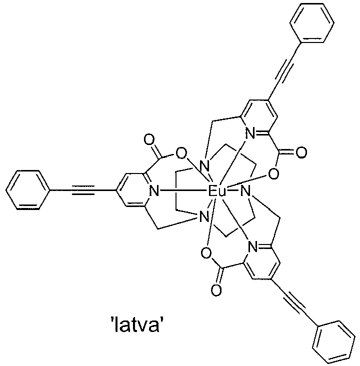

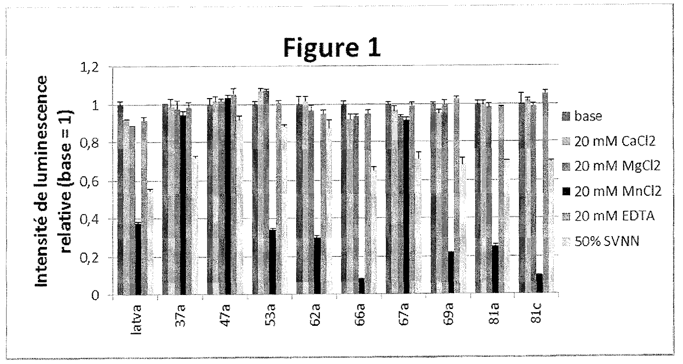

Des solutions de chacun de ces composés à la concentration de 100 à 500 pM ont été préparées dans un tampon HEPES à 50 mM à pH 7,4 avec 0,1% d'albumine sérique bovine (BSA).
50µl de chaque solution ont été déposés dans des puits de plaques 96 puits (Corning Costar 3915), auxquels ont été ajoutés 45 µl de tampon HEPES ou 50 µl de SVNN (« serum de veau de nouveau-né » (fournisseur : Bayer)). Les puits ont été finalement complétés par soit 5µl d'eau (pour obtenir un signal basal, « base » dans la figure 1), soit 5µl d'une solution à 400 mM de CaCl₂, MgCl₂, MnCl₂ ou d'EDTA. Ces solutions sont connues pour leur effet sur les complexes de terres rares : les ions Ca++ et Mg++ peuvent rentrer en compétition avec l'europium pour la liaison avec l'agent complexant et l'EDTA peut avoir pour effet de chélater l'Europium. L'ion Mn++ est également connu pour sa capacité à provoquer une extinction de luminescence.
Après 10 min, la luminescence a été mesurée sur un lecteur Rubystar à 620 nm avec un délai de mesure de 50 µs et un temps d'intégration de 400 µs. Les signaux mesurés ont été normalisés par le signal de « base » de chaque composé et les résultats obtenus sont présentés dans la Figure 1. Les barres d'erreur représentent l'écart type sur trois puits.
La figure 1 montre que la présence de 20 mM de CaCl₂, MgCl₂, ou d'EDTA n'affecte pas de manière significative la luminescence des composés selon l'invention, ce que l'on peut corréler à la stabilité de ces complexes.
On remarque que les complexes avec des bras « phosphinate » comme 37a, 47a et 67a montrent une excellente stabilité de luminescence en présence de MnCl₂ par rapport aux autres complexes, y compris celui de l'art antérieur (Latva).
Enfin, la luminescence de tous les complexes selon l'invention est significativement plus stable que celle du complexe de l'art antérieur en présence de SVNN, ce qui est particulièrement avantageux pour utiliser ces composés par exemple pour l'analyse d'échantillons sanguins dans le domaine du diagnostic.

### Exemple 2 : Spectres d'excitation des composés de l'invention

Les solutions de composés en tampon HEPES (pH 7,4, 0,1% BSA) de l'exemple précédent ont été utilisées pour obtenir les spectres d'excitation de ces composés. Pour cela, ces solutions ont été placées dans des cuves de quartz dans un spectrophotometre de fluorescence « Cary Eclipse » (Varian). On a vérifié que l'absorption à 337 nm de ces solutions était inférieure à 0,05 unités dans une cuve de 1 cm.
Les spectres d'excitation ont été obtenus dans le mode « phosphorescence » en mesurant l'émission à 615 nm après excitation à différentes longueurs d'onde. Les mesures ont été effectuées avec un délai de 0,1 ms et un temps d'intégration de 10 ms. La fente d'excitation était de 5 nm, la fente d'émission de 2,5 nm et le voltage du photomultiplicateur de 800 V.

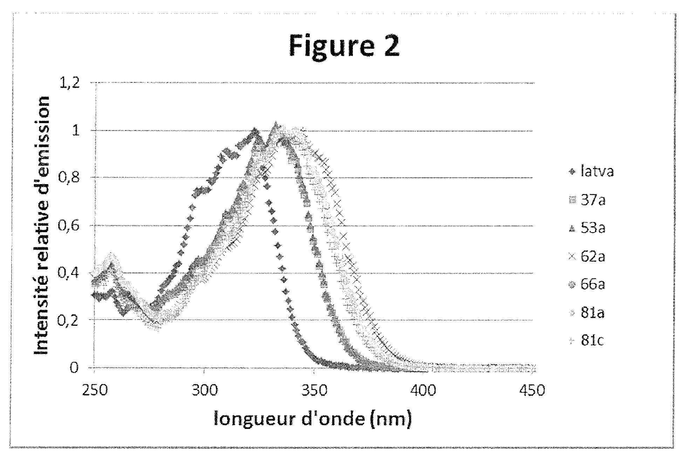

La Figure 2 montre les spectres d'excitation obtenus après normalisation du signal mesuré par le signal maximum pour chaque composé. Les spectres d'excitation des composés selon l'invention présentent un déplacement bathochrome par rapport à celui du composé de l'art antérieur (composé Latva), les rendant particulièrement sensibles à une excitation à 337nm, qui correspond à la longueur d'onde de la lumière émise par les lasers à azote, largement utilisés dans les lecteurs de plaque. Ces spectres montrent également que les composés de l'invention peuvent également être excités à des longueurs d'onde plus élevées, par exemple 365 nm ou 380 nm. Il est particulièrement avantageux d'utiliser ces dernières longueurs d'onde pour des applications en microscopie, puisque elles limitent l'absorption de lumière par les matériels biologiques présents dans le milieu de mesure et par la verrerie présente dans les microscopes.

### Exemple 3 : Propriétés photophysiques des composés de l'invention

Plusieurs composés selon l'invention ont été préparés et leurs propriétés photophysiques, en particulier les longueurs d'onde correspondant à leurs pics d'absorption maximale (λmax), ont été déterminées. Les structures de ces composés et les résultats obtenus sont décrits dans le tableau 2.

Les chromophores (similaires au composé **78,** fonction carboxylate protégée sous forme d'ester) ont été synthétisés selon le protocole décrit dans le schéma 13 en utilisant une des deux approches « A » ou « B » telles que décrites dans ce schéma (y compris pour les chromophores comportant des cycles ou hétérocycles naphtyle, thiolanyle, imidazolyle et anthracènyle). Pour chaque chromophore, les spectres UV ont été enregistrés et les longueurs d'ondes maximales (λₘₐₓ) d'absorbtion ont été déterminées et reportées dans le tableau 2.

Pour certains chromophores, des complexes d'europium selon l'invention dans lesquels les 3 chromophores sont identiques (complexes symmétriques) ont été préparés en utilisant le protocole décrit dans le schéma 14, avec un macrocycle TACN (les rendements de synthèse des composés intermédiares **79, 80** et **81** du schéma 14 ont été reportés dans le tableau 2). Les propriétés photo-physiques de ces complexes d'europium ont été déterminées et également reportées dans ce tableau

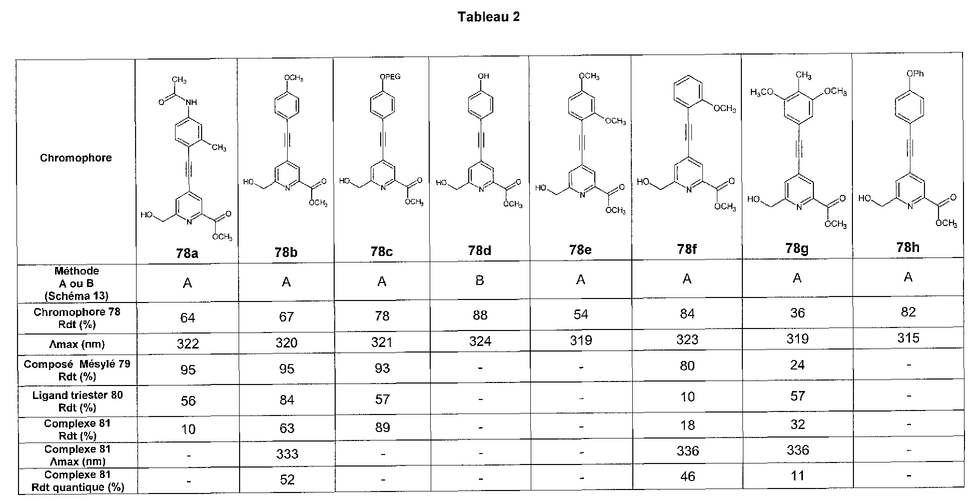

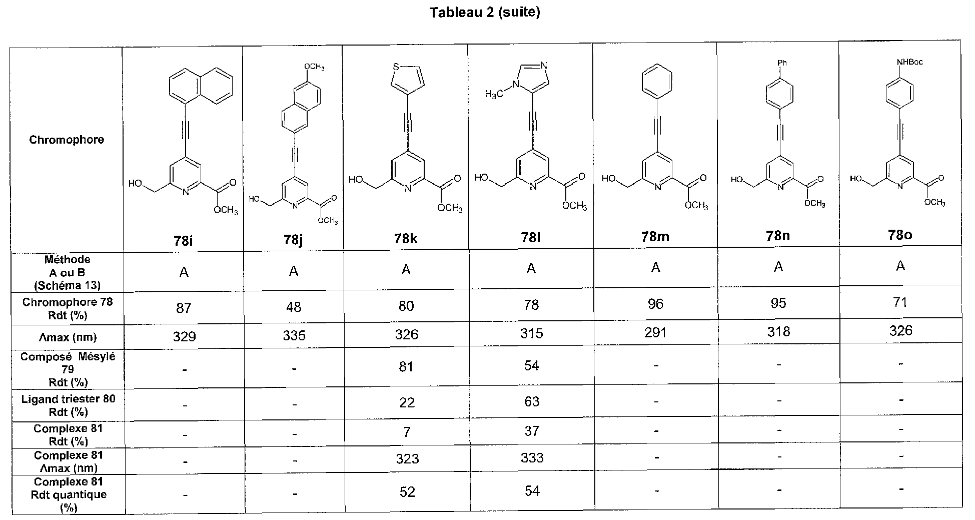

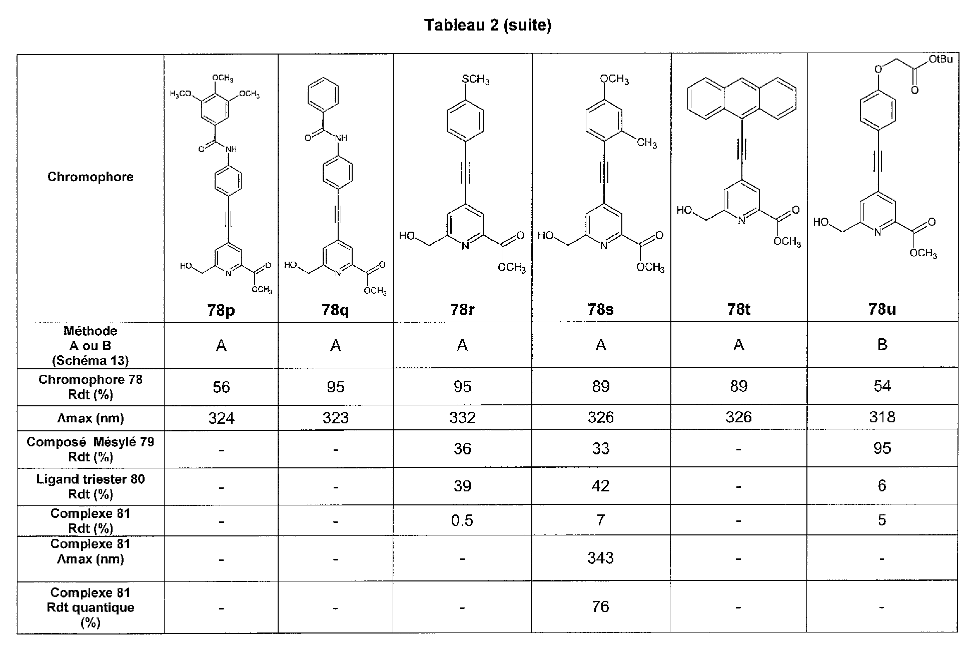

### Résultats :

A l'exception du composé **78m,** correspondant au composé de référence de l'art antérieur (composé **39** de Latva), tous les chromophores testés ont une longueur d'onde maximale d'absorption (λmax) comprise entre 310 et 332 nm . Il a été déterminé que la complexation de l'europium avec un chromophore induit un effet bathochrome de 10 à 20 nm en fonction du type de structure du ligand (voir les λmax des chromophores **78b, 78f, 78g, 78k, 78l,** et les λmax des complexes d'europium correspondant). Ainsi le Tableau 2 montre que tous les complexes d'Europium symmétriques (composés de formule 81) comportant les chromophores décrits dans ce tableau sont très adaptés à une excitation laser à 337 nm. Par ailleurs, les complexes d'europium présentent également un rendement quantique suffisant (>10%) pour que ces composés puissent être utilisés comme marqueurs fluorescents.

### Exemple 4 : propriétés comparées

Les propriétés photophysiques de plusieurs composés selon l'invention ont été comparées et les élements suivants ont été découverts :
- les propriétés photophysiques d'un complexe d'europium symétrique (trois chromophores chrom₁ chrom₂ et chrom₃ identiques) sont les mêmes que celles d'un complexe d'europium dissymétrique (deux chromophores identiques et un chromophore comportant un groupe-L-G permettant le couplage avec une molécule à marquer).
- Les propriétés photophysiques des complexes d'europium phénylphosphinates (R₁=-PO(OH)-C₆H₅), méthylphosphinates (R₁= -PO(OH)CH₃)) et carboxylates (R₁= -COO⁻) sont similaires.
- Les complexes d'europium hybrides dont les chromophores comportent des groupes R₁ différents, par exemple dans lesquels un des trois groupes R₁ est un groupe carboxylate et les deux autres groupes R₁ sont des groupes phénylphosphinates ou méthylphosphinates, possèdent des caractéristiques photophysiques identiques à celles des complexes dont les trois groupes R₁ sont identiques et sont des groupes carboxylates.

Ainsi, les résultats obtenus dans l'exemple 3, à savoir le fait que ces composés sont particulièrement adaptés à une excitation par laser à 337 nm, sont généralisables aux autres composés selon l'invention, c'est-à-dire aux complexes d'europium dissymétriques et comportant divers groupes R₁ (carboxylates ou dérivés de phénylphosphinates).

## Revendications

1. Agent complexant de formule (I) : 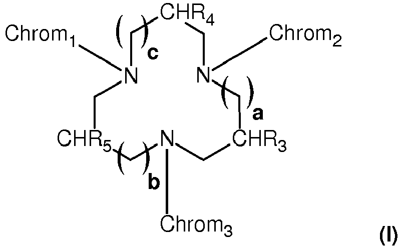 dans laquelle :
- soit a=b=c=1, soit a=b=c=0, soit a=1 et b=c=0, soit a=b=1 et c=0 ;
- R₃, R₄ et R₅ sont chacun choisis parmi les atomes ou groupes suivants : H, -L-G
- Chrom₁, Chrom₂ et Chrom₃ sont des chromophores identiques ou différents et répondent tous trois à une seule des formules suivantes : 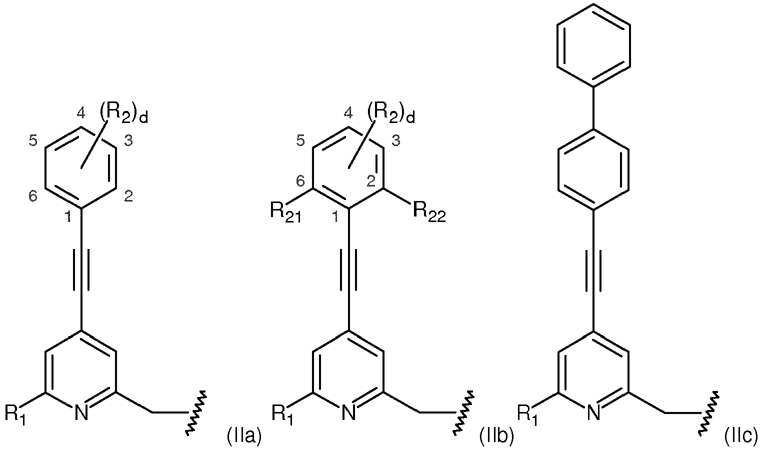 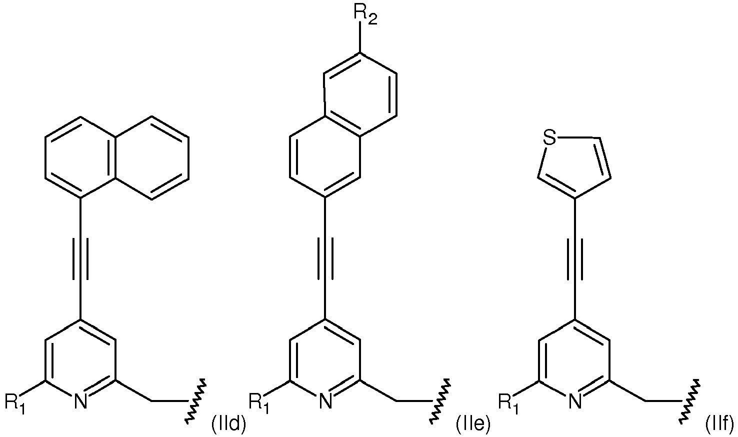 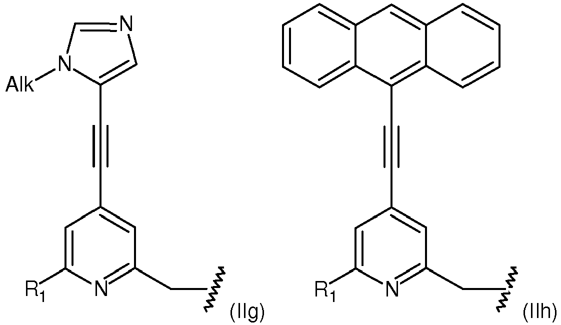 dans lesquelles
- d est un nombre entier allant de 1 à 3
- chaque R₁ est choisi parmi les groupes suivants : -COOH, -PO(OH)R₆, R₆ représentant un groupe choisi parmi : phényle, benzyle, méthyle, éthyle, propyle, n-butyle, sec-butyle, isobutyle, *tert*-butyle;
- R₂₁ et R₂₂ représentent indépendamment l'un de l'autre un atome d'hydrogène ou un un alkyle linéaire ou ramifié comprenant de 1 à 6 atomes de carbone ;
- chaque R₂ est choisi parmi les groupes suivants :
• *groupes alkyles* linéaires ou ramifiés comprenant de 1 à 6 atomes carbones éventuellement substitués par un groupe PEG ;
• *groupes O-donneurs choisis parmi* : OH ; -OPhényle ; -O-CH₂-CO-N-Alk ; -O-CH₂-CO-O-Alk ; -O-CH₂-CO-NH ; -O-CH₂-CO-OH; -O-CH₂-CO-N-LG ; -O-CH₂-CO-O-L-G; -O-L-G ; -O-Alk; -O-PEG; le groupe de formule : 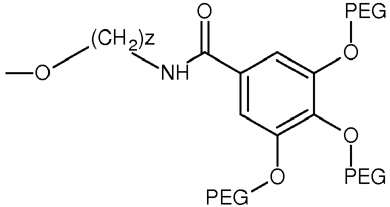 dans laquelle z est un nombre entier allant de 1 à 5 ;
• *groupes S-donneurs choisis parmi :* -S-L-G ; -S-Alk; -S-PEG; le groupe de formule : 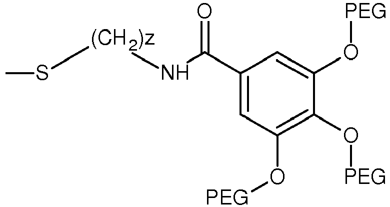 dans laquelle z est un nombre entier compris entre 1 et 5 ;
• *groupes NHCO-donneurs choisis parmi* : -NHCO-L-G ; -NHCO(OAlk); -NHCO(NHAlk); -NHCO(NAlk₁Alk₂); -NHCO(SAlk); -NHCO(Alk); -NHCO-PEG; -NHCO-phényle ; un groupe de formule : 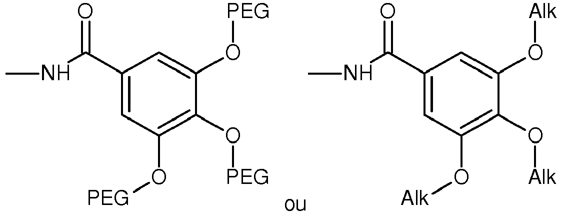
• *groupes SCO-donneurs choisis parmi :* -SCO-L-G; -SCO(OAlk); -SCO(NHAlk); -SCO(NAlk₁Alk₂); -SCO(SAlk); -SCO(Alk) ; -SCO-PEG;
• *groupes NHCS-donneurs choisis parmi* : -NHCS(OAlk); -NHCS(NHAlk); -NHCS(NAlk₁Alk₂); -NHCS(SAlk); -NHCS(Alk); -NHCS-PEG; -NHCS-L-G;
• *groupes SCS-donneurs choisis parmi* : -SCS(OAlk); -SCS(NHAlk); -SCS(NAlk₁Alk₂); -SCS(SAlk); -SCS(Alk) ; -SCS-PEG; -SCS-L-G;
- PEG est un groupe de formule-CH₂-(CH₂OCH₂)y-CH₂OCH₃, y étant un nombre entier allant de 1 à 5;
- Alk, Alk₁ et Alk₂ sont chacun un alkyle linéaire ou ramifié en C₁-C₁₀ éventuellement substitué par un groupe -O-PEG ;
- L est un bras d'espacement choisi parmi les groupes suivants :
1) 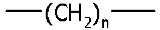
2) 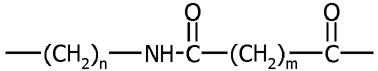
3) 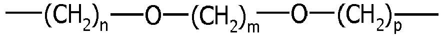
4) 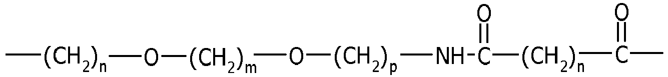
5) 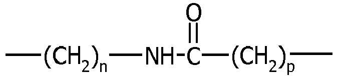
6) 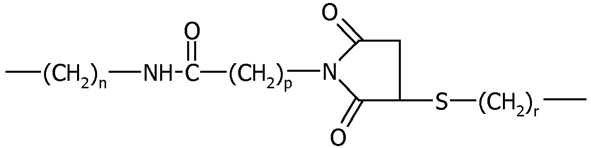
7) 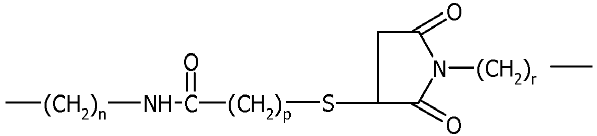
8) 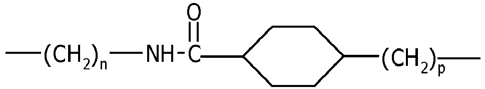
9) 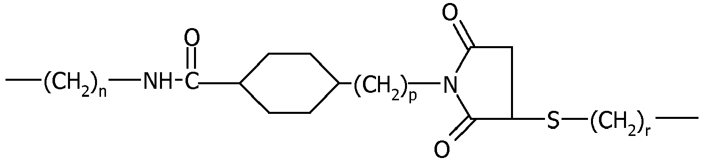
10) 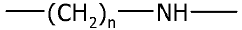
11) 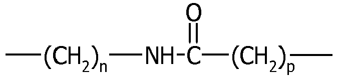
12) 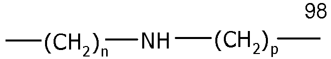
13) 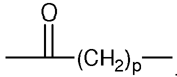
dans lesquels n, m, p, r sont des nombres entiers de 1 à 16;
G est un groupe réactif permettant la liaison du complexe ou de l'agent complexant à une molécule à marquer, ledit groupe réactif étant choisi parmi : un acrylamide, une amine activée, un ester activé, un aldéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, une amine, un halogénure d'acide, un ester d'hydroxysuccinimidyle, un ester d'hydroxysulfosuccinimidyle, un azidonitrophényle, un azidophényle, un 3-(2-pyridyl dithio)-propionamide, un glyoxal, une triazine, un groupe acétylénique, et un groupe de formule : 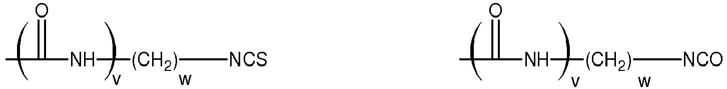 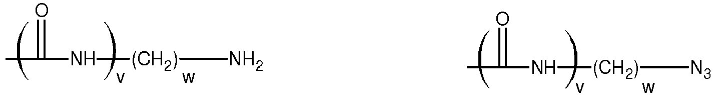 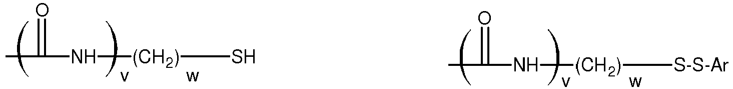 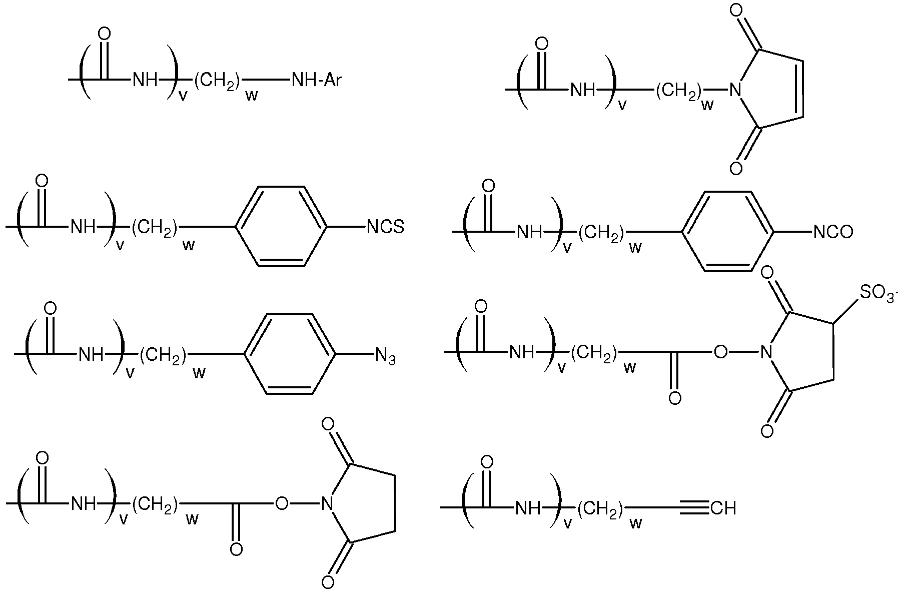 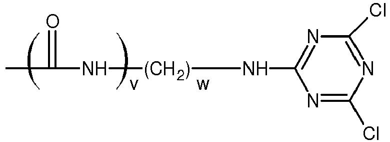 dans lequel w varie de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène;
étant entendu que :
- lorsque R₂₂ et R₂₁ sont des groupes alkyles, d=1 et lorsque l'un des groupes R₂₂ ou R₂₁ est un groupe alkyle et que l'autre est un atome d'hydrogène, d=1 ou d=2 ;
- lorsque R₂ comporte un groupe -L-G, R₃ = R₄ = R₅ = H ;
- lorsque R₂ ne comporte pas de groupe -L-G, soit l'un des groupes R₃, R₄ et R₅ est un groupe
- L-G soit R₃ = R₄ = R₅ = H ; et
- lorsque plusieurs groupes R₂ sont présents, au moins un est en position 4 du cycle benzénique.

2. Agent complexant selon la revendication 1 dans laquelle :
- Chrom₁, Chrom₂ et Chrom₃ sont des chromophores identiques ou différents et répondent tous trois à une seule des formules suivantes :

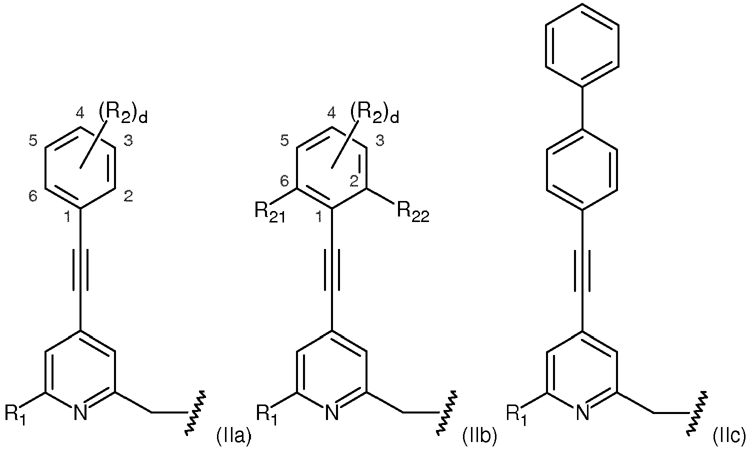

3. Agent complexant selon la revendication 1, de formule (I) : 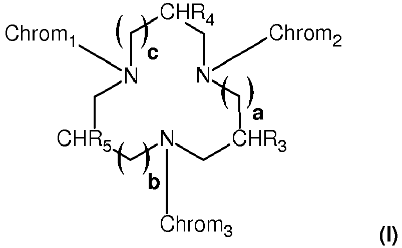 dans laquelle :
- soit a=b=c=1, soit a=b=c=0, soit a=1 et b=c=0, soit a=b=1 et c=0 ;
- R₃, R₄ et R₅ sont chacun choisis parmi les atomes ou groupes suivants : H, -L-G
- Chrom₁, Chrom₂ et Chrom₃ sont des chromophores identiques ou différents de formule (II) : 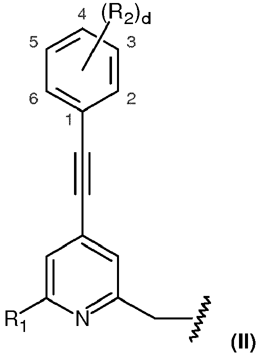 dans laquelle
- d est un nombre entier allant de 1 à 3
- R₁ est choisi parmi les groupes suivants : -COOH, -PO(OH)R₆, R₆ représentant un groupe choisi parmi : phényle, benzyle, méthyle, éthyle, propyle, *n*-butyle, sec-butyle, isobutyle, *tert*-butyle;
- chaque R₂ est choisi parmi les groupes suivants :
• *groupes alkyles* linéaires ou ramifiés comprenant de 1 à 6 atomes carbones éventuellement substitués par un groupe PEG ;
• *groupes O-donneurs choisis parmi* :-O-L-G ; -O-Alk; -O-PEG; le groupe de formule : 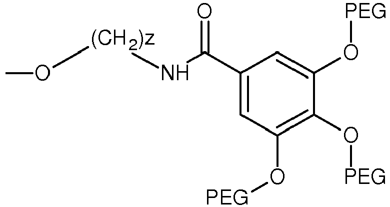 dans laquelle z est un nombre entier allant de 1 à 5 ;
• *groupes S-donneurs choisis parmi :* -S-L-G ; -S-Alk; -S-PEG; le groupe de formule : 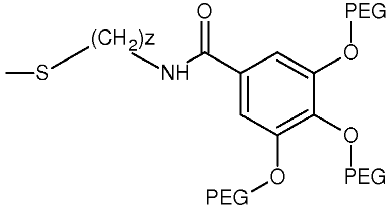 dans laquelle z est un nombre entier compris entre 1 et 5 ;
• *groupes NHCO-donneurs choisis parmi* : -NHCO-L-G ; -NHCO(OAlk); -NHCO(NHAlk); -NHCO(NAlk₁Alk₂); -NHCO(SAlk); -NHCO(Alk); -NHCO-PEG; le groupe de formule : 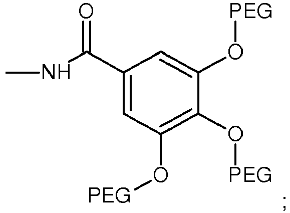
• *groupes SCO-donneurs choisis parmi :* -SCO-L-G; -SCO(OAlk); -SCO(NHAlk); -SCO(NAlk₁Alk₂); -SCO(SAlk); -SCO(Alk) ; -SCO-PEG;
• *groupes NHCS-donneurs choisis parmi* -NHCS(OAlk); -NHCS(NHAlk); -NHCS(NAlk₁Alk₂); -NHCS(SAlk); -NHCS(Alk); -NHCS-PEG; -NHCS-L-G;
• *groupes SCS-donneurs choisis parmi* : -SCS(OAlk); -SCS(NHAlk); -SCS(NAlk₁Alk₂); -SCS(SAlk); -SCS(Alk) ; -SCS-PEG; -SCS-L-G;
- PEG est un groupe de formule-CH₂-(CH₂OCH₂)y-CH₂OCH₃, y étant un nombre entier allant de 1 à 5;
- Alk, Alk₁ et Alk₂ sont chacun un alkyle linéaire ou ramifié en C₁-C₁₀ éventuellement substitué par un groupe -O-PEG ;
- L est un bras d'espacement tel que défini à la revendication 1 ;
- G est un groupe réactif tel que défini à la revendication 1, permettant la liaison du complexe ou de l'agent complexant à une molécule à marquer;
étant entendu que :
- lorsque R₂ comporte un groupe -L-G, R₃ = R₄ = R₅ = H ;
- lorsque R₂ ne comporte pas de groupe -L-G, soit l'un des groupes R₃, R₄ et R₅ est un groupe
- L-G soit R₃ = R₄ = R₅ = H ; et
- lorsque plusieurs groupes R₂ sont présents, au moins un est en position 4 du cycle benzénique.

4. Agent complexant selon l'une quelconque des revendications précédentes, **caractérisé en ce que**
*soit* deux des groupes chrom₁, chrom₂ et chrom₃ sont identiques et sont chacun substitués par 1 à 3 groupes R₂ ne comportant pas de groupe -L-G et le troisième chromophore est substitué par un groupe R₂ comportant un groupe -L-G, les groupes R₃-R₅ sont des atomes d'hydrogène ;
*soit* chrom₁, chrom₂ et chrom₃ sont identiques et sont chacun substitués par 1 à 3 groupes R₂ ne comportant pas de groupe -L-G, et les groupes R₃-R₅ sont des atomes d'hydrogène ;
*soit* chrom₁, chrom₂ et chrom₃ sont identiques et sont substitués par 1 à 3 groupes R₂ ne comportant pas de groupe -L-G, et un des groupes R₃-R₅ est un groupe L-G, les autres étant des atomes d'hydrogène.

5. Agent complexant selon l'une quelconque des revendications précédentes, **caractérisé en ce que** l'un des groupes R₂ comporte un groupe -L-G.

6. Agent complexant selon l'une quelconque des revendications précédentes, **caractérisé en ce que** l'un des groupes R₃, R₄ ou R₅ comporte un groupe -L-G.

7. Agent complexant selon l'une quelconque des revendications précédentes, **caractérisé en ce qu'**il ne comporte aucun groupe -L-G.

8. Agent complexant selon l'une quelconque des revendications précédentes, **caractérisé en ce que** a=b=c=0 (1,4,7 triazacyclononane).

9. Agent complexant selon l'une quelconque des revendications précédentes, **caractérisé en ce que** les groupes R₁ des chromophores chrom₁, chrom₂ et chrom₃ sont des groupes -COOH.

10. Agent complexant selon l'une quelconque des revendications précédentes, **caractérisé en ce que** les groupes R₁ des chromophores chrom₁, chrom₂ et chrom₃ sont des groupes -PO(OH)R₆.

11. Agent complexant selon l'une quelconque des revendications précédentes, **caractérisé en ce que** le groupe R₁ du chromophore chrom₁ est un groupe -COOH et les groupes R₁ des chromophores chrom₂ et chrom₃ sont des groupes -PO(OH)R₆.

12. Agent complexant selon l'une quelconque des revendications précédentes, **caractérisé en ce que** le groupe R₁ du chromophore chrom₁ est un groupe -PO(OH)R₆ et les groupes R₁ des chromophores chrom₂ et chrom₃ sont des groupes -COOH.

13. Agent complexant selon l'une quelconque des revendications précédentes, **caractérisé en ce que** les groupes R₂ sont des groupes O-donneurs choisis parmi : -O-L-G ; -O-Alk; -O-PEG; le groupe de formule :

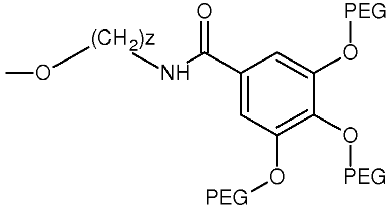

14. Agent complexant selon l'une quelconque des revendications précédentes, **caractérisé en ce que** les groupes R₂ sont des groupes NHCO-donneurs choisis parmi : -NHCO-L-G ; -NHCO(OAlk); -NHCO(NHAlk); -NHCO(NAlk₁Alk₂); -NHCO(SAlk); -NHCO(Alk); -NHCO-PEG; le groupe de formule :

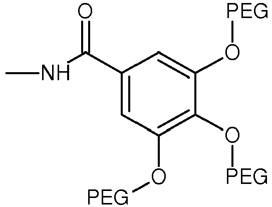

15. Agent complexant selon l'une quelconque des revendications précédentes, **caractérisé en ce qu'**il comporte au moins un groupe PEG

16. Agent complexant selon l'une quelconque des revendications précédentes, **caractérisé en ce que** le groupe -L-G, lorsqu'il est présent, est constitué d'un groupement réactif G choisi parmi : un acide carboxylique, une amine, un ester de succinimidyle, un haloacétamide, une hydrazine, un isothiocyanate, un groupe maléimide, une amine aliphatique, et d'un bras d'espacement L constitué d'une chaine alkylène comprenant de 1 à 5 atomes de carbone.

17. Complexe de lanthanide comprenant un agent complexant selon l'une quelconque des revendications précédentes et un lanthanide.

18. Complexe de lanthanide selon la revendication 17, **caractérisé en ce que** le lanthanide est choisi parmi : Eu³⁺, Tb³⁺, Gd³⁺, Dy³⁺, Nd³⁺, Er³⁺, de préférence le lanthanide est Eu³⁺.

19. Conjugué fluorescent d'une molécule à marquer obtenu par la mise en contact d'un complexe de lanthanide selon l'une des revendications 17 à 18, ledit complexe comprenant un groupe -L-G, avec une molécule d'intérêt.

20. Méthode de synthèse de macrocycles triazotés monoprotégés **caractérisée en ce qu'**elle comprend donc les étapes suivantes :
(i) protection de deux des trois amines du macrocycle triazoté par un groupe protecteur Gp₁ sensible à l'hydrogénation ;
(ii) purification des composés disubstitués sur colonne choisie parmi : une colonne d'alumine basique ; une colonne de silice ayant subi un traitement préalable avec de la triéthylamine ou de l'ammoniac; une colonne d'alumine neutre, cette dernière étant préférée ;
(iii) protection de la troisième amine dudit macrocycle triazoté par un deuxième groupe protecteur Gp₂ insensible à l'hydrogénation ;
(iv) déprotection des amines protégées par les groupes protecteurs Gp₁ par hydrogénation, de préférence avec le catalyseur de Pearlman ;
(v) purification des produits monoprotégés, par exemple par formation de sels de dihydrochlorure par ajout d'acide chlorhydrique à froid et précipitation dans l'éther diéthylique ;
Gp1 et Gp2 étant choisis parmi les groupes suivants: Carbobenzyloxy (Cbz), *p-*Méthoxybenzylcarbonyle (Moz) *tert*-Butyloxycarbonyle (Boc), 9-Fluorénylméthyloxycarbonyle (Fmoc), Acétyle (Ac), Benzoyle (Bz), Benzyle (Bn), *p*-Méthoxybenzyle (PMB), 3,4-Diméthoxvbenzvle (DMPM), *p*-méthoxyphenyle (PMP), Tosyl (Ts), Nosyl (Ns), trifluoroacetamides, alkoxycarbonyles, allyloxycarbonyle (Aloc), 2-(triméthylsilyl)éthoxycarbonyle, 2,2,2-trichloroéthoxycarbonyle (Troc), 2-(triméthylsilyl)éthoxycarbonyle (Teoc), B-(triméthylsilyl)éthanesulfonyl (SES), benzhydryl, trityl, 9-phénylfluorényl et N-silyl imines.

21. Sel de dihydrochlorure d'un composé de formule : 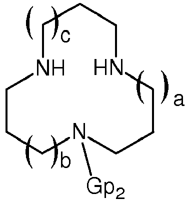 dans laquelle
Gp₂ est un groupement protecteur insensible à l'hydrogénation tel que défini à la revendication 20 ;
a, b et c ont les mêmes valeurs que dans la revendication 1.

## Patentansprüche

1. Komplexbildner mit der Formel (I): 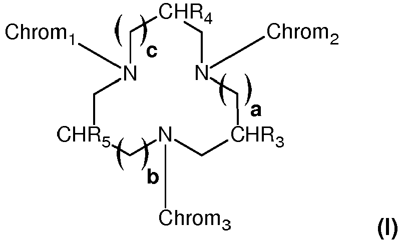 wobei:
- entweder a = b = c = 1, oder a = b = c = 0, oder a = 1 und b = c = 0, oder a = b = 1 und c = 0 gilt,
- R₃, R₄ und R₅ jeweils aus den folgenden Atomen oder Gruppen ausgewählt sind: H, -L-G
- Chrom₁, Chrom₂ und Chrom₃ Chromophore sind, die gleich oder verschieden sind und alle drei einer einzigen der folgenden Formeln entsprechen: 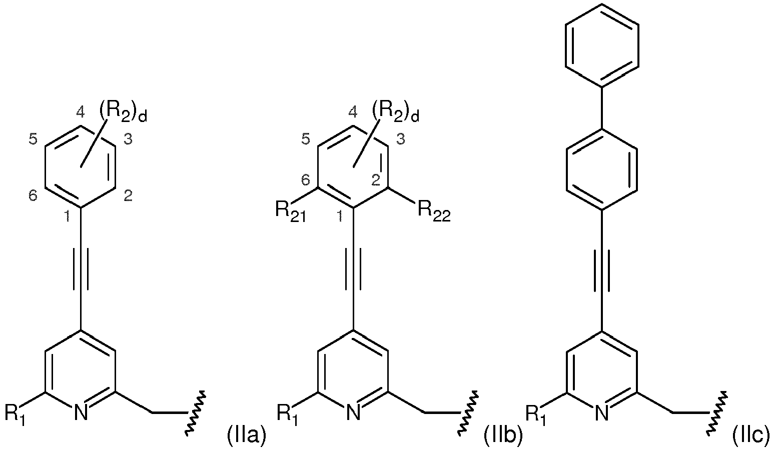 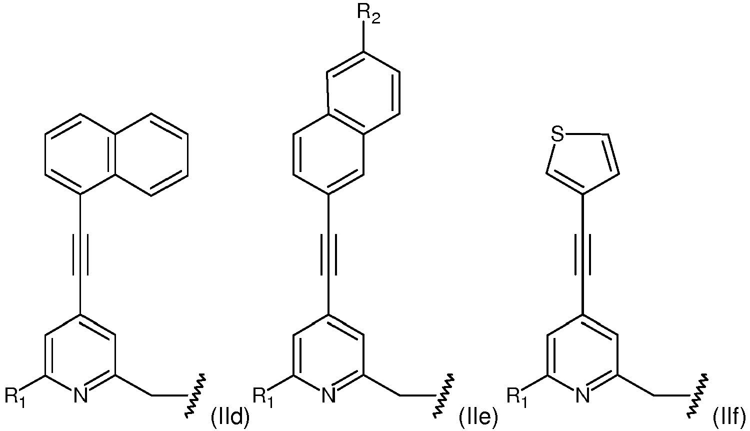 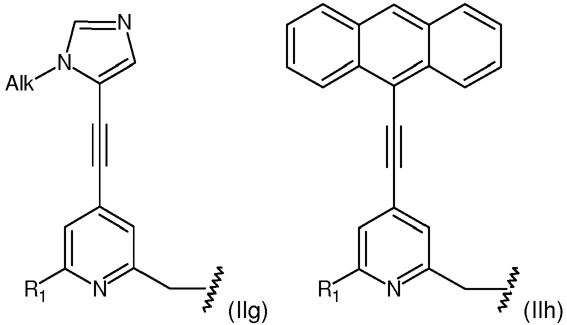
wobei
- d eine ganze Zahl zwischen 1 und 3 ist,
- jeder R₁ aus den folgenden Gruppen ausgewählt ist: -COOH, -PO(OH)R₆, wobei R₆ für eine Gruppe steht, ausgewählt aus: Phenyl, Benzyl, Methyl, Ethyl, Propyl, *n*-Butyl, sec-Butyl, Isobutyl, *tert*-Butyl,
- R₂₁ und R₂₂ unabhängig voneinander für ein Wasserstoffatom oder ein lineares oder verzweigtes Alkyl, das 1 bis 6 Kohlenstoffatome umfasst, stehen,
- jeder R₂ aus den folgenden Gruppen ausgewählt ist:
• linearen oder verzweigten Alkylgruppen, die 1 bis 6 Kohlenstoffatome aufweisen, gegebenenfalls substituiert mit einer PEG-Gruppe,
• O-Donor-Gruppen, ausgewählt aus: OH, -OPhenyl, -O-CH₂-CO-N-Alk, -O-CH₂-CO-O-Alk, -O-CH₂-CO-NH, -O-CH₂-CO-OH, -O-CH₂-CO-N-LG, -O-CH₂-CO-O-L-G, -O-L-G, -O-Alk, -O-PEG, der Gruppe mit der Formel: 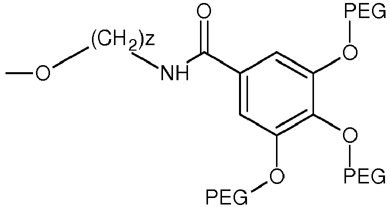 worin z eine ganze Zahl zwischen 1 und 5 ist,
• S-Donor-Gruppen, ausgewählt aus: -S-L-G, -S-Alk, -S-PEG, der Gruppe mit der Formel: 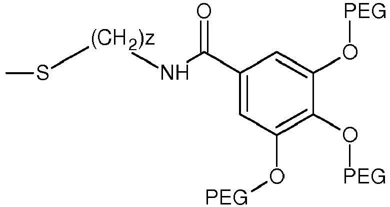 worin z eine ganze Zahl im Bereich zwischen 1 und 5 ist,
• NHCO-Donor-Gruppen, ausgewählt aus: -NHCO-L-G, -NHCO(OAlk), -NHCO(NHAlk), -NHCO(NAlk₁Alk₂), -NHCO(SAlk), -NHCO(Alk), -NHCO-PEG, -NHCO-Phenyl, einer Gruppe mit der Formel: 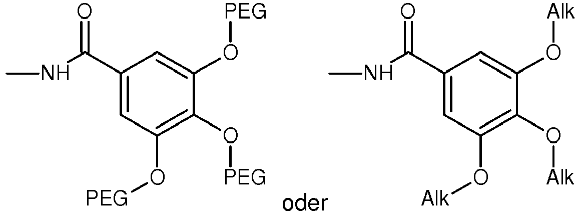
• SCO-Donor-Gruppen, ausgewählt aus: -SCO-L-G, -SCO(OAlk), -SCO(NHAlk), -SCO(NAlk₁Alk₂), -SCO(SAlk), -SCO*(Alk),* -*SCO*-*PEG*,
• NHCS-Donor-Gruppen, ausgewählt aus: -NHCS(OAlk), -NHCS(NHAlk), -NHCS(NAlk₁Alk₂), -NHCS(SAlk), -NHCS(Alk), -NHCS-PEG, -NHCS-L-G,
• SCS-Donor-Gruppen, ausgewählt aus: -SCS(OAlk), -SCS(NHAlk), -SCS(NAlk₁Alk₂), -SCS(SAlk), -SCS(Alk), -SCS-PEG, -SCS-L-G,
- PEG eine Gruppe mit der Formel -CH₂-(CH₂OCH₂)y-CH₂OCH₃ ist, wobei y eine ganze Zahl zwischen 1 und 5 ist,
- Alk, Alk₁ und Alk₂ jeweils ein lineares oder verzweigtes C₁-C₁₀-Alkyl sind, gegebenenfalls substituiert mit einer -O-PEG-Gruppe,
- L ein Spacerarm ist, ausgewählt aus den folgenden Gruppen:
1) 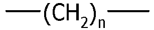
2) 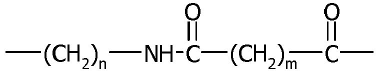
3) 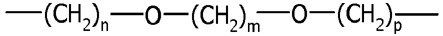
4) 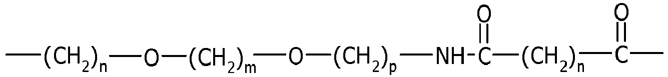
5) 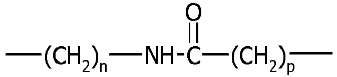
6) 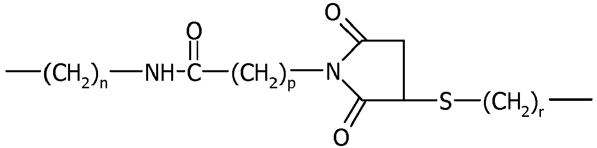
7) 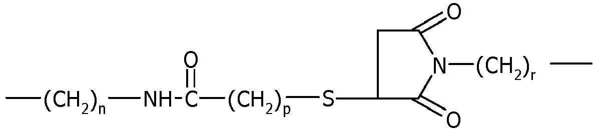
8) 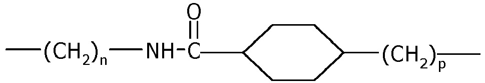
9) 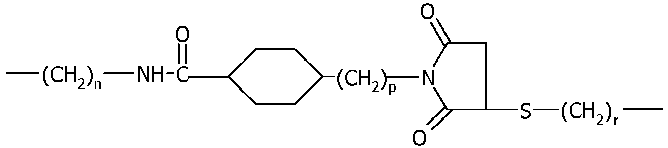
10) 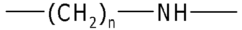
11) 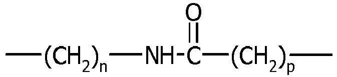
12) 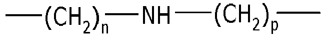
13) 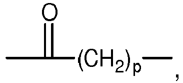
wobei n, m, p, r ganze Zahlen von 1 bis 16 sind,
G eine reaktive Gruppe ist, die die Bindung des Komplexes oder des Komplexbildners an ein zu markierendes Molekül ermöglicht, wobei die reaktive Gruppe ausgewählt ist aus: einem Acrylamid, einem aktivierten Amin, einem aktivierten Ester, einem Aldehyd, einem Alkylhalogenid, einem Anhydrid, einem Anilin, einem Azid, einem Aziridin, einer Carboxylsäure, einem Diazoalkan, einem Haloacetamid, einem Halotriazin, einem Hydrazin, einem Imidoester, einem Isocyanat, einem Isothiocyanat, einem Maleimid, einem Sulfonylhalogenid, einem Thiol, einem Keton, einem Amin, einem Säurehalogenid, einem Hydroxysuccinimidylester, einem Hydroxysulfosuccinimidylester, einem Azidonitrophenyl, einem Azidophenyl, einem 3-(2-Pyridyldithio)-propionamid, einem Glyoxal, einem Triazin, einer acetylenischen Gruppe und einer Gruppe mit der folgenden Formel: 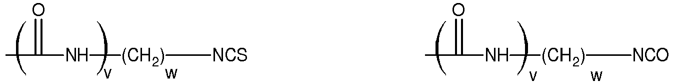 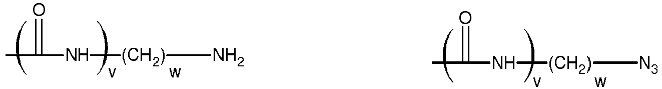 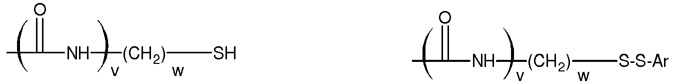 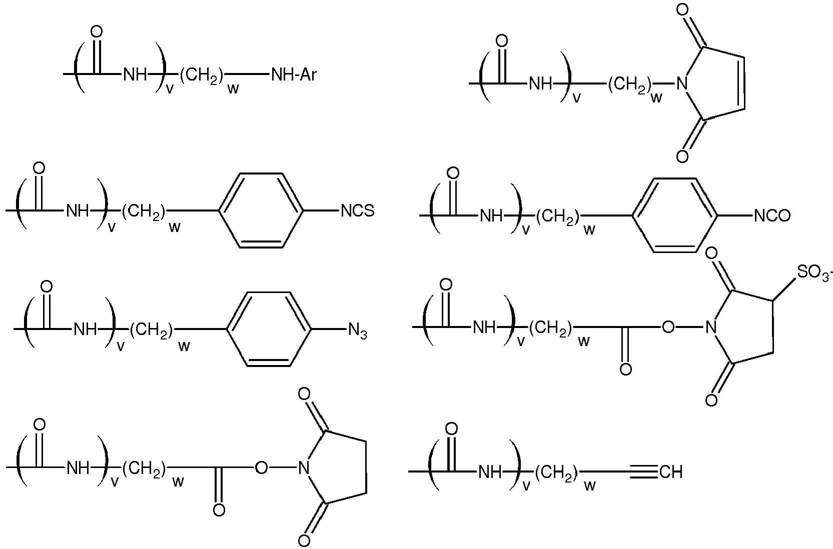 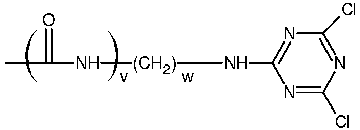 wobei w zwischen 0 und 8 variiert und v gleich 0 oder 1 ist, und Ar ein gesättigten oder ungesättigten Heterozyklus mit 5 oder 6 Gliedern ist, der 1 bis 3 Heteroatome aufweist, gegebenenfalls substituiert mit einem Halogenatom,
mit der Maßgabe, dass:
- wenn R₂₂ und R₂₁ Alkylgruppen sind, d=1 ist und wenn eine der Gruppen R₂₂ oder R₂₁ eine Alkylgruppe ist und wenn die andere ein Wasserstoffatom ist, d=1 oder d=2 ist,
- wenn R₂ eine Gruppe -L-G umfasst, R₃ = R₄ = R₅ = H ist,
- wenn R₂ keine Gruppe -L-G umfasst, entweder eine der Gruppen R₃, R₄ und R₅ eine Gruppe -L-G ist oder R₃ = R₄ = R₅ = H ist, und
- wenn mehrere Gruppen R₂ vorhanden sind, sich mindestens eine an Position 4 des Benzolzyklus befindet.

2. Komplexbildner nach Anspruch 1, wobei:
- Chrom₁, Chrom₂ und Chrom₃ Chromophore sind, die gleich oder verschieden sind und alle drei einer einzigen der folgenden Formeln entsprechen:

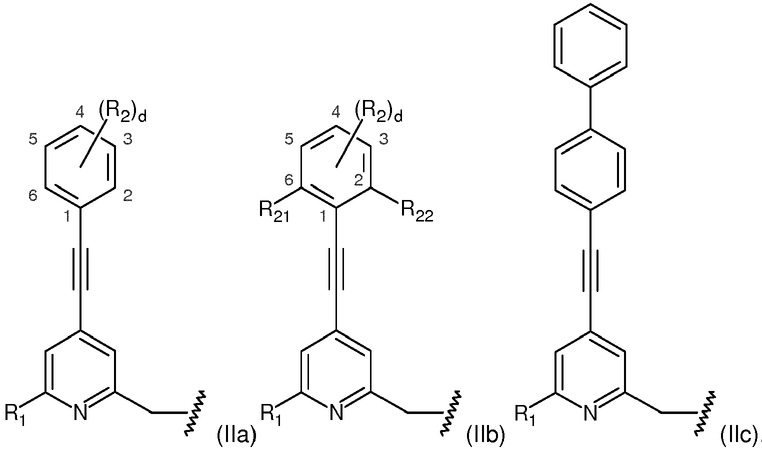

3. Komplexbildner nach Anspruch 1 mit der Formel (I): 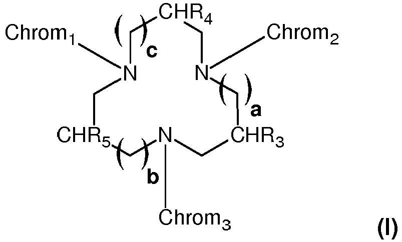 wobei:
- entweder a = b = c = 1, oder a = b = c = 0, oder a = 1 und b = c = 0, oder a = b = 1 und c = 0 gilt,
- R₃, R₄ und R₅ jeweils aus den folgenden Atomen oder Gruppen ausgewählt sind: H, -L-G
- Chrom₁, Chrom₂ und Chrom₃ gleiche oder verschiedene Chromophore mit der Formel (II) sind: 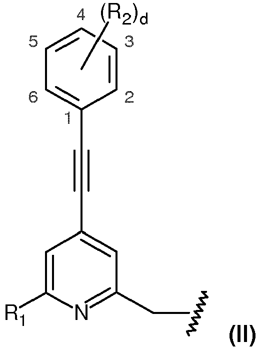 wobei
- d eine ganze Zahl zwischen 1 und 3 ist,
- R₁ aus den folgenden Gruppen ausgewählt ist: -COOH, -PO(OH)R₆, wobei R₆ für eine Gruppe steht, ausgewählt aus: Phenyl, Benzyl, Methyl, Ethyl, Propyl, *n*-Butyl, sec-Butyl, Isobutyl, *tert*-Butyl,
- jeder R₂ aus den folgenden Gruppen ausgewählt ist:
• linearen oder verzweigten Alkylgruppen, die 1 bis 6 Kohlenstoffatome aufweisen, gegebenenfalls substituiert mit einer PEG-Gruppe,
• O-Donor-Gruppen, ausgewählt aus: -O-L-G, -O-Alk, -O-PEG, der Gruppe mit der Formel: 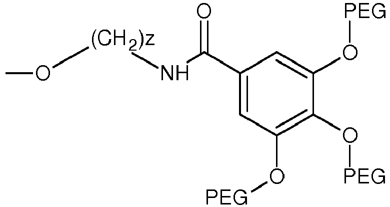 worin z eine ganze Zahl zwischen 1 und 5 ist,
• S-Donor-Gruppen, ausgewählt aus: -S-L-G, -S-Alk, -S-PEG, der Gruppe mit der Formel: 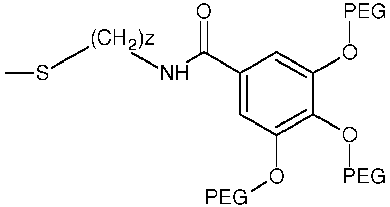 worin z eine ganze Zahl im Bereich zwischen 1 und 5 ist,
• NHCO-Donor-Gruppen, ausgewählt aus: -NHCO-L-G, -NHCO(OAlk), -NHCO(NHAlk), -NHCO(NAlk₁Alk₂), -NHCO(SAlk), -NHCO(Alk), -NHCO-PEG, der Gruppe mit der Formel: 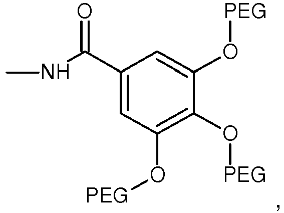
• SCO-Donor-Gruppen, ausgewählt aus: -SCO-L-G, -SCO(OAlk), -SCO(NHAlk), -SCO(NAlk₁Alk₂), -SCO(SAlk), -SCO(Alk), -SCO-PEG,
• NHCS-Donor-Gruppen, ausgewählt aus: -NHCS(OAlk), -NHCS(NHAlk), -NHCS(NAlk₁Alk₂), -NHCS(SAlk), -NHCS(Alk), -NHCS-PEG, -NHCS-L-G,
• SCS-Donor-Gruppen, ausgewählt aus: -SCS(OAlk), -SCS(NHAlk), -SCS(NAlk₁Alk₂), -SCS(SAlk), -SCS(Alk), -SCS-PEG, -SCS-L-G,
- PEG eine Gruppe mit der Formel -CH₂-(CH₂OCH₂)y-CH₂OCH₃ ist, wobei y eine ganze Zahl zwischen 1 und 5 ist,
- Alk, Alk₁ und Alk₂ jeweils ein lineares oder verzweigtes C₁-C₁₀-Alkyl sind, gegebenenfalls substituiert mit einer -O-PEG-Gruppe,
- L ein Spacerarm ist, wie in Anspruch 1 definiert,
- G eine reaktive Gruppe ist, wie in Anspruch 1 definiert, die die Bindung des Komplexes oder des Komplexbildners an ein zu markierendes Molekül ermöglicht, mit der Maßgabe, dass:
- wenn R₂ eine Gruppe -L-G umfasst, R₃ = R₄ = R₅ = H ist,
- wenn R₂ keine Gruppe -L-G umfasst, entweder eine der Gruppen R₃, R₄ und R₅ eine Gruppe -L-G ist oder R₃ = R₄ = R₅ = H ist, und
- wenn mehrere Gruppen R₂ vorhanden sind, sich mindestens eine an Position 4 des Benzolzyklus befindet.

4. Komplexbildner nach einem der vorhergehenden Ansprüche, **dadurch gekennzeichnet, dass**
entweder zwei der Gruppen Chrom₁, Chrom₂ und Chrom₃ gleich sind und jeweils mit 1 bis 3 Gruppen R₂ substituiert sind, die keine Gruppe -L-G umfassen und das dritte Chromophor mit einer Gruppe R₂ substituiert ist, die eine Gruppe -L-G umfasst, wobei die Gruppen R₃-R₅ Wasserstoffatome sind,
oder Chrom₁, Chrom₂ und Chrom₃ gleich sind und jeweils mit 1 bis 3 Gruppen R₂ substituiert sind, die keine Gruppe -L-G umfassen, und die Gruppen R₃-R₅ Wasserstoffatome sind,
oder Chrom₁, Chrom₂ und Chrom₃ gleich sind und mit 1 bis 3 Gruppen R₂ substituiert sind, die keine Gruppe -L-G umfassen, und eine der Gruppen R₃-R₅ eine Gruppe L-G ist, wobei die anderen Wasserstoffatome sind.

5. Komplexbildner nach einem der vorhergehenden Ansprüche, **dadurch gekennzeichnet, dass** eine der Gruppen R₂ eine Gruppe -L-G umfasst.

6. Komplexbildner nach einem der vorhergehenden Ansprüche, **dadurch gekennzeichnet, dass** eine der Gruppen R₃, R₄ oder R₅ eine Gruppe -L-G umfasst.

7. Komplexbildner nach einem der vorhergehenden Ansprüche, **dadurch gekennzeichnet, dass** er keine Gruppe -L-G umfasst.

8. Komplexbildner nach einem der vorhergehenden Ansprüche, **dadurch gekennzeichnet, dass** a=b=c=0 ist (1,4,7-Triazacyclononan).

9. Komplexbildner nach einem der vorhergehenden Ansprüche, **dadurch gekennzeichnet, dass** die Gruppen R₁ der Chromophore Chrom₁, Chrom₂ und Chrom₃ -COOH-Gruppen sind.

10. Komplexbildner nach einem der vorhergehenden Ansprüche, **dadurch gekennzeichnet, dass** die Gruppen R₁ der Chromophore Chrom₁, Chrom₂ und Chrom₃ -PO(OH)R₆-Gruppen sind.

11. Komplexbildner nach einem der vorhergehenden Ansprüche, **dadurch gekennzeichnet, dass** die Gruppe R₁ des Chromophors Chrom₁ eine -COOH-Gruppe ist und die Gruppen R₁ der Chromophore Chrom₂ und Chrom₃ -PO(OH)R₆-Gruppen sind.

12. Komplexbildner nach einem der vorhergehenden Ansprüche, **dadurch gekennzeichnet, dass** die Gruppe R₁ des Chromophors Chrom₁ eine -PO(OH)R₆-Gruppe ist und die Gruppen R₁ der Chromophore Chrom₂ und Chrom₃ -COOH-Gruppen sind.

13. Komplexbildner nach einem der vorhergehenden Ansprüche, **dadurch gekennzeichnet, dass** die Gruppen R₂ O-Donor-Gruppen sind, ausgewählt aus: -O-L-G, -O-Alk, -O-PEG, der Gruppe mit der Formel:

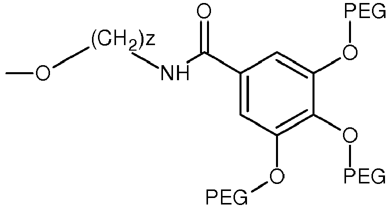

14. Komplexbildner nach einem der vorhergehenden Ansprüche, **dadurch gekennzeichnet, dass** die Gruppen R₂ NHCO-Donor-Gruppen sind, ausgewählt aus: -NHCO-L-G, -NHCO(OAlk), -NHCO(NHAlk), -NHCO(NAlk₁Alk₂), -NHCO(SAlk), -NHCO(Alk), -NHCO-PEG, der Gruppe mit der Formel:

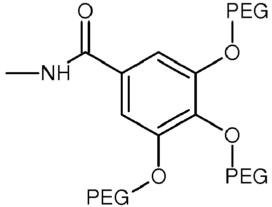

15. Komplexbildner nach einem der vorhergehenden Ansprüche, **dadurch gekennzeichnet, dass** er mindestens eine PEG-Gruppe umfasst.

16. Komplexbildner nach einem der vorhergehenden Ansprüche, **dadurch gekennzeichnet, dass** die Gruppe -L-G, wenn sie vorhanden ist, aus einer reaktiven Gruppe G besteht, ausgewählt aus: einer Carboxylsäure, einem Amin, einem Succinimidylester, einem Haloacetamid, einem Hydrazin, einem Isothiocyanat, einer Maleimidgruppe, einem aliphatischen Amin und aus einem Spacerarm L besteht, der aus einer Alkylenkette besteht, die 1 bis 5 Kohlenstoffatome aufweist.

17. Lanthanidkomplex, der einen Komplexbildner nach einem der vorhergehenden Ansprüche und ein Lanthanid umfasst.

18. Lanthanidkomplex nach Anspruch 17, **dadurch gekennzeichnet, dass** das Lanthanid ausgewählt ist aus: Eu³⁺, Tb³⁺, Gd³⁺, Dy³⁺, Nd³⁺, Er³⁺, wobei das Lanthanid bevorzugt Eu³⁺ ist.

19. Fluoreszierendes Konjugat eines zu markierenden Moleküls, das durch das Inkontaktbringen eines Lanthanidkomplexes nach einem der Ansprüche 17 bis 18, wobei der Komplex eine Gruppe -L-G umfasst, mit einem interessierenden Molekül erhalten wird.

20. Verfahren zur Synthese von einfach geschützten Trinitrogen-Makrozyklen, **dadurch gekennzeichnet, dass** es demnach die folgenden Schritte umfasst:
(i) Schützen von zwei der drei Amine des Trinitrogen-Makrozyklus mit einer Schutzgruppe Gp₁, die empfindlich für die Hydrogenierung ist,
(ii) Reinigen der zweifach substituierten Verbindungen auf eine Säule, ausgewählt aus: einer basischen Aluminiumoxidsäule, einer Siliciumdioxidsäule, die vorher einer Behandlung mit Triethylamin oder Ammoniak unterzogen wurde, einer neutralen Aluminiumoxidsäule, wobei letztere bevorzugt ist,
(iii) Schützen des dritten Amins des Trinitrogen-Makrozyklus mit einer zweiten Schutzgruppe Gp₂, die nicht empfindlich für die Hydrogenierung ist,
(iv) Entschützen der mit den Schutzgruppen Gp₁ geschützten Amine durch Hydrogenierung, bevorzugt mit dem Pearlman-Katalysator,
(v) Reinigen der einfach geschützten Produkte, zum Beispiel durch Bildung von Dihydrochloridsalzen durch Zugeben von kalter Chlorwasserstoffsäure und Ausfällen in Diethylether,
wobei Gp1 und Gp2 aus den folgenden Gruppen ausgewählt sind:
Carbobenzyloxy (Cbz), *p*-Methoxybenzylcarbonyl (Moz), *tert*-Butyloxycarbonyl (Boc), 9-Fluorenylmethyloxycarbonyl (Fmoc), Acetyl (Ac), Benzoyl (Bz), Benzyl (Bn), p-Methoxybenzyl (PMB), 3,4-Dimethoxybenzyl (DMPM), *p*-Methoxyphenyl (PMP), Tosyl (Ts), Nosyl (Ns), Trifluoracetamiden, Alkoxycarbonylen, Allyloxycarbonyl (Aloc), 2-(Trimethylsilyl)ethoxycarbonyl, 2,2,2-Trichlorethoxycarbonyl (Troc), 2-(Trimethylsilyl)ethoxycarbonyl (Teoc), B-(Trimethylsilyl)ethansulfonyl (SES), Benzhydryl, Trityl, 9-Phenylfluorenyl und N-Silyliminen.

21. Dihydrochloridsalz einer Verbindung mit der Formel: 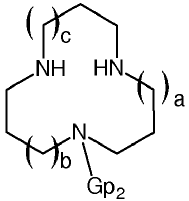 wobei
Gp₂ eine Schutzgruppe ist, die unempfindlich für die Hydrogenierung ist, wie in Anspruch 20 definiert,
a, b und c die gleichen Werte aufweisen wie in Anspruch 1.

## Claims

1. A complexing agent of formula (I): 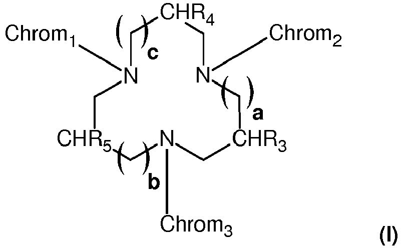 wherein:
- either a=b=c=1, or a=b=c=0, or a=1 and b=c=0, or a=b=1 and c=0;
- R₃, R₄ and R₅ are each selected from the following groups or atoms: H, -L-G;
- Chrom₁, Chrom₂ and Chrom₃ are identical or different chromophores and all three correspond to only one of the following formulae: 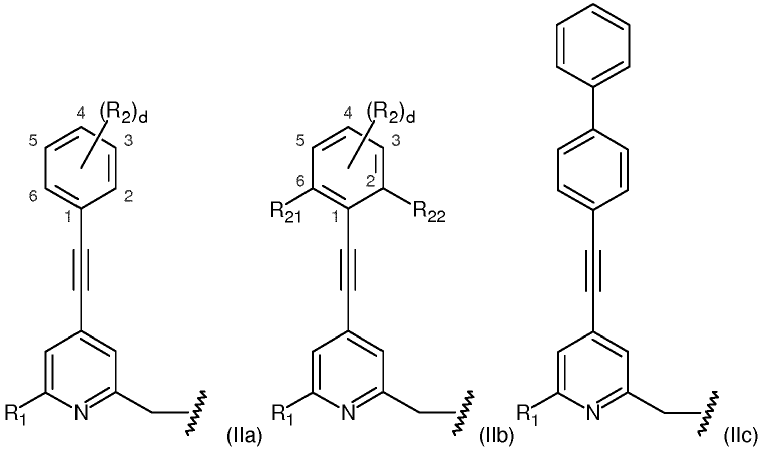 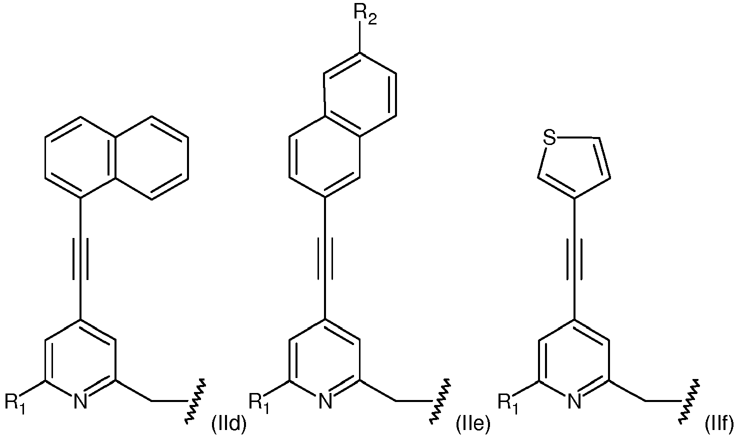 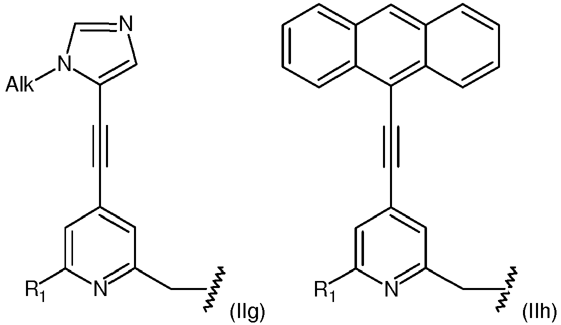 in which
- d is an integer ranging from 1 to 3;
- each R₁ is selected from the following groups: -COOH, -PO(OH)R₆, R₆ representing a group selected from: phenyl, benzyl, methyl, ethyl, propyl, *n*-butyl, *sec*-butyl, isobutyl, *tert*-butyl;
- R₂₁ and R₂₂ represent, independently of each other, a hydrogen atom or a linear or branched alkyl comprising from 1 to 6 carbon atoms;
- each R₂ is selected from the following groups:
• linear or branched alkyl groups comprising from 1 to 6 carbon atoms optionally substituted with a PEG group;
• O-donating groups selected from: OH; -OPhenyl; -O-CH₂-CO-N-Alk; -O-CH₂-CO-O-Alk; -O-CH₂-CO-NH; -O-CH₂-CO-OH; O-CH₂-CO-N-LG; -O-CH₂-CO-O-L-G; -O-L-G; -O-Alk; -O-PEG; the group of formula: 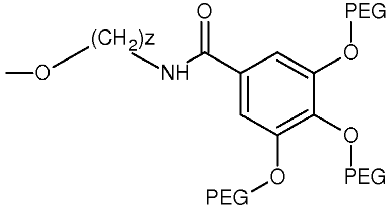 in which z is an integer in the range from 1 to 5;
• S-donating groups selected from: -S-L-G; -S-Alk; -S-PEG; the group of formula: 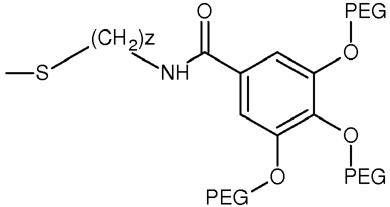 in which z is an integer in the range from 1 to 5;
• NHCO-donating groups selected from: -NHCO-L-G; -NHCO(OAlk); -NHCO(NHAlk); -NHCO(NAlk₁Alk₂); -NHCO(SAlk); -NHCO(Alk); -NHCO-PEG; -NHCO-phenyl; a group of formula: 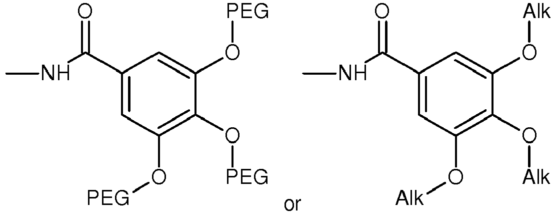
• SCO-donating groups selected from: -SCO-L-G; -SCO(OAlk); -SCO(NHAlk); -SCO(NAlk₁Alk₂); -SCO(SAlk); -SCO(Alk); -SCO-PEG;
• NHCS-donating groups selected from: -NHCS(OAlk); -NHCS(NHAlk); -NHCS(NAlk₁Alk₂); -NHCS(SAlk); -NHCS(Alk); -NHCS-PEG; -NHCS-L-G;
• SCS-donating groups selected from: -SCS(OAlk); -SCS(NHAlk); -SCS(NAlk₁Alk₂); -SCS(SAlk); -SCS(Alk); -SCS-PEG; -SCS-L-G;
- PEG is a group of formula -CH₂-(CH₂OCH₂)y-CH₂OCH₃, y being an integer ranging from 1 to 5;
- Alk, Alk₁ and Alk₂ are each a linear or branched C₁-C₁₀ alkyl optionally substituted with an -O-PEG group;
- L is a spacer arm selected from the following groups:
1) 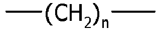
2) 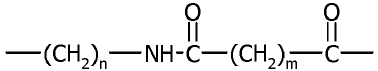
3) 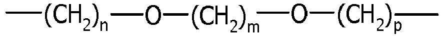
4) 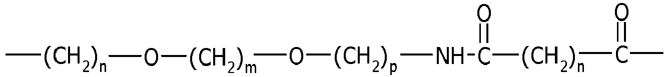
5) 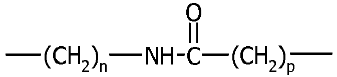
6) 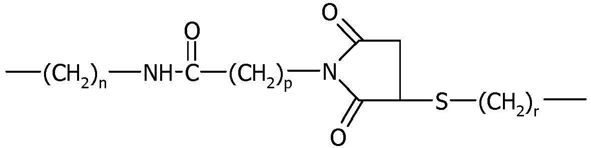
7) 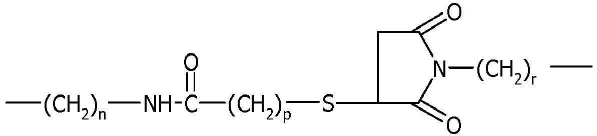
8) 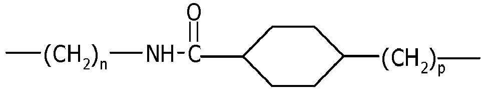
9) 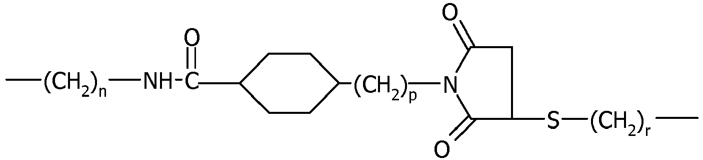
10) 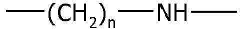
11) 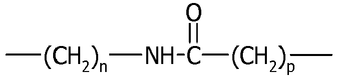
12) 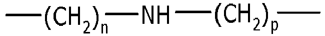
13) 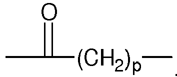
in which n, m, p, r are integers from 1 to 16;
- G is a reactive group for linking the complex or the complexing agent to a molecule to be labeled, said reactive group being selected from: an acrylamide, an activated amine, an activated ester, an aldehyde, an alkyl halide, an anhydride, an aniline, an azide, an aziridine, a carboxylic acid, a diazoalkane, a haloacetamide, a halotriazine, a hydrazine, an imido ester, an isocyanate, an isothiocyanate, a maleimide, a sulfonyl halide, a thiol, a ketone, an amine, an acid halide, a hydroxysuccinimidyl ester, a hydroxysulfosuccinimidyl ester, an azidonitrophenyl, an azidophenyl, a 3-(2-pyridyldithio)propionamide, a glyoxal, a triazine, an acetylenic group, and a group of formula: 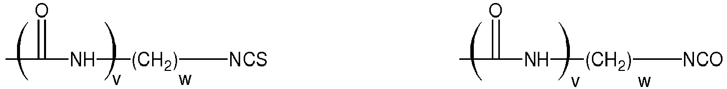 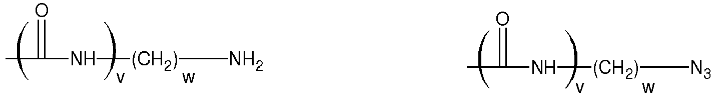 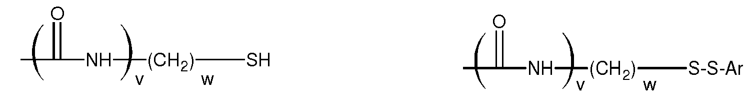 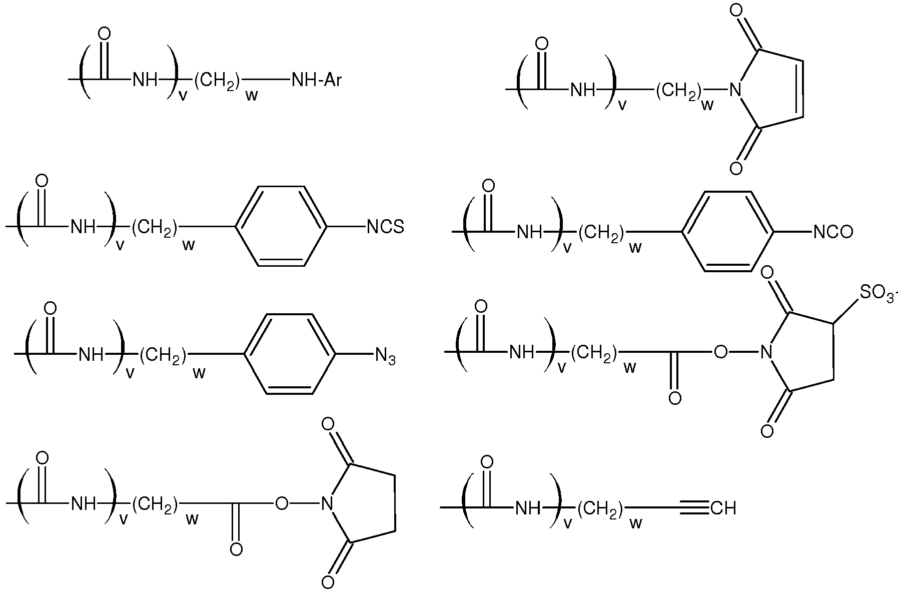 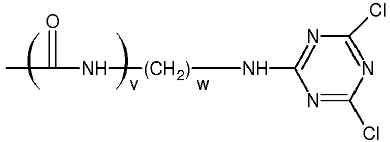 in which w is in the range from 0 to 8 and v is equal to 0 or 1, and Ar is a saturated or unsaturated 5- or 6-membered heterocycle, comprising 1 to 3 heteroatoms, optionally substituted with a halogen atom;
it being understood that:
- when R₂₂ and R₂₁ are alkyl groups, d=1 and when one of the groups R₂₂ or R₂₁ is an alkyl group and the other is a hydrogen atom, d=1 or d=2;
- when R₂ comprises a group -L-G, R₃ = R₄ = R₅ = H;
- when R₂ does not comprise a group -L-G, either one of the groups R₃, R₄ and R₅ is a group -L-G or R₃ = R₄ = R₅ = H; and
- when several groups R₂ are present, at least one is in position 4 of the benzene ring.

2. The complexing agent of claim 1, wherein:
- Chrom₁ Chrom₂ and Chrom₃ are identical or different chromophores and all three correspond to only one of the following formulae:

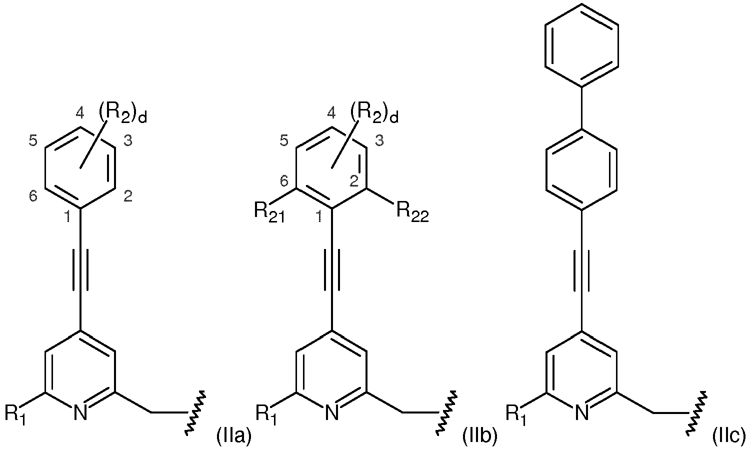

3. The complexing agent of claim 1, of formula (I): 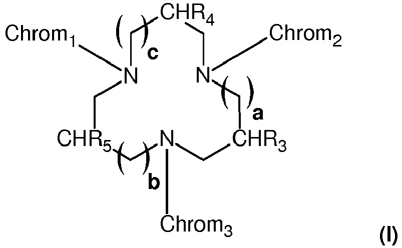 wherein:
- either a=b=c=1, or a=b=c=0, or a=1 and b=c=0, or a=b=1 and c=0;
- R₃, R₄ and R₅ are each selected from the following groups or atoms: H, -L-G;
- Chrom₁ Chrom₂ and Chrom₃ are identical or different chromophores of formula (II): 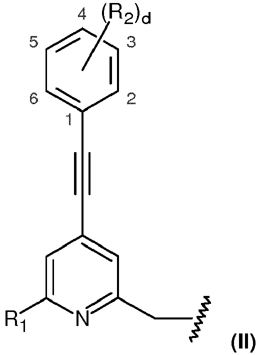 wherein
- d is an integer in the range from 1 to 3;
- R₁ is chosen from the following groups: -COOH, -PO(OH)R₆, R₆ representing a group selected from: phenyl, benzyl, methyl, ethyl, propyl, *n*-butyl, *sec*-butyl, isobutyl, *tert-*butyl;
- each R₂ is selected from the following groups:
• linear or branched alkyl groups comprising from 1 to 6 carbon atoms optionally substituted with a PEG group;
• O-donating groups selected from: -O-L-G; -O-Alk; -O-PEG; the group of formula: 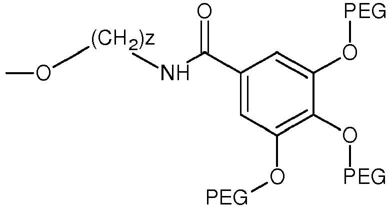 in which z is an integer in the range from 1 to 5;
• S-donating groups selected from: -S-L-G; -S-Alk; -S-PEG; the group of formula: 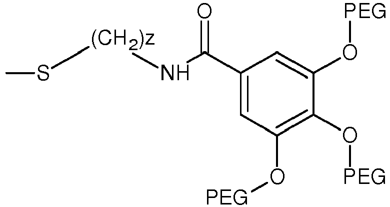 in which z is an integer in the range from 1 to 5;
• NHCO-donating groups selected from: -NHCO-L-G; -NHCO(OAlk); -NHCO(NHAlk); -NHCO(NAlk₁Alk₂); -NHCO(SAlk); -NHCO(Alk); -NHCO-PEG; the group of formula: 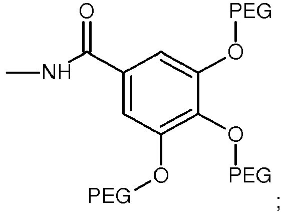
• SCO-donating groups selected from: -SCO-L-G; -SCO(OAlk); -SCO(NHAlk); -SCO(NAlk₁Alk₂); -SCO(SAlk); -SCO(Alk); -SCO-PEG;
• NHCS-donating groups selected from: -NHCS(OAlk); -NHCS(NHAlk); -NHCS(NAlk₁Alk₂); -NHCS(SAlk); -NHCS(Alk); -NHCS-PEG; -NHCS-L-G;
• SCS-donating groups selected from: -SCS(OAlk); -SCS(NHAlk); -SCS(NAlk₁Alk₂); -SCS(SAlk); -SCS(Alk); -SCS-PEG; -SCS-L-G;
- PEG is a group of formula -CH₂-(CH₂OCH₂)y-CH₂OCH₃, y being an integer in the range from 1 to 5;
- Alk, Alk₁ and Alk₂ are each a linear or branched C₁-C₁₀ alkyl optionally substituted with an -O-PEG group;
- L is a spacer arm as defined in claim 1;
- G is a reactive group as defined in claim 1, for linking the complex or the complexing agent to a molecule to be labeled;
it being understood that:
- when R₂ comprises a group -L-G, R₃ = R₄ = R₅ = H;
- when R₂ does not comprise a group -L-G, either one of the groups R₃, R₄ and R₅ is a group -L-G, or R₃ = R₄ = R₅ = H; and
- when several groups R₂ are present, at least one is in position 4 of the benzene ring.

4. The complexing agent of any one of the preceding claims, **characterized in that**:
*either* two of the groups chrom₁, chrom₂ and chrom₃ are identical and are each substituted with one to three groups R₂ not comprising a group -L-G and the third chromophore is substituted with a group R₂ comprising a group -L-G, the groups R₃-R₅ are hydrogen atoms;
*or* chrom₁, chrom₂ and chrom₃ are identical and are each substituted with one to three groups R₂ not comprising a group -L-G, and the groups R₃-R₅ are hydrogen atoms;
*or* chrom₁, chrom₂ and chrom₃ are identical and are substituted with one to three groups R₂ not comprising a group -L-G, and one of the groups R₃-R₅ is a group L-G, the others being hydrogen atoms.

5. The complexing agent ofany one of the preceding claims, **characterized in that** one of the groups R₂ comprises a group -L-G.

6. The complexing agent of any one of the preceding claims, **characterized in that** one of the groups R₃, R₄ or R₅ comprises a group -L-G.

7. The complexing agent of any one of the preceding claims, **characterized in that** it does not comprise any group -L-G.

8. The complexing agent of any one of the preceding claims, **characterized in that** a=b=c=0 (1,4,7-triazacyclononane).

9. The complexing agent of any one of the preceding claims, **characterized in that** the groups R₁ of the chromophores chrom₁, chrom₂ and chrom₃ are -COOH groups.

10. The complexing agent of any one of the preceding claims, **characterized in that** the groups R₁ of the chromophores chrom₁, chrom₂ and chrom₃ are groups -PO(OH)R₆.

11. The complexing agent of any one of the preceding claims, **characterized in that** the group R₁ of the chromophore chrom₁ is a -COOH group and the groups R₁ of the chromophores chrom₂ and chrom₃ are groups -PO(OH)R₆.

12. The complexing agent of any one of the preceding claims, **characterized in that** the group R₁ of the chromophore chrom₁ is a group -PO(OH)R₆ and the groups R₁ of the chromophores chrom₂ and chrom₃ are -COOH groups.

13. The complexing agent of any one of the preceding claims, **characterized in that** the groups R₂ are O-donating groups selected from: -O-L-G; -O-Alk; -O-PEG; the group of formula:

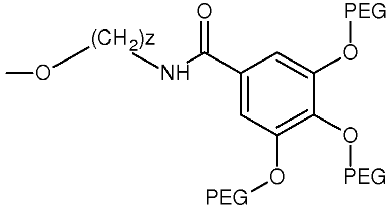

14. The complexing agent of any one of the preceding claims, **characterized in that** the groups R₂ are NHCO-donating groups selected from: -NHCO-L-G; -NHCO(OAlk); -NHCO(NHAlk); -NHCO(NAlk₁Alk₂); -NHCO(SAlk); -NHCO(Alk); -NHCO-PEG; the group of formula:

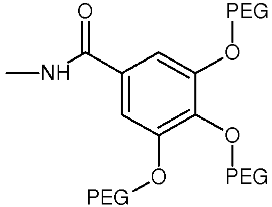

15. The complexing agent of any one of the preceding claims, **characterized in that** it comprises at least one PEG group.

16. The complexing agent of any one of the preceding claims, **characterized in that** the group -L-G, when it is present, consists of a reactive group G selected from: a carboxylic acid, an amine, a succinimidyl ester, a haloacetamide, a hydrazine, an isothiocyanate, a maleimide group, an aliphatic amine, and of a spacer arm L consisting of an alkylene chain comprising from 1 to 5 carbon atoms.

17. A lanthanide complex comprising a complexing agent of any one of the preceding claims and a lanthanide.

18. The lanthanide complex of claim 19, **characterized in that** the lanthanide is selected from: Eu³⁺, Tb³⁺, Gd³⁺, Dy³⁺, Nd³⁺, Er³⁺, preferably the lanthanide is Eu³⁺.

19. A fluorescent conjugate of a molecule to be labeled, obtained by placing a lanthanide complex of one of claims 17 and 18, said complex comprising a group -L-G, in contact with a molecule of interest.

20. A method for synthesizing mono-protected trinitrogenous macrocycles, **characterized in that** it comprises the following steps:
(i) protection of two of the three amines of the trinitrogenous macrocycle with a hydrogenation-sensitive protecting group Pg₁;
(ii) purification of the disubstituted compounds on a column selected from: a basic alumina column; a silica column which has undergone a pretreatment with triethylamine or ammonia; a neutral alumina column, the latter being preferred;
(iii) protection of the third amine of said trinitrogenous macrocycle with a second protecting group Pg₂ which is hydrogenation-insensitive;
(iv) deprotection of the amines protected with the protecting groups Pg₁ by hydrogenation, preferably with Pearlman's catalyst;
(v) purification of the monoprotected products, for example by formation of dihydrochloride salts by adding cold hydrochloric acid and precipitation from diethyl ether;
Pg₁ and Pg₂ being selected from the following groups: Carbobenzyloxy (Cbz), p-Methoxybenzylcarbonyl (Moz) *tert*-Butyloxycarbonyl (Boc), 9-Fluorenylmethyloxycarbonyl (Fmoc), Acetyl (Ac), Benzoyl (Bz), Benzyl (Bn), p-Methoxybenzyl (PMB), 3,4-Dimethoxybenzyl (DMPM), p-methoxyphenyl (PMP), Tosyl (Ts), Nosyl (Ns), trifluoroacetamides, alkoxycarbonyls, allyloxycarbonyl (Aloc), 2-(trimethylsilyl)ethoxycarbonyl, 2,2,2-trichloroethoxycarbonyl (Troc), 2-(trimethylsilyl)ethoxycarbonyl (Teoc), B-(trimethylsilyl)ethanesulfonyl (SES), benzhydryl, trityl, 9-phenylfluorenyl and N-silyl imines.

21. A dihydrochloride salt of a compound of formula: 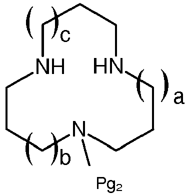 wherein
Pg₂ is a hydrogenation-insensitive protecting group as defined in claim 20;
a, b and c have the same values as in claim 1.
